(19) **Europäisches Patentamt / European Patent Office / Office européen des brevets**

(11) **EP 2 123 751 A1**

(12) **EUROPEAN PATENT APPLICATION**
published in accordance with Art. 153(4) EPC

(43) Date of publication:
**25.11.2009 Bulletin 2009/48**

(21) Application number: **07832741.8**

(22) Date of filing: **29.11.2007**

(51) Int Cl.:
*C12N 15/09* (2006.01)  *A61K 38/00* (2006.01)
*A61P 1/14* (2006.01)  *A61P 3/00* (2006.01)
*A61P 3/04* (2006.01)  *A61P 3/14* (2006.01)
*A61P 9/10* (2006.01)  *A61P 25/20* (2006.01)
*A61P 43/00* (2006.01)  *C07K 14/475* (2006.01)
*C07K 16/22* (2006.01)  *C12N 1/15* (2006.01)
*C12N 1/19* (2006.01)  *C12N 1/21* (2006.01)
*C12N 5/10* (2006.01)  *C12P 21/02* (2006.01)
*C12Q 1/02* (2006.01)  *G01N 33/15* (2006.01)
*G01N 33/50* (2006.01)  *G01N 33/53* (2006.01)

(86) International application number:
**PCT/JP2007/073026**

(87) International publication number:
**WO 2008/090678 (31.07.2008 Gazette 2008/31)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **25.01.2007 JP 2007014455**

(71) Applicants:
- **Kyowa Hakko Kirin Co., Ltd.**
  **Tokyo 100-8185 (JP)**
- **JAPAN AS REPRESENTED BY THE PRESIDENT OF NATIONAL CARDIOVASCULAR CENTER**
  **Suita-shi, Osaka 565-8565 (JP)**
- **Osaka University**
  **Suita-shi**
  **Osaka 565-0871 (JP)**

(72) Inventors:
- **YAMASAKI, Motoo**
  **Machida-shi**
  **Tokyo 194-8533 (JP)**

- **TAKAHASHI, Noriyuki**
  **Machida-shi**
  **Tokyo 194-8533 (JP)**
- **MINAMINO, Naoto**
  **Suita-shi**
  **Osaka 565-8565 (JP)**
- **SASAKI, Kazuki**
  **Suita-shi**
  **Osaka 565-8565 (JP)**
- **TAKAO, Toshifumi**
  **Suita-shi**
  **Osaka 565-0871 (JP)**
- **SATOMI, Yoshinori**
  **Suita-shi**
  **Osaka 565-0875 (JP)**
- **UETA, Yoichi**
  **Kitakyushu-shi**
  **Fukuoka 807-8555 (JP)**

(74) Representative: **Grünecker, Kinkeldey, Stockmair & Schwanhäusser Anwaltssozietät**
  **Leopoldstrasse 4**
  **80802 München (DE)**

(54) **NOVEL PEPTIDE**

(57) The present invention provides novel peptides with energy-modulating activity or circulatory function-modulating activity. The peptides of the present invention have energy-modulating activity or circulatory function-modulating activity and thus are useful for treating food consumption disorders and diseases of the circulatory system.

EP 2 123 751 A1

**Description**

Technical Field

The present invention relates to novel peptides, DNAs encoding these peptides, methods for producing these peptides, pharmaceuticals comprising these peptides, substances that inhibit or promote the activities of these peptides, and methods of screening for agonists or antagonists for the receptors of these peptides.

Background Art

The hypothalamus and pituitary gland are tissues that produce and receive biologically active peptides such as hormones and neurotransmitters. The biologically active peptides produced or received by the hypothalamus or pituitary gland regulate the homeostasis of the body.

Biologically active peptides produced in the hypothalamus or pituitary gland include, for example, thyroid stimulating hormone releasing hormone (TRH), gonadotropin releasing hormone (GnRH), corticotropin releasing hormone (CRH), growth hormone releasing hormone (GRH), somatostatin (growth hormone inhibiting hormone), prolactin inhibiting hormone (PIH), prolactin releasing hormone (PRH), neuropeptide Y (NPY), orexin (OX), melanin-concentrating hormone (MCH), agouti-related protein (AGRP), melanocyte-stimulating hormone (MSH), and cocaine- and amphetamine-regulated transcript (CART).

Receptors of biologically active peptides that have been confirmed to be expressed in the hypothalamus or pituitary gland include type 1 neuromedin U receptor (NMU1R), type 2 neuromedin U receptor (NMU2R), ghrelin receptor, type 1 orexin receptor (OX1R), type 2 orexin receptor (OX2R), type 1 neuropeptide Y receptor (NPY1R), type 5 neuropeptide Y receptor (NPY5R), type 4 melanocortin receptor (MC4R), type 1 corticotropin-releasing hormone receptor (CRHR-1), and type 2 corticotropin-releasing hormone receptor (CRHR-2).

Various types of intracellular signaling are induced by the interaction of such biologically active peptide receptors with the biologically active peptides as ligands. Especially, the signaling mediated by an increase in the intracellular calcium concentration is a well-known reaction.

In the hypothalamus or pituitary gland, increase in the intracellular calcium concentration is induced, for example, upon binding of type 1 neuromedin U receptor (NMU1R), type 2 neuromedin U receptor (NMU2R), ghrelin receptor, type 1 orexin receptor (OX1R), type 2 orexin receptor (OX2R) or such to each biologically active peptide as the ligand. The increase of the intracellular calcium concentration serves as a second messenger to induce further reactions for modulating the homeostasis of the body. For example, type 1 neuromedin U receptor (NMU1R), type 2 neuromedin U receptor (NMU2R), and ghrelin receptor regulate the feeding reaction, while type 1 orexin receptor (OX1R) and type 2 orexin receptor (OX2R) modulate the feeding reaction, awaking and motivation reaction.

Accordingly, biologically active peptide-mediated increases in the calcium concentration in hypothalamic or pituitary cells can serve as an indicator for the presence of the activity of regulating reactions such as energy modulation. The energy modulation includes activities such as food consumption enhancement, food consumption suppression, water consumption enhancement, water consumption suppression, sleep induction, enhancement of arousal, metabolic enhancement, and metabolic suppression.

Muscle contraction in cardiac muscles, skeletal muscles, and smooth muscles occur through interaction between actin and myosin filaments and the interaction is triggered by an increase in the intracellular calcium concentration. Thus, substances that increase the calcium concentration in muscle cells are used as muscle contracting agents. In particular, substances that increase the calcium concentration in circulatory system tissues such as cardiac muscle and vascular smooth muscle elicit blood pressure increase and the like due to the enhanced muscle contraction.

For example, biologically active peptides such as endothelin and angiotensin II are known to increase the intracellular calcium concentration via interaction with the respective receptors expressed in circulatory system tissues, including kidney, which results in blood pressure increase or the like, .

Thus, increases in the intracellular calcium concentration of circulatory system tissues caused by biologically active peptides can serve as an indicator for the presence of circulation-modulating activity.

The VGF gene was identified as a gene whose expression is increased in rat PC12 cells stimulated with nerve growth factor (see Non-patent Document 1), which in turn led to the isolation of a human VGF gene (see Non-patent Document 2). In VGF gene-disrupted mice, food consumption remained the same, but a decrease in body weight and body fat, an increase in oxygen consumption and locomotor activity, and abnormal reproductive functions were observed. In particular, enhanced energy metabolism was observed (see Non-patent Document 3).

VGF genes are expressed in the central and peripheral nervous systems, as well as in endocrine and neuroendocrine cells. Their expression and distribution are similar to the expression patterns of neuropeptide Y, peptide YY, ghrelin, cholecystokinin, and the like, which regulate feeding behavior and the gastrointestinal motility; and they are present in parts that are related to energy metabolism (see Non-patent Document 4).

Proteins encoded by the VGF genes (hereinafter referred to as VGFs) comprise 615 amino acids in human and 617 amino acids in rats/mice. Amino acids 1 to 22 of VGF is a signal peptide, and there are sequences processed by amidation, cleaved by prohormone convertase, or the like. Processing of VGF has been investigated mainly in rats, and the following peptides derived from VGF have been found in rat brains: rat VGF (598-617) (the numbers in the parentheses indicate the positions of the peptide sequence in the VGF amino acid sequence; the same applies to the peptides shown below), rat VGF (599-617), rat VGF (601-617), rat VGF (602-617), rat VGF (587-617), rat VGF (588-617), rat VGF (567-617), rat VGF (556-617), rat VGF (489-617), and rat VGF18 (18 kDa; unknown sequence) (see Non-patent Document 5). Furthermore, rat VGF (556-576) peptide has also been found in rat brain extract, and reported to have the activity of suppressing weight gain in rats fed with high-calorie diets when it is administered into the rat ventricle (see Non-patent Document 6). Direct administration of partial peptides of rat VGF (556-617): rat VGF (577-617), rat VGF (588-617), and rat VGF (599-617) to the paraventricular nucleus of the hypothalamus (PVN) of male rats showed an erection inducing activity; however, this activity was not observed with rat VGF (556-576) (see Non-patent Document 7). Furthermore, it is reported that when VGF (588-596) was administered intraperitoneally to VGF gene-disrupted mice, the body weight increased by about 10% to 15% (see Patent Document 1).

The following peptides are derived from human VGF and present in the human cerebrospinal fluid: human VGF (23-62), human VGF (23-59), and human VGF (26-62) (see Non-patent Document 8); and human VGF (23-58), human VGF (24-59), human VGF (24-62), human VGF (26-57), human VGF (26-58), human VGF (26-59), human VGF (26-61), human VGF (26-64), human VGF (49-62), human VGF (90-114), human VGF (350-367), human VGF (350-370), human VGF (373-404), human VGF (373-417), human VGF (420-471), and human VGF (420-478) (see Patent Document 2). Furthermore, a peptide whose sequence corresponds to rat VGF (588-617) and is identical to positions 586-615 of the human VGF sequence has been isolated from the bovine posterior pituitary gland (see Non-patent Document 9). However, there has been no report on the physiological activities of these peptides derived from human or bovine VGF.

As antibodies that specifically recognize VGF or peptides derived from VGF, polyclonal antibodies against antigenic peptide comprising the C-terminal 573-617th amino acid sequence of rat VGF (see Non-patent Document 10), polyclonal antibodies against antigenic peptide comprising the 556-565th amino acid sequence of human VGF (see Non-patent Document 4), polyclonal antibodies against antigenic peptide comprising the 443-588th amino acid sequence of rat VGF (see Non-patent Document 11), and the like have been reported.

[Patent Document 1] WO01/07477
[Patent Document 2] WO02/82075
[Non-patent Document 1] Science, (USA), 1985, Vol. 229, No. 4711, pp. 393-395.
[Non-patent Document 2] Genomics, (USA), 1997, Vol. 45, No. 2, pp. 443-446.
[Non-patent Document 3] Neuron, (USA), 1999, Vol. 23, No. 3, pp. 537-548.
[Non-patent Document 4] Cellular and Molecular Neurobiology, (USA), 2004, Vol. 24, No. 4, pp. 517-533.
[Non-patent Document 5] Journal of Neurochemistry, (UK), 2002, Vol. 81, No. 3, pp. 565-574.
[Non-patent Document 6] Proceeding of the National Academy of Sciences of the United States of America, (USA), 2006, Vol. 103, No. 39, pp. 14584-14589.
[Non-patent Document 7] European Journal of Neuroscience, (France), 2004, Vol. 20, No. 11, pp. 3035-3040.
[Non-patent Document 8] Journal of Chromatography B: Biomedical Sciences and Applications, (Holland), 2001, Vol. 754, No. 2, pp. 357-367.
[Non-patent Document 9] Endocrinology, (USA), 1994, Vol. 135, No. 6, pp. 2742-2748.
[Non-patent Document 10] Endocrinology, (USA), 1999, Vol. 140, No. 8, pp. 3727-3735.
[Non-patent Document 11] The EMBO Journal, (UK), 1989, Vol. 8, No.8, pp. 2217-2223.

Disclosure of the Invention

[Problems to be Solved by the Invention]

An objective of the present invention is to provide novel peptides having an energy- or circulation-modulating activity, as well as to provide DNAs encoding these peptides, methods for producing these peptides, pharmaceuticals comprising these peptides, and methods of screening for substances that inhibit or promote the activity of these peptides.

[Means for Solving the Problems]

The present invention relates to the following [1] to [12]:

[1] a peptide of any one of (a) to (d) below or a pharmaceutically acceptable salt thereof:

(a) a peptide comprising the amino acid sequence of any one of SEQ ID NOS: 1 to 9, 11, 12, and 26 (but excluding a peptide consisting of the amino acid sequence of any one of SEQ ID NOS: 13 to 21);

(b) a peptide comprising an amino acid sequence with substitution, deletion, or addition of one to five amino acids in the amino acid sequence of any one of SEQ ID NOS: 1 to 9, 11, 12, and 26, wherein the peptide has an activity of increasing the intracellular calcium ion concentration in a cell of hypothalamus, pituitary gland, kidney, heart, blood vessel, or brain tissue;

(c) a peptide comprising an amino acid sequence having 90% or higher homology to the amino acid sequence of any one of SEQ ID NOS: 1 to 9, 11, 12, and 26, wherein the peptide has an activity of increasing the intracellular calcium ion concentration in a cell of hypothalamus, pituitary gland, kidney, heart, blood vessel, or brain tissue; and

(d) a peptide represented by the following formula (I)

$$R^1\text{-}A\text{-}R^2 \qquad (I)$$

(wherein, $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue of the peptide of any one of the above-mentioned (a) to (c));

[2] a DNA encoding any one of the peptides of (a) to (c) of [1];

[3] a recombinant vector obtainable by incorporating the DNA of [2] into a vector;

[4] a transformant obtainable by introducing the recombinant vector of [3] into a host cell;

[5] a method for producing a peptide, which comprises culturing the transformant of [4] in a medium so as to produce and accumulate said peptide in the culture, and recovering said peptide from the culture;

[6] an antibody that binds to an epitope present in the amino acid sequence of SEQ ID NO: 1 or 26;

[7] a method of detecting or quantifying the peptide of [1], which comprises using the antibody of [6];

[8] an energy-modulating agent comprising as an active ingredient at least one peptide selected from (a) to (f) below or a pharmaceutically acceptable salt thereof:

(a) a peptide comprising the amino acid sequence of any one of SEQ ID NOS: 1 to 12, and 23 to 29;

(b) a peptide comprising an amino acid sequence with substitution, deletion, or addition of one to five amino acids in the amino acid sequence of any one of SEQ ID NOS: 1 to 12, and 23 to 29, wherein the peptide has an activity of increasing the intracellular calcium ion concentration in a cell of hypothalamus, pituitary gland, or brain tissue;

(c) a peptide comprising an amino acid sequence having 90% or higher homology to the amino acid sequence of any one of SEQ ID NOS: 1 to 12, and 23 to 29, wherein the peptide has an activity of increasing the intracellular calcium ion concentration in a cell of hypothalamus, pituitary gland, or brain tissue;

(d) a peptide comprising an amino acid sequence with substitution, deletion, or addition of one to five amino acids in the amino acid sequence of any one of SEQ ID NOS: 1, 25, 28, and 29, wherein the peptide has an activity of promoting vasopressin secretion from the posterior pituitary gland;

(e) a peptide comprising an amino acid sequence having 90% or higher homology to the amino acid sequence of any one of SEQ ID NOS: 1, 25, 28, and 29, wherein the peptide has an activity of promoting vasopressin secretion from the posterior pituitary gland; and

(f) a peptide represented by the following formula (II)

$$R^3\text{-}B\text{-}R^4 \qquad (II)$$

(wherein, $R^3$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^4$ represents hydroxy, substituted or unsubstituted alkoxy, or substituted or unsubstituted amino; and B represents a peptide residue of the peptide of any one of the above-mentioned (a) to (e));

[9] a circulation-modulating agent comprising as an active ingredient at least one peptide selected from (a) to (f) below or a pharmaceutically acceptable salt thereof:

(a) a peptide comprising the amino acid sequence of any one of SEQ ID NOS: 1 to 12;

(b) a peptide comprising an amino acid sequence with substitution, deletion, or addition of one to five amino acids in the amino acid sequence of any one of SEQ ID NOS: 1 to 12, wherein the peptide has an activity of increasing the intracellular calcium ion concentration in a cell of kidney, heart, or blood vessel;

(c) a peptide comprising an amino acid sequence having 90% or higher homology to the amino acid sequence of any one of SEQ ID NOS: 1 to 12, wherein the peptide has an activity of increasing the intracellular calcium ion concentration in a cell of kidney, heart, or blood vessel;

(d) a peptide comprising an amino acid sequence with substitution, deletion, or addition of one to five amino acids in the amino acid sequence of any one of SEQ ID NOS: 1, 25, 28, and 29, wherein the peptide has an activity of promoting vasopressin secretion from the posterior pituitary gland;

(e) a peptide comprising an amino acid sequence having 90% or higher homology to the amino acid sequence of any one of SEQ ID NOS: 1, 25, 28, and 29, wherein the peptide has an activity of promoting vasopressin secretion from the posterior pituitary gland; and

(f) a peptide represented by the following formula (III)

$$R^5\text{-}C\text{-}R^6 \qquad (III)$$

(wherein, $R^5$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^6$ represents hydroxy, substituted or unsubstituted alkoxy, or substituted or unsubstituted amino; and C represents a peptide residue of the peptide of any one of the above-mentioned (a) to (e));

[10] a method of screening for a substance that inhibits peptide-induced increase of intracellular calcium ion concentration in a cell of hypothalamus, pituitary gland, kidney, heart, blood vessel, or brain tissue, which comprises:

measuring the cellular response elicited when a test substance and the peptide of any one of (a) to (f) of [8] or [9] or a pharmaceutically acceptable salt thereof are contacted with a cell of hypothalamus, pituitary gland, kidney, heart, blood vessel, or brain tissue; and

identifying the test substance as a substance that inhibits the peptide-induced increase of intracellular calcium ion concentration in the cell of hypothalamus, pituitary gland, kidney, heart, blood vessel, or brain tissue, if the test substance suppresses the cellular response compared to the cellular response when said peptide or a pharmaceutically acceptable salt thereof is contacted with said cell in the absence of the test substance;

[11] a method of screening for a substance that promotes peptide-induced increase of intracellular calcium ion concentration in a cell of hypothalamus, pituitary gland, kidney, heart, blood vessel, or brain tissue, which comprises:

measuring the cellular response elicited when a test substance and the peptide of any one of (a) to (f) of [8] or [9] or a pharmaceutically acceptable salt thereof are contacted with a cell of hypothalamus, pituitary gland, kidney, heart, blood vessel, or brain tissue; and

identifying the test substance as a substance that promotes the peptide-induced increase of intracellular calcium ion concentration in the cell of hypothalamus, pituitary gland, kidney, heart, blood vessel, or brain tissue, if the test substance promotes the cellular response as compared to the cellular response when said peptide or a pharmaceutically acceptable salt thereof is contacted with said cell in the absence of the test substance; and

[12] a method of screening for a peptide receptor agonist or antagonist, the method comprising:

measuring the binding level of the peptide of any one of (a) to (f) of [8] or [9] or a pharmaceutically acceptable salt thereof to a cell of hypothalamus, pituitary gland, kidney, heart, blood vessel, or brain tissue, or a membrane fraction of said cell, when the test substance and the peptide or a pharmaceutically acceptable salt thereof are contacted with said cell or cell membrane fraction; and

identifying the test substance as an agonist or antagonist for the receptor of said peptide if the test substance causes a decrease in the binding level of said peptide or a pharmaceutically acceptable salt thereof as compared to the binding level when said peptide or a pharmaceutically acceptable salt thereof is contacted with said cell or a membrane fraction of said cell in the absence of the test substance.

[Effects of the Invention]

The present invention provides novel peptides having energy- or circulation-modulating activity, DNAs encoding these peptides, antibodies that specifically bind to these peptides, methods for producing these peptides, pharmaceuticals comprising these peptides, methods that use these peptides for screening for substances that promote or suppress the activity of these peptides, or for agonists or antagonists for the receptors of these peptides. The peptides of the present invention are useful for treating diseases associated with energy modulation, such as food or water consumption disorders, such as obesity and cibophobia, metabolic disorders, sleep disorders and the like, and diseases of the circulatory system, such as myocardial infarction, ischemic heart disease, cerebral infarction, and the like.

Brief Description of the Drawings

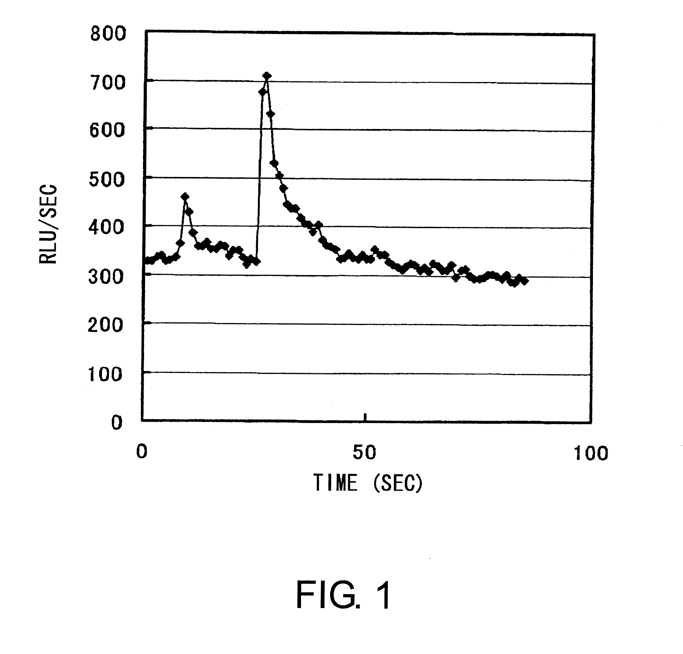

Fig. 1 shows an increase of intracellular calcium concentration in hypothalamic cells of the apoaequorin-expressing mice due to 1 $\mu$mol/L of Peptide 1 (SEQ ID NO: 1). The horizontal axis indicates the time (seconds) after addition of the medium, and the vertical axis indicates the relative luminescence unit (RLU) per second. Peptide 1 was added at 25 seconds.

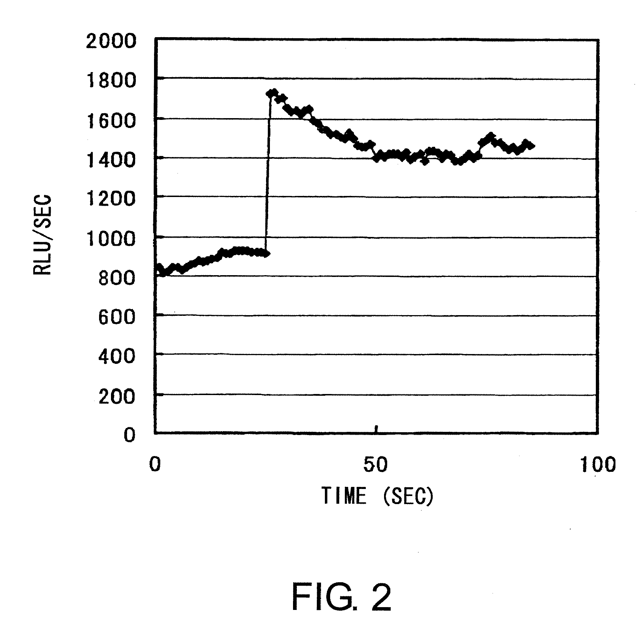

Fig. 2 shows an increase of intracellular calcium concentration in pituitary cells of the apoaequorin-expressing mice due to 5 $\mu$mol/L of Peptide 1. The horizontal axis indicates the time (seconds) after addition of the medium, and the vertical axis indicates the relative luminescence unit (RLU) per second. Peptide 1 was added at 25 seconds.

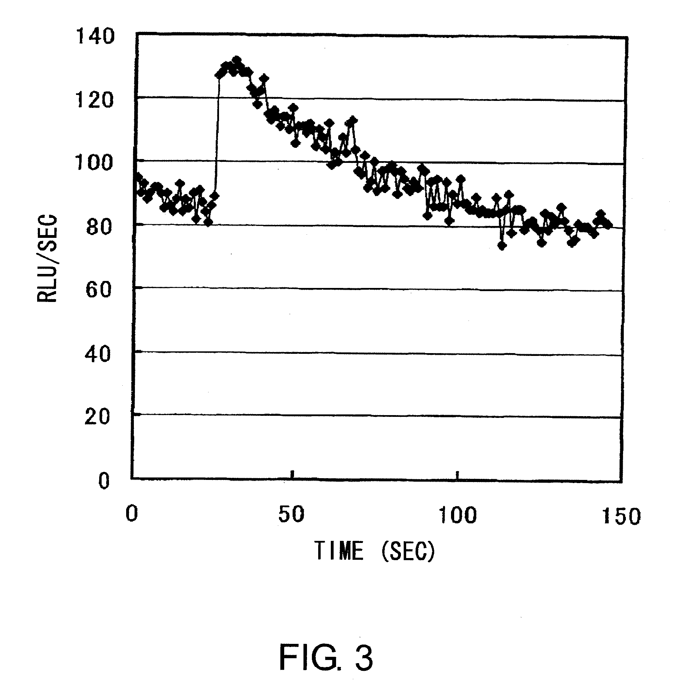

Fig. 3 shows an increase of intracellular calcium concentration in hypothalamic cells of the apoaequorin-expressing mice due to 1 $\mu$mol/L of Peptide 2 (SEQ ID NO: 2). The horizontal axis indicates the time (seconds) after addition of the medium, and the vertical axis indicates the relative luminescence unit (RLU) per second. Peptide 2 was added at 25 seconds.

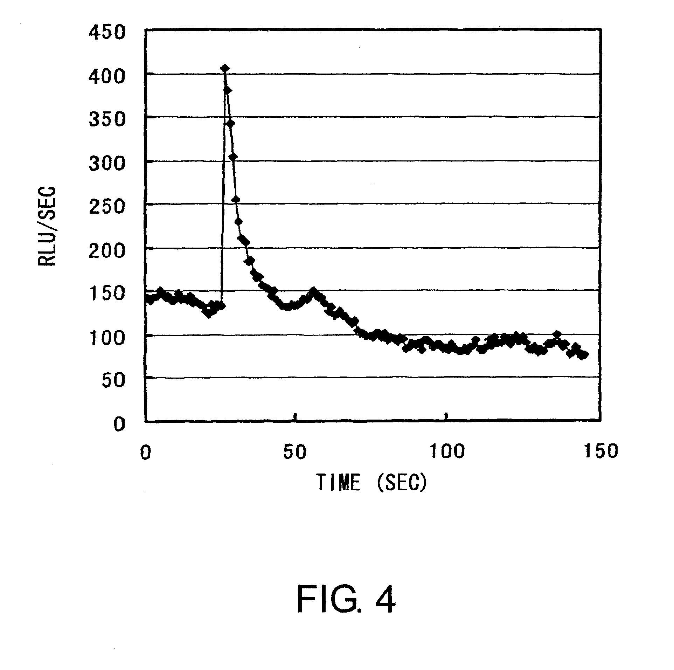

Fig. 4 shows an increase of intracellular calcium concentration in pituitary cells of the apoaequorin-expressing mice due to 1 $\mu$mol/L of Peptide 2. The horizontal axis indicates the time (seconds) after addition of the medium, and the vertical axis indicates the relative luminescence unit (RLU) per second. Peptide 2 was added at 25 seconds.

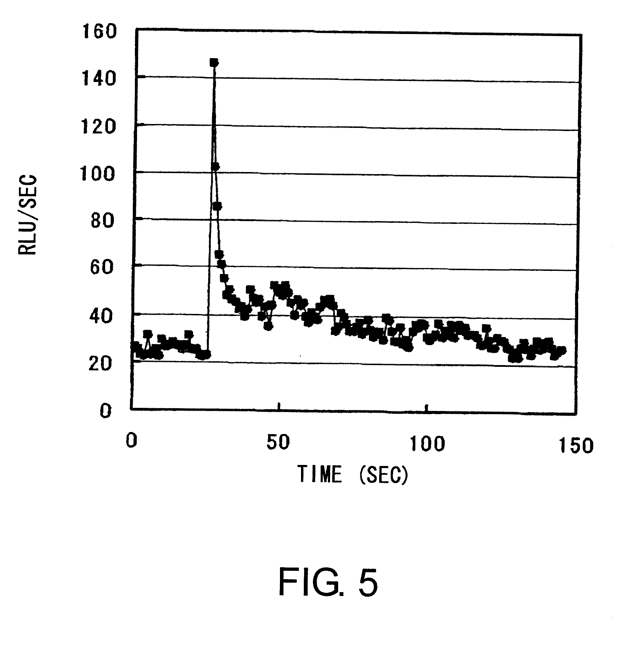

Fig. 5 shows an increase of intracellular calcium concentration in cardiac cells of the apoaequorin-expressing mice due to 1 $\mu$mol/L of Peptide 2. The horizontal axis indicates the time (seconds) after addition of the medium, and the vertical axis indicates the relative luminescence unit (RLU) per second. Peptide 2 was added at 25 seconds.

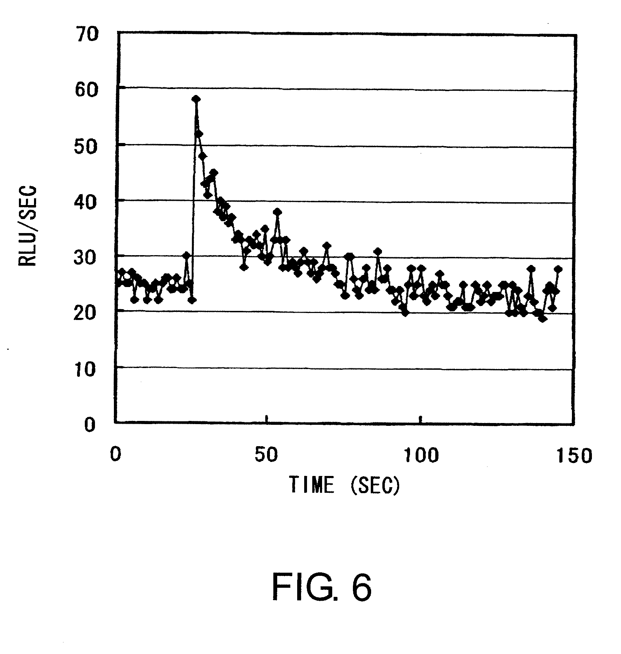

Fig. 6 shows an increase of intracellular calcium concentration in hypothalamic cells of the apoaequorin-expressing mice due to 1 $\mu$mol/L of Peptide 3 (SEQ ID NO: 3). The horizontal axis indicates the time (seconds) after addition of the medium, and the vertical axis indicates the relative luminescence unit (RLU) per second. Peptide 3 was added at 25 seconds.

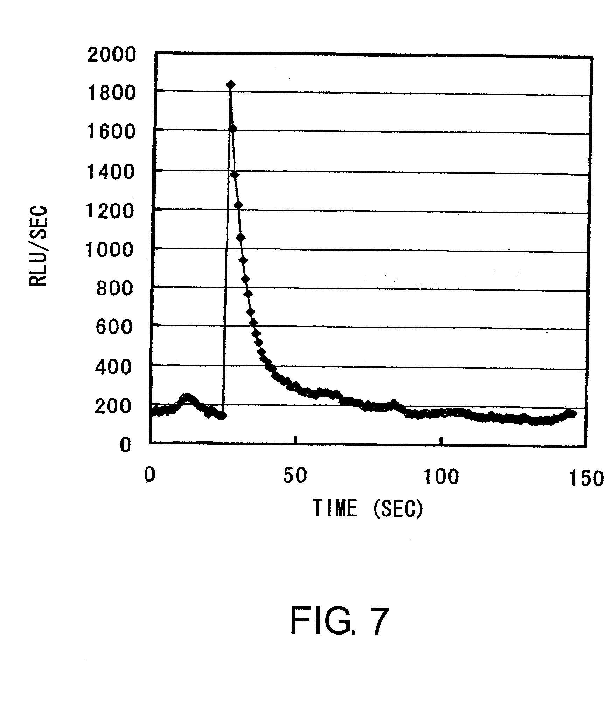

Fig. 7 shows an increase of intracellular calcium concentration in pituitary cells of the apoaequorin-expressing mice due to 5 $\mu$mol/L of Peptide 3. The horizontal axis indicates the time (seconds) after addition of the medium, and the vertical axis indicates the relative luminescence unit (RLU) per second. Peptide 3 was added at 25 seconds.

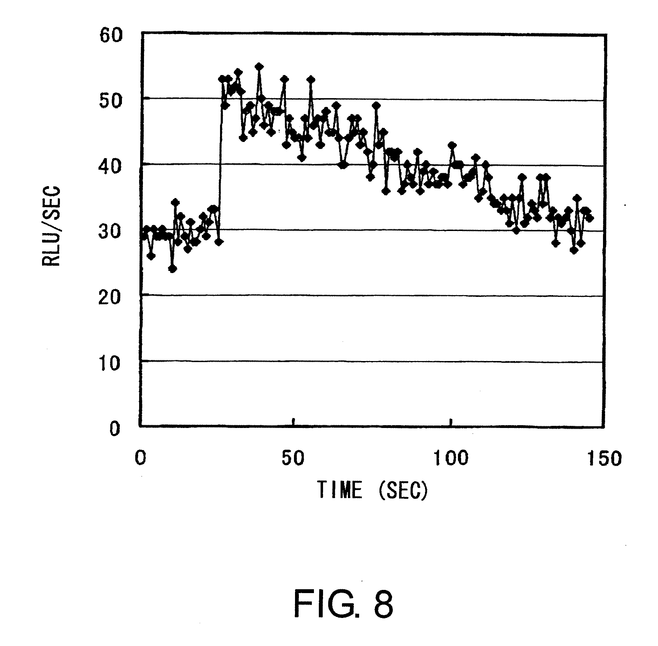

Fig. 8 shows an increase of intracellular calcium concentration in kidney cells of the apoaequorin-expressing mice due to 1 $\mu$mol/L of Peptide 3. The horizontal axis indicates the time (seconds) after addition of the medium, and the vertical axis indicates the relative luminescence unit (RLU) per second. Peptide 3 was added at 25 seconds.

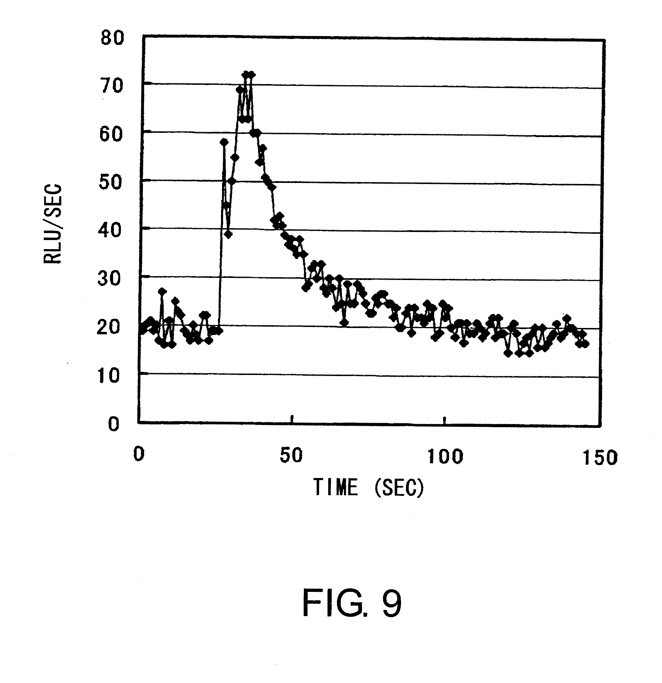

Fig. 9 shows an increase of intracellular calcium concentration in hypothalamic cells of the apoaequorin-expressing mice due to 1 $\mu$mol/L of Peptide 4 (SEQ ID NO: 4). The horizontal axis indicates the time (seconds) after addition of the medium, and the vertical axis indicates the relative luminescence unit (RLU) per second. Peptide 4 was added at 25 seconds.

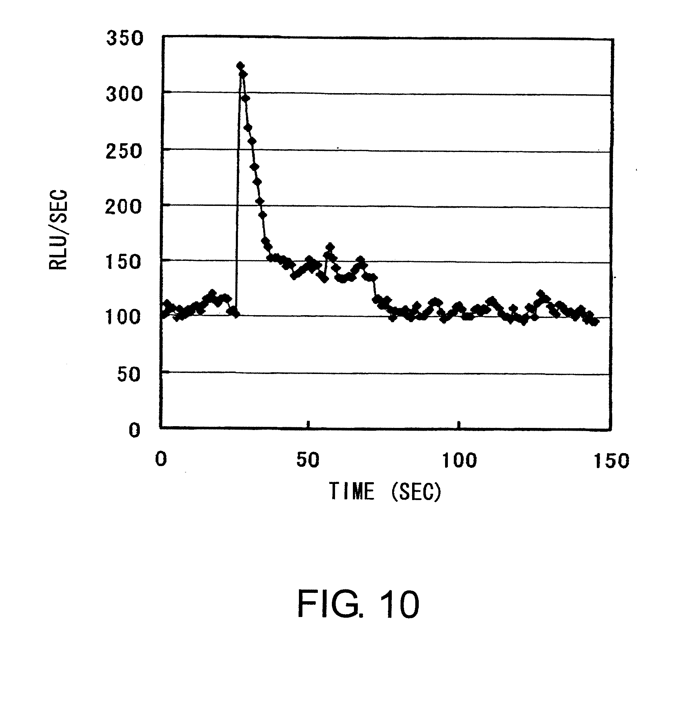

Fig. 10 shows an increase of intracellular calcium concentration in pituitary cells of the apoaequorin-expressing mice due to 1 $\mu$mol/L of Peptide 4. The horizontal axis indicates the time (seconds) after addition of the medium, and the vertical axis indicates the relative luminescence unit (RLU) per second. Peptide 4 was added at 25 seconds.

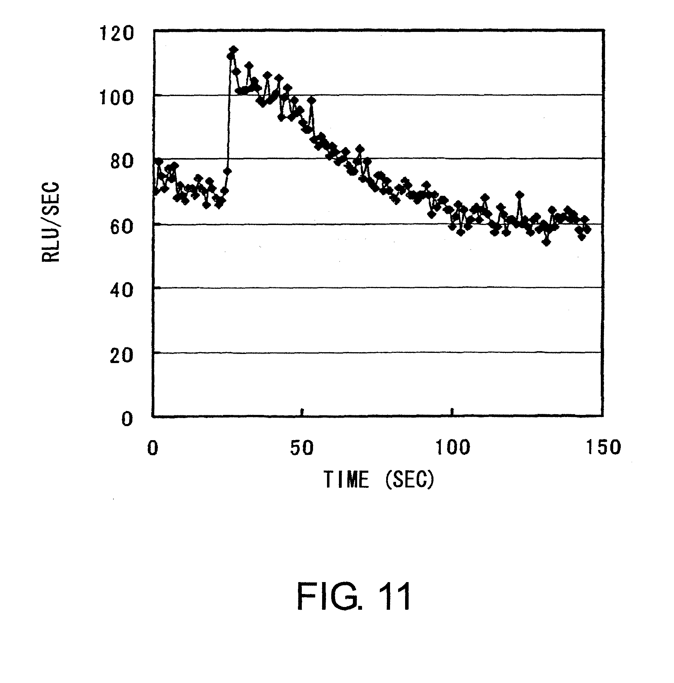

Fig. 11 shows an increase of intracellular calcium concentration in hypothalamic cells of the apoaequorin-expressing mice due to 5 $\mu$mol/L of Peptide 5 (SEQ ID NO: 5). The horizontal axis indicates the time (seconds) after addition of the medium, and the vertical axis indicates the relative luminescence unit (RLU) per second. Peptide 5 was added at 25 seconds.

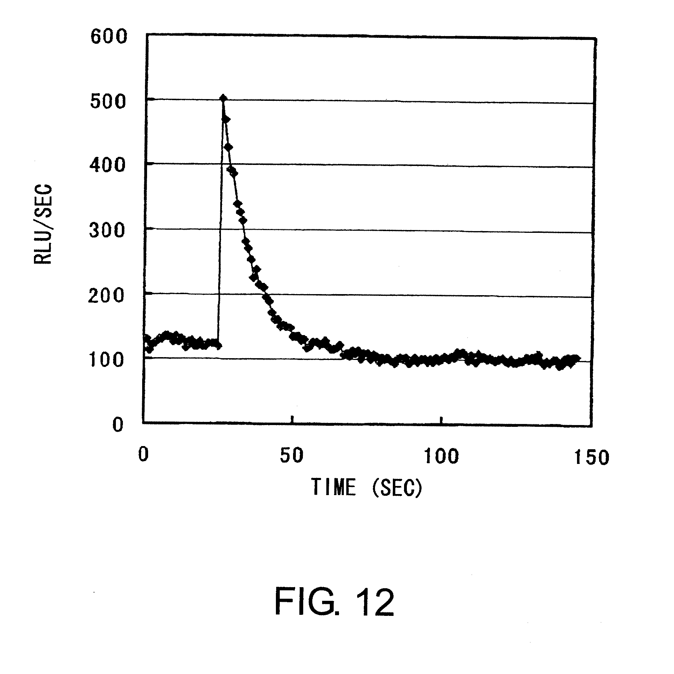

Fig. 12 shows an increase of intracellular calcium concentration in pituitary cells of the apoaequorin-expressing mice due to 5 $\mu$mol/L of Peptide 5. The horizontal axis indicates the time (seconds) after addition of the medium, and the vertical axis indicates the relative luminescence unit (RLU) per second. Peptide 5 was added at 25 seconds.

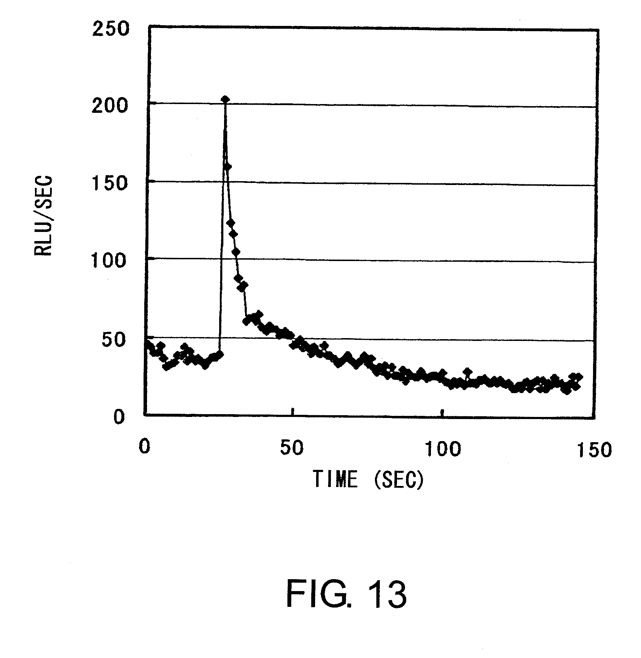

Fig. 13 shows an increase of intracellular calcium concentration in cardiac cells of the apoaequorin-expressing mice due to 5 $\mu$mol/L of Peptide 5. The horizontal axis indicates the time (seconds) after addition of the medium, and the vertical axis indicates the relative luminescence unit (RLU) per second. Peptide 5 was added at 25 seconds.

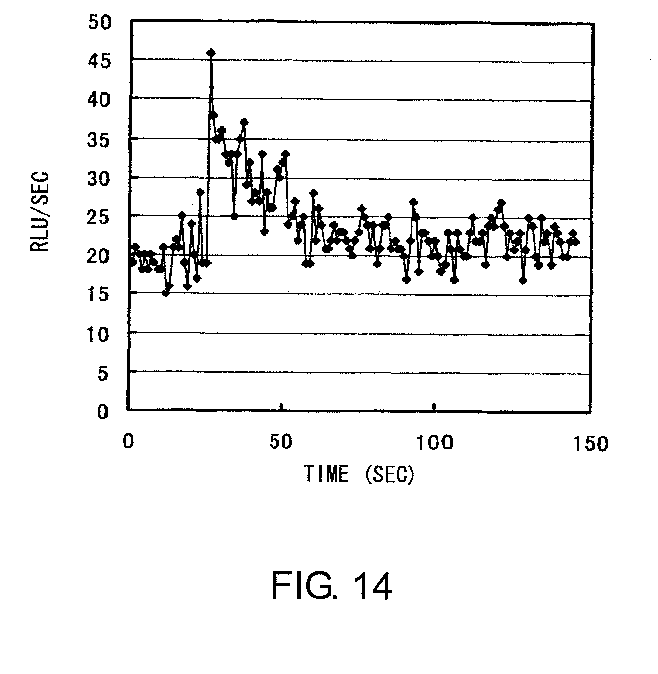

Fig. 14 shows an increase of intracellular calcium concentration in kidney cells of the apoaequorin-expressing mice due to 1 $\mu$mol/L of Peptide 5. The horizontal axis indicates the time (seconds) after addition of the medium, and the vertical axis indicates the relative luminescence unit (RLU) per second. Peptide 5 was added at 25 seconds.

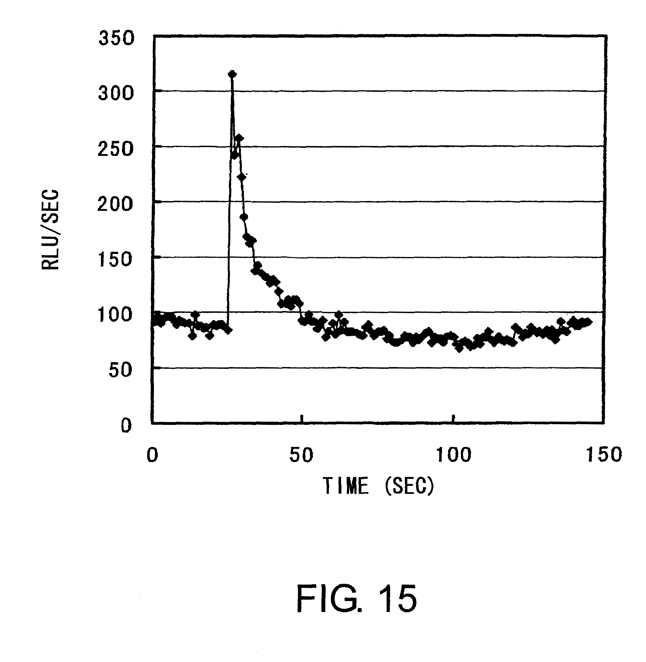

Fig. 15 shows an increase of intracellular calcium concentration in hypothalamic cells of the apoaequorin-expressing mice due to 5 μmol/L of Peptide 6 (SEQ ID NO: 6). The horizontal axis indicates the time (seconds) after addition of the medium, and the vertical axis indicates the relative luminescence unit (RLU) per second. Peptide 6 was added at 25 seconds.

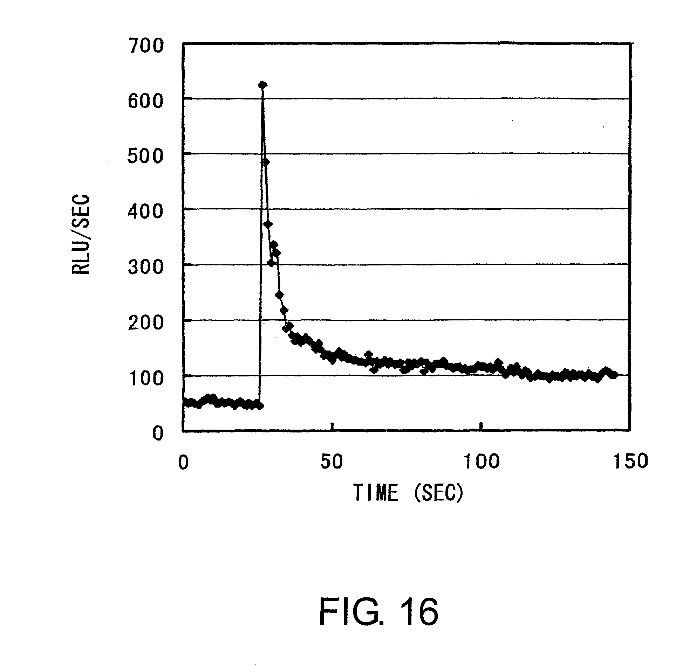

Fig. 16 shows an increase of intracellular calcium concentration in pituitary cells of the apoaequorin-expressing mice due to 5 μmol/L of Peptide 6. The horizontal axis indicates the time (seconds) after addition of the medium, and the vertical axis indicates the relative luminescence unit (RLU) per second. Peptide 6 was added at 25 seconds.

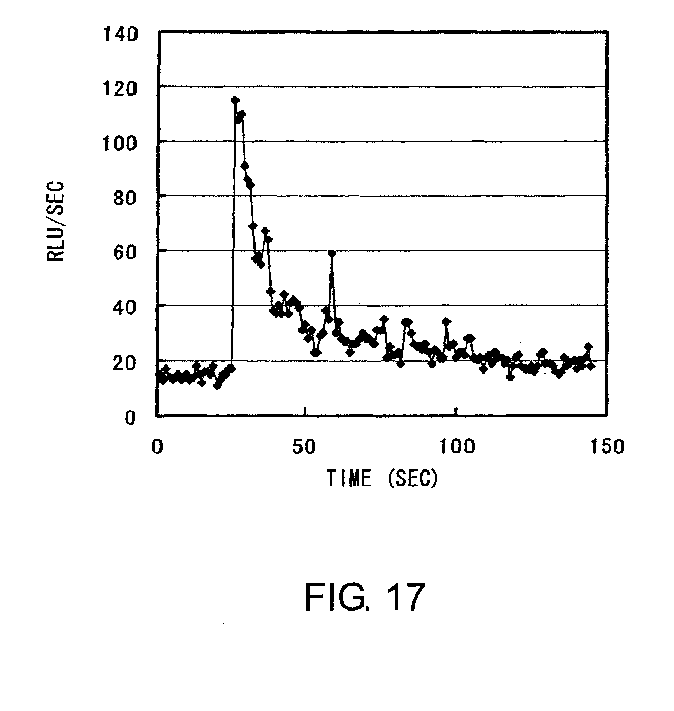

Fig. 17 shows an increase of intracellular calcium concentration in aortic cells of the apoaequorin-expressing mice due to 1 μmol/L of Peptide 6. The horizontal axis indicates the time (seconds) after addition of the medium, and the vertical axis indicates the relative luminescence unit (RLU) per second. Peptide 6 was added at 25 seconds.

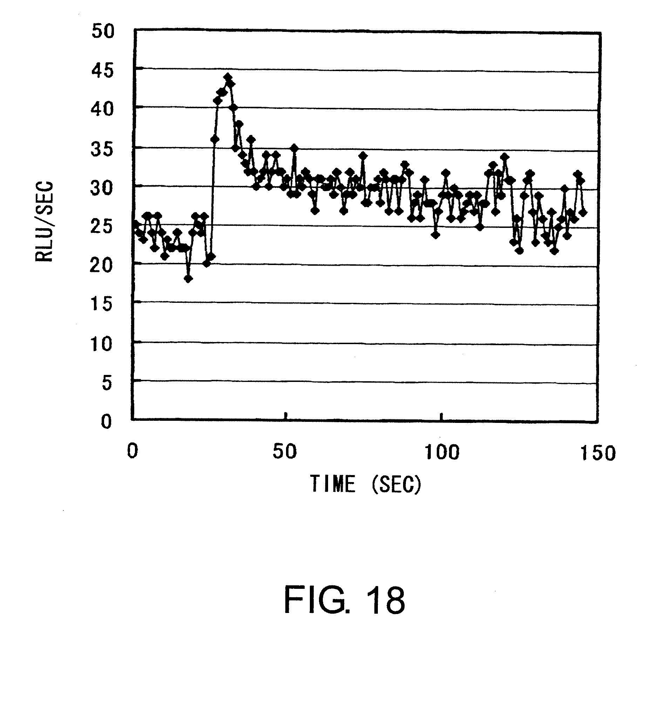

Fig. 18 shows an increase of intracellular calcium concentration in kidney cells of the apoaequorin-expressing mice due to 1 μmol/L of Peptide 6. The horizontal axis indicates the time (seconds) after addition of the medium, and the vertical axis indicates the relative luminescence unit (RLU) per second. Peptide 6 was added at 25 seconds.

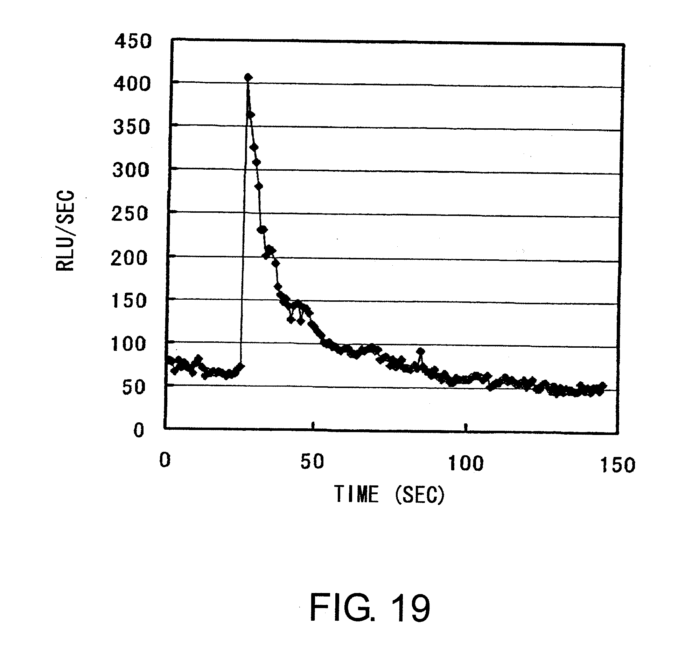

Fig. 19 shows an increase of intracellular calcium concentration in hypothalamic cells of the apoaequorin-expressing mice due to 1 μmol/L of Peptide 7 (SEQ ID NO: 7). The horizontal axis indicates the time (seconds) after addition of the medium, and the vertical axis indicates the relative luminescence unit (RLU) per second. Peptide 7 was added at 25 seconds.

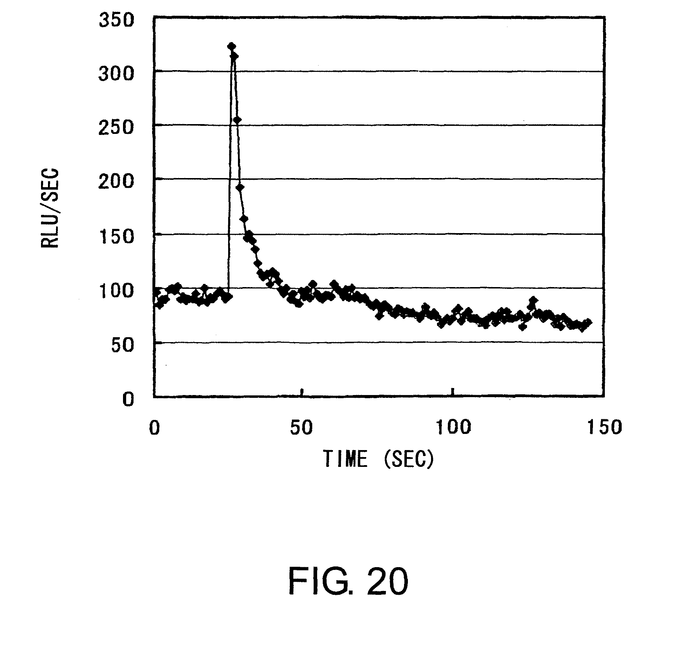

Fig. 20 shows an increase of intracellular calcium concentration in pituitary cells of the apoaequorin-expressing mice due to 1 μmol/L of Peptide 7. The horizontal axis indicates the time (seconds) after addition of the medium, and the vertical axis indicates the relative luminescence unit (RLU) per second. Peptide 7 was added at 25 seconds.

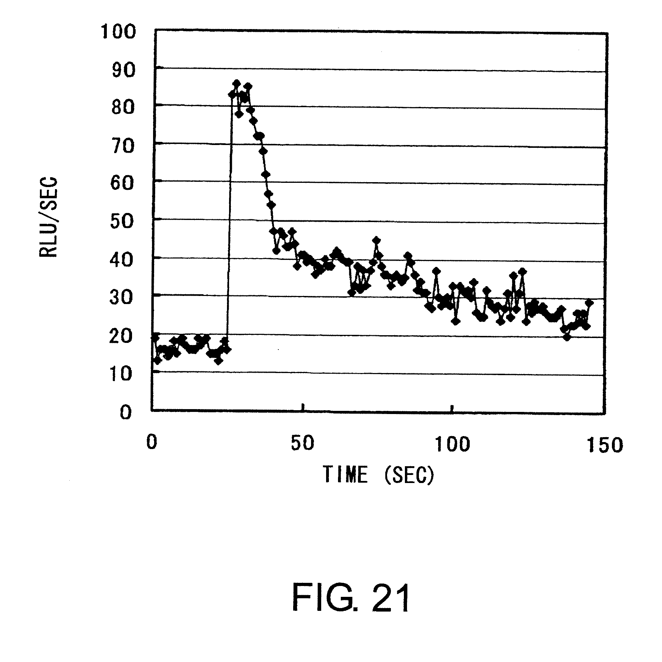

Fig. 21 shows an increase of intracellular calcium concentration in aortic cells of the apoaequorin-expressing mice due to 1 μmol/L of Peptide 7. The horizontal axis indicates the time (seconds) after addition of the medium, and the vertical axis indicates the relative luminescence unit (RLU) per second. Peptide 7 was added at 25 seconds.

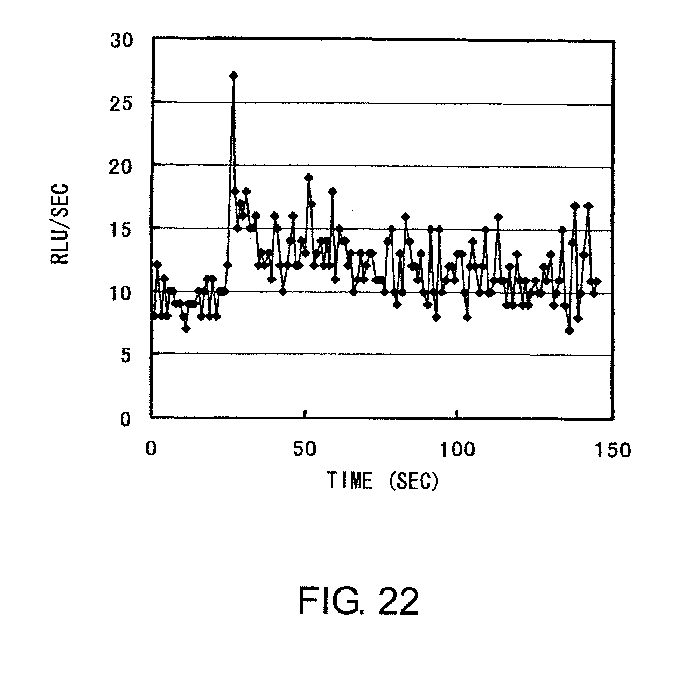

Fig. 22 shows an increase of intracellular calcium concentration in cardiac cells of the apoaequorin-expressing mice due to 1 μmol/L of Peptide 7. The horizontal axis indicates the time (seconds) after addition of the medium, and the vertical axis indicates the relative luminescence unit (RLU) per second. Peptide 7 was added at 25 seconds.

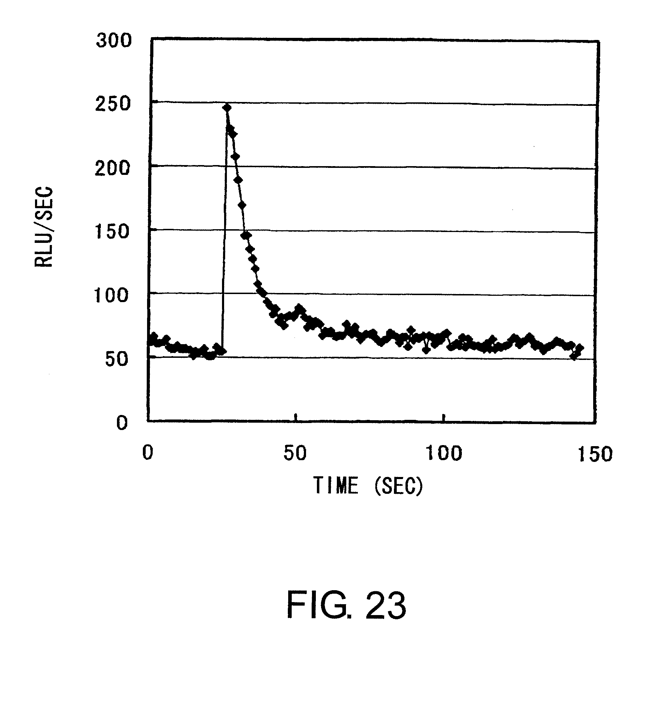

Fig. 23 shows an increase of intracellular calcium concentration in pituitary cells of the apoaequorin-expressing mice due to 1 μmol/L of Peptide 8 (SEQ ID NO: 8). The horizontal axis indicates the time (seconds) after addition of the medium, and the vertical axis indicates the relative luminescence unit (RLU) per second. Peptide 8 was added at 25 seconds.

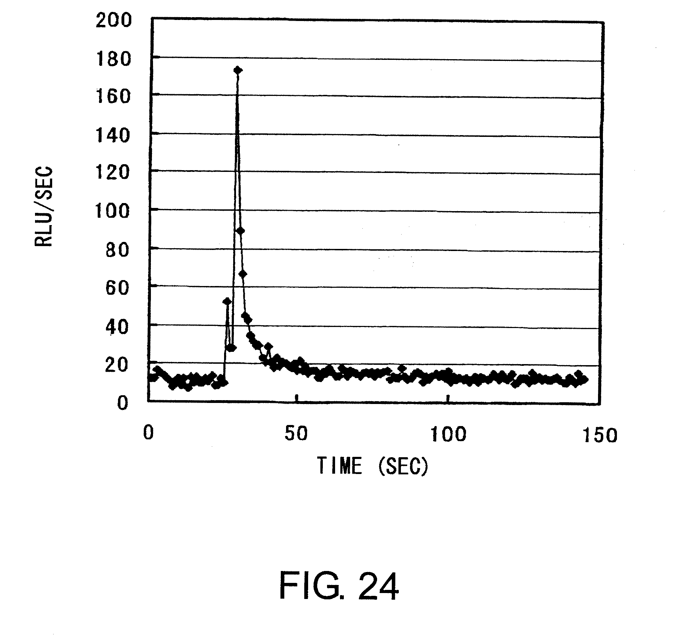

Fig. 24 shows an increase of intracellular calcium concentration in cardiac cells of the apoaequorin-expressing mice due to 5 μmol/L of Peptide 8. The horizontal axis indicates the time (seconds) after addition of the medium, and the vertical axis indicates the relative luminescence unit (RLU) per second. Peptide 8 was added at 25 seconds.

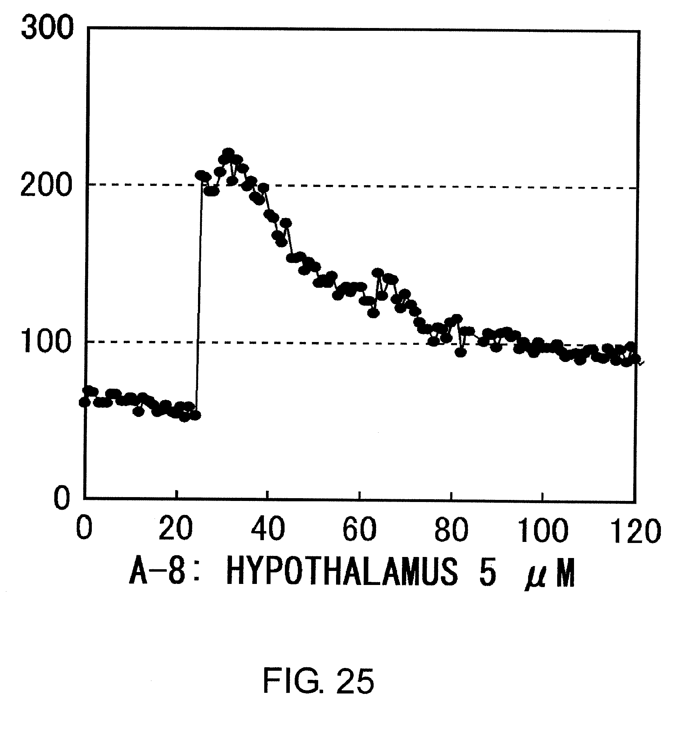

Fig. 25 shows an increase of intracellular calcium concentration in hypothalamic cells of the apoaequorin-expressing mice due to 5 μmol/L of Peptide 9 (SEQ ID NO: 23). The horizontal axis indicates the time (seconds) after addition of the medium, and the vertical axis indicates the relative luminescence unit (RLU) per second. Peptide 9 was added at 25 seconds.

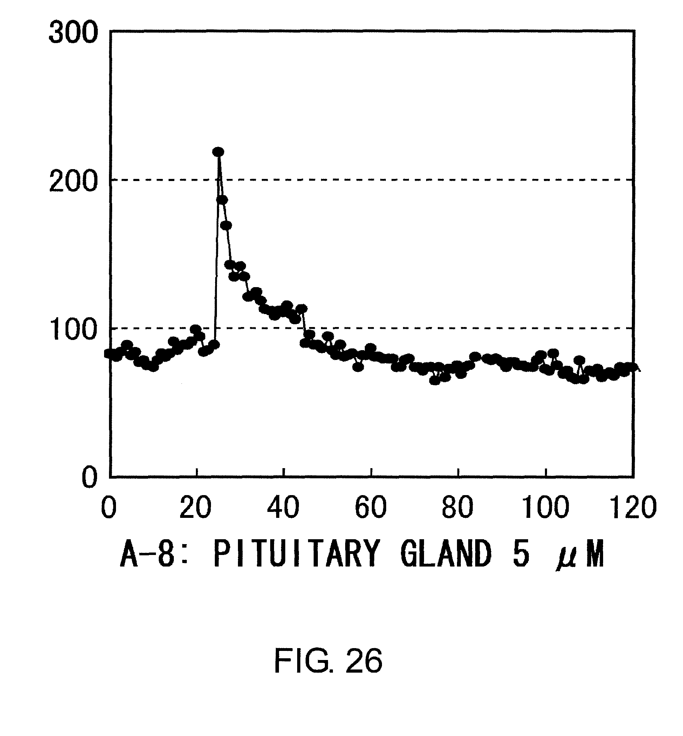

Fig. 26 shows an increase of intracellular calcium concentration in pituitary cells of the apoaequorin-expressing mice due to 5 μmol/L of Peptide 9. The horizontal axis indicates the time (seconds) after addition of the medium, and the vertical axis indicates the relative luminescence unit (RLU) per second. Peptide 9 was added at 25 seconds.

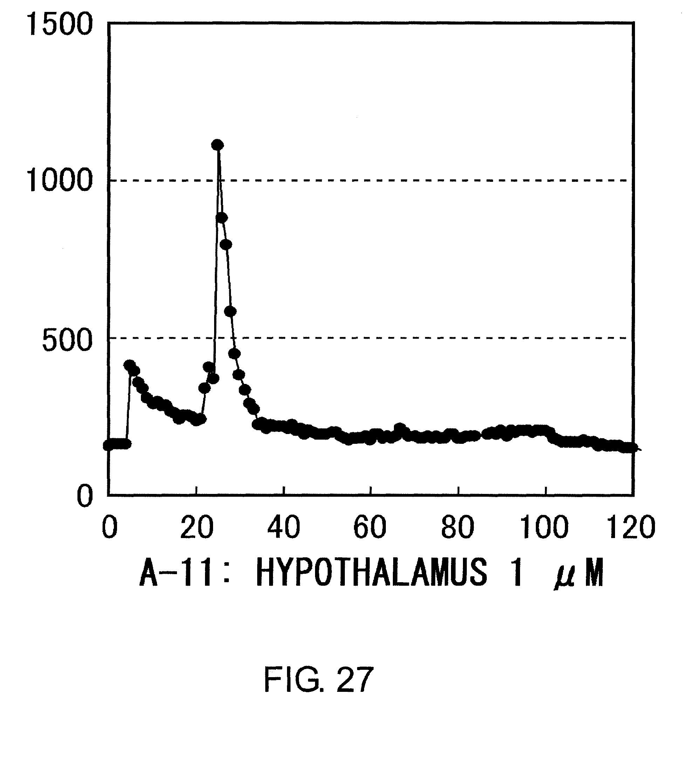

Fig. 27 shows an increase of intracellular calcium concentration in hypothalamic cells of the apoaequorin-expressing mice due to 1 μmol/L of Peptide 10 (SEQ ID NO: 24). The horizontal axis indicates the time (seconds) after addition of the medium, and the vertical axis indicates the relative luminescence unit (RLU) per second. Peptide 10 was added at 25 seconds.

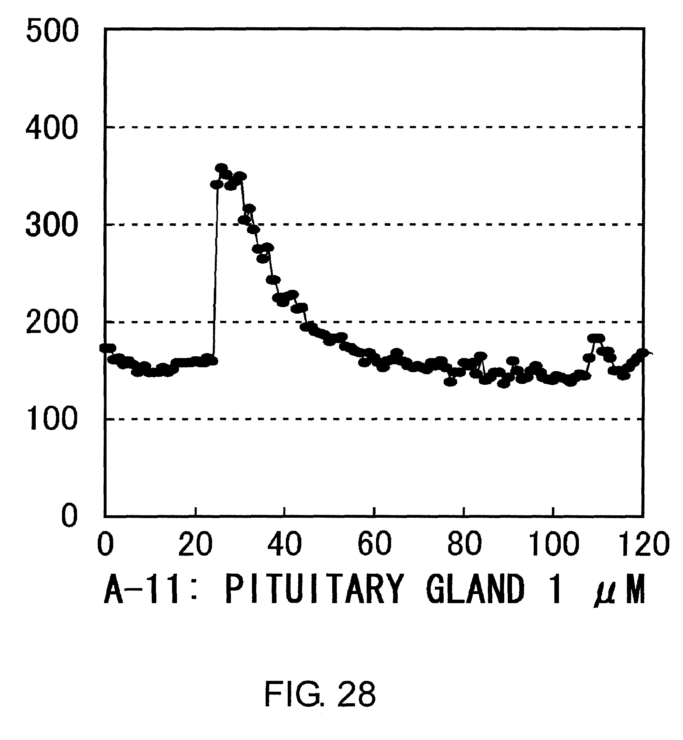

Fig. 28 shows an increase of intracellular calcium concentration in pituitary cells of the apoaequorin-expressing mice due to 1 μmol/L of Peptide 10. The horizontal axis indicates the time (seconds) after addition of the medium, and the vertical axis indicates the relative luminescence unit (RLU) per second. Peptide 10 was added at 25 seconds.

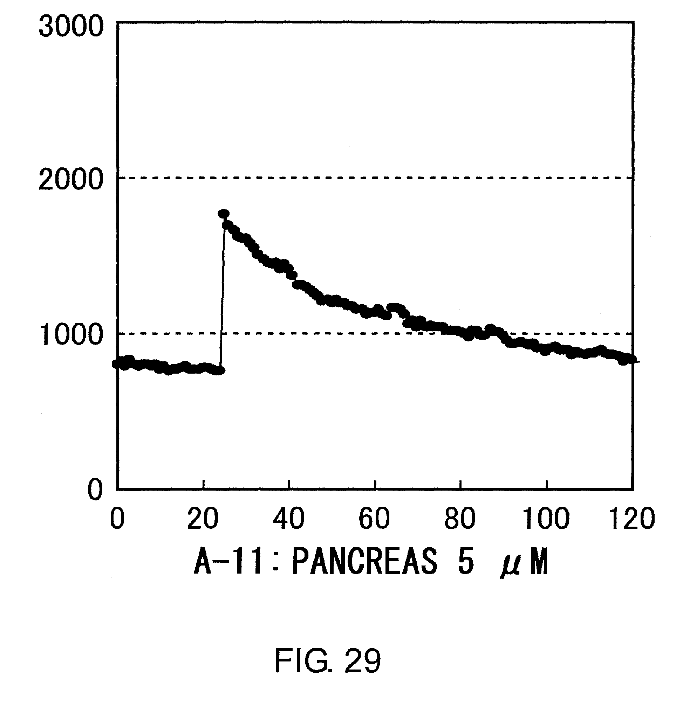

Fig. 29 shows an increase of intracellular calcium concentration in pancreatic cells of the apoaequorin-expressing mice due to 5 μmol/L of Peptide 10. The horizontal axis indicates the time (seconds) after addition of the medium, and the vertical axis indicates the relative luminescence unit (RLU) per second. Peptide 10 was added at 25 seconds.

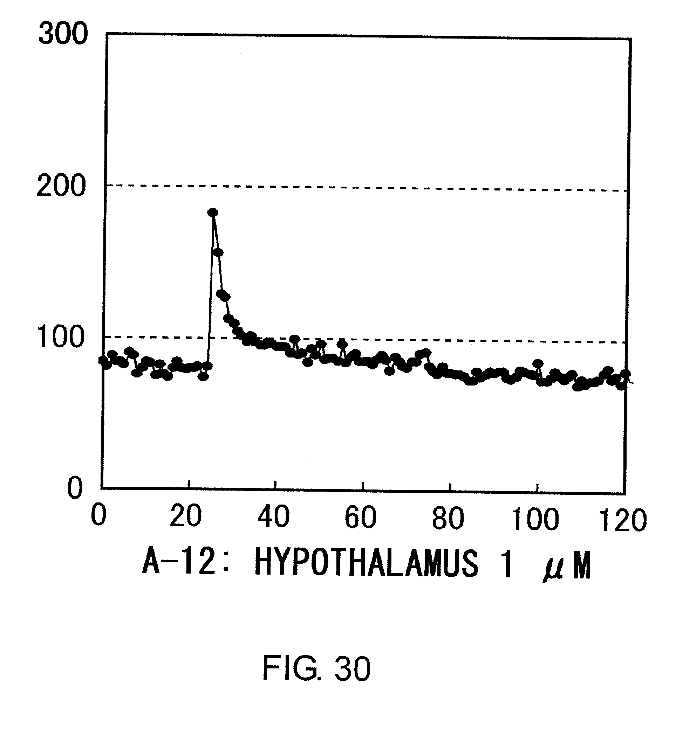

Fig. 30 shows an increase of intracellular calcium concentration in hypothalamic cells of the apoaequorin-expressing mice due to 1 μmol/L of Peptide 11 (SEQ ID NO: 25). The horizontal axis indicates the time (seconds) after addition of the medium, and the vertical axis indicates the relative luminescence unit (RLU) per second. Peptide 11 was added at 25 seconds.

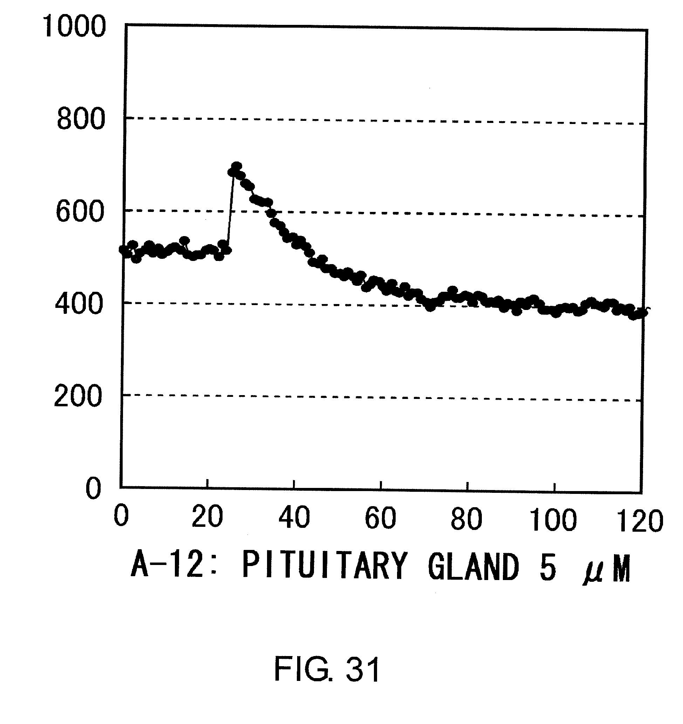

Fig. 31 shows an increase of intracellular calcium concentration in pituitary cells of the apoaequorin-expressing mice due to 5 μmol/L of Peptide 11. The horizontal axis indicates the time (seconds) after addition of the medium, and the vertical axis indicates the relative luminescence unit (RLU) per second. Peptide 11 was added at 25 seconds.

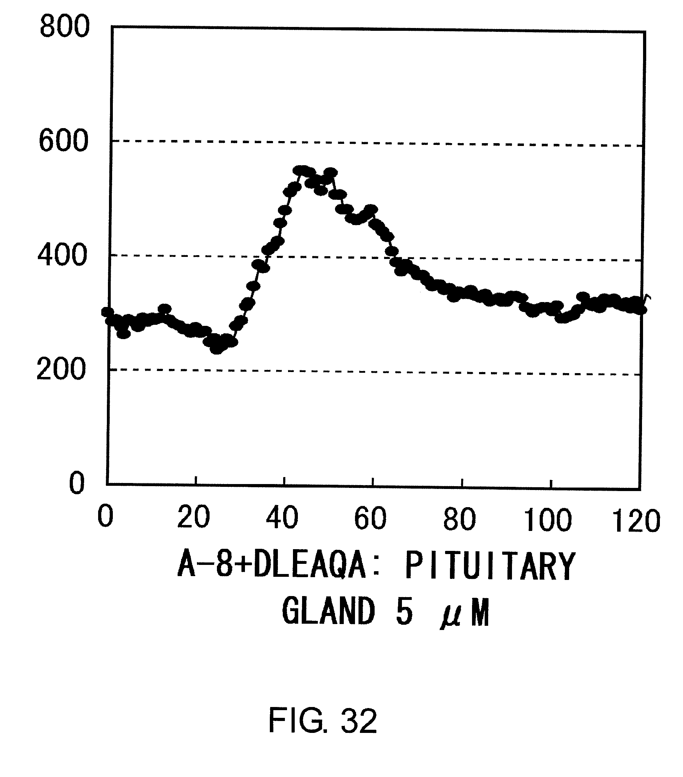

Fig. 32 shows an increase of intracellular calcium concentration in pituitary cells of the apoaequorin-expressing mice due to 5 μmol/L of Peptide 12 (SEQ ID NO: 28). The horizontal axis indicates the time (seconds) after addition of the medium, and the vertical axis indicates the relative luminescence unit (RLU) per second. Peptide 12 was added at 25 seconds.

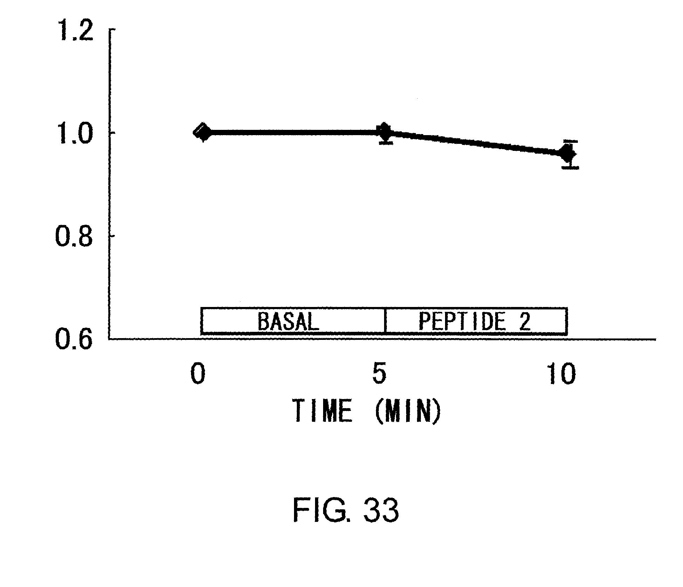

Fig. 33 shows vasopressin release from nerve endings in the posterior pituitary gland due to administration of Peptide 12 (SEQ ID NO: 28). This figure shows "mean ± standard error" determined in triplicate. The horizontal axis indicates the time (minutes), and the vertical axis indicates the rate of signal decrease after administration of Peptide 12 when the control (basal) is taken as 1.

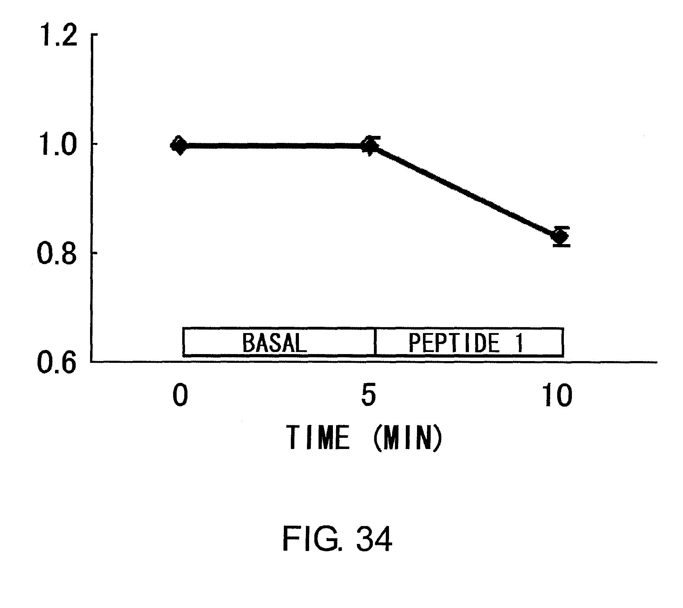

Fig. 34 shows vasopressin release from nerve endings in the posterior pituitary gland due to administration of Peptide 1 (SEQ ID NO: 1). This figure shows "mean ± standard error" determined in triplicate. The horizontal axis indicates the time (minutes), and the vertical axis indicates the rate of signal decrease after administration of Peptide 1 when the control (basal) is taken as 1.

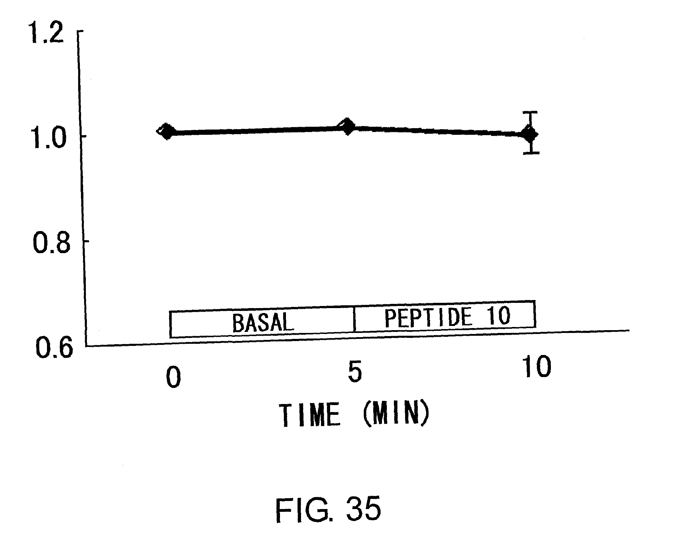

Fig. 35 shows vasopressin release from nerve endings in the posterior pituitary gland due to administration of Peptide 11 (SEQ ID NO: 25). This figure shows "man ± standard error" determined in triplicate. The horizontal axis indicates the time (minutes), and the vertical axis indicates the rate of signal decrease after administration of Peptide 11 when the control (basal) is taken as 1.

Best Mode for Carrying Out the Invention

1. Peptides of the present invention

A peptide of the present invention includes, for example, a peptide of any one of the following (a) to (f), or a pharmaceutically acceptable salt thereof.

(a) A peptide comprising the amino acid sequence of any one of SEQ ID NOS: 1 to 12, and 23 to 29. The amino acid sequence of SEQ ID NO: 1 corresponds to the amino acid sequence at position 177 to 206 in human VGF; the amino acid sequence of SEQ ID NO: 2 corresponds to the amino acid sequence at position 195 to 206 in human VGF; the amino acid sequence of SEQ ID NO: 3 corresponds to the amino acid sequence at position 485 to 495 in human VGF; the amino acid sequence of SEQ ID NO: 4 corresponds to the amino acid sequence at position 533 to 543 in human VGF; the amino acid sequence of SEQ ID NO: 5 corresponds to the amino acid sequence at position 211 to 236 in rat VGF; the amino acid sequence of SEQ ID NO: 6 corresponds to the amino acid sequence at position 353 to 372 in rat VGF; the amino acid sequence of SEQ ID NO: 7 corresponds to the amino acid sequence at position 400 to 417 in human VGF; the amino acid sequence of SEQ ID NO: 8 corresponds to the amino acid sequence at position 423 to 430 in rat VGF; the amino acid sequence of SEQ ID NO: 9 corresponds to the amino acid sequence at position 208 to 233 in human VGF; the amino acid sequence of SEQ ID NO: 10 corresponds to the amino acid sequence at position 350 to 370 in human VGF; the amino acid sequence of SEQ ID NO: 11 corresponds to the amino acid sequence at position 420 to 427 in human VGF; the amino acid sequence of SEQ ID NO: 12 corresponds to the amino acid sequence at position 535 to 546 in rat VGF; the amino acid sequence of SEQ ID NO: 23 corresponds to the amino acid sequence at position 554 to 577 in human VGF; the amino acid sequence of SEQ ID NO: 24 corresponds to the amino acid sequence at position 485 to 503 in human VGF; the amino acid sequence of SEQ ID NO: 25 corresponds to the amino acid sequence at position 533 to 552 in human VGF; the amino acid sequence of SEQ ID NO: 26 corresponds to the amino acid sequence at position 177 to 206 in human VGF; the amino acid sequence of SEQ ID NO: 27 corresponds to the amino acid sequence at position 554 to 577 in human VGF; the amino acid sequence of SEQ ID NO: 28 corresponds to the amino acid sequence at position 556 to 585 in rat VGF; and the amino acid sequence of SEQ ID NO: 29 corresponds to the amino acid sequence at position 556 to 585 in rat VGF and the amino acid sequence at position 554 to 583 in human VGF. The amino acid sequences of the peptides of (a) may comprise any number of amino acids as long as they comprise the amino acid sequence of SEQ ID NOS: 1 to 12, or 23 to 29; however, the number is preferably 80 or less, more preferably 60 or less, and especially preferably 40 or less.

(b) A peptide comprising an amino acid sequence with a substitution, deletion, or addition of one to five amino acids in the amino acid sequence shown in any of SEQ ID NOS: 1 to 12, and 23 to 29, wherein the peptide has an activity of increasing the intracellular calcium ion concentration in cells of the hypothalamus, pituitary gland, kidney, heart, blood vessel, or brain tissue. Herein, the brain tissue refers to tissues encompassed in the brain, such as cerebrum, midbrain, cerebellum, diencephalon, and medulla oblongata.

(c) A peptide comprising an amino acid sequence having 90% or higher homology to the amino acid sequence shown in any of SEQ ID NOS: 1 to 12, and 23 to 29, wherein the peptide has an activity of increasing the intracellular calcium ion concentration in cells of the hypothalamus, pituitary gland, kidney, heart, blood vessel, or brain tissue.

(d) A peptide comprising an amino acid sequence with a substitution, deletion, or addition of one to five amino acids in the amino acid sequence shown in any of SEQ ID NOS: 1, 25, 28, and 29, wherein the peptide has an activity of promoting vasopressin secretion from the posterior pituitary gland.

(e) A peptide comprising an amino acid sequence having 90% or higher homology to the amino acid sequence shown in any of SEQ ID NOS: 1, 25, 28, and 29, wherein the peptide has an activity of promoting vasopressin secretion from the posterior pituitary gland.

(f) A peptide represented by the following formula (IV)

$$R^7\text{-}D\text{-}R^8 \qquad (IV)$$

(wherein, $R^7$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^8$ represents hydroxy, substituted or unsubstituted alkoxy, or substituted or unsubstituted amino; and D represents a peptide residue of the peptide of any one of the above-mentioned (a) to (e)).

The phrase "substitution, deletion, or addition of one to five amino acids in the amino acid sequence shown in any of SEQ ID NOS: 1 to 12, and 23 to 29" means that one to five, preferably one to four, more preferably one to three, even more preferably one or two and especially preferably one amino acid substitutions, deletions, or additions are present at any of one or more positions in the same amino acid sequences, and the substitutions, deletions, or additions may occur simultaneously. Examples of amino acids that are substituted or added include the twenty L-amino acids known as essential amino acids, which are specifically, L-alanine, L-asparagine, L-aspartic acid, L-arginine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, and L-cysteine, but are not limited thereto; and for example, other amino acids such as *tert*-leucine, norleucine, norvaline, 2-aminobutanoic acid, *O*-methylserine, *t*-butylglycine, *t*-butylalanine, cyclohexylalanine, isoaspartic acid, isoglutamic acid, 2-aminoadipic acid, 2-aminosuberic acid, ornithine, 2,4-diaminobutanoic acid, 2,3-diaminopropionic acid, 3-hydroxyproline, 4-hydroxyproline, homoserine, D-amino acids and β-amino acids may be included.

Examples of amino acid residues that can be mutually substituted are shown below. Amino acid residues included in the same group can be mutually substituted.

Group A: leucine, isoleucine, norleucine, valine, norvaline, alanine, 2-aminobutanoic acid, methionine, *O*-methylserine, *t*-butylglycine, *t*-butylalanine, cyclohexylalanine, *tert*-leucine
Group B: aspartic acid, glutamic acid, isoaspartic acid, isoglutamic acid, 2-aminoadipic acid, 2-aminosuberic acid
Group C: asparagine, glutamine
Group D: lysine, arginine, ornithine, 2,4-diaminobutanoic acid, 2,3-diaminopropionic acid Group E: proline, 3-hydroxyproline, 4-hydroxyproline
Group F: serine, threonine, homoserine
Group G: phenylalanine, tyrosine

The phrase "amino acid sequence having 90% or higher homology to the amino acid sequence shown in any of SEQ ID NOS: 1 to 12, and 23 to 29" refers to an amino acid sequence having 90% or higher, preferably 92% or higher, more preferably 95% or higher, even more preferably 96% or higher, and especially preferably 98% or higher homology (the number of identical amino acids between the sequence of interest and the sequence of any one of SEQ ID NOS: 1 to 12 with which homology analysis was performed/ (total number of amino acids of the sequence of any one of SEQ ID NOS: 1 to 12, and 23 to 29 with which homology analysis was performed + number of gaps inserted during the alignment)) when alignment is performed by calculation using a homology analysis program BLAST 2 Sequences (FEMS Microbiol Lett. 174, 247 (1999)) under default settings (program: blastp; matrix: BLOSUM62; open gap: 11 penalties; extension gap: 1 penalty; gap x_dropoff: 50; expect: 10.0; word size: 3).

In the definition of each group of the above-mentioned formulas (I) to (IV), examples of alkanoyl include a straight chain or branched chain alkanoyl comprising one to twenty carbon atoms, such as formyl, acetyl, propionyl, butyryl, isobutyryl, valeryl, isovaleryl, pivaloyl, hexanoyl, heptanoyl, lauroyl, eicosanoyl, and the like.

Examples of the aryl moiety of aroyl and aryloxycarbonyl include phenyl, naphthyl, and the like, and comprise 6 to 15 carbons.

Examples of the heteroaryl moiety of heteroarylcarbonyl and heteroaryloxycarbonyl include furyl, thienyl, pyrrolyl, pyrazolyl, imidazolyl, oxazolyl, thiazolyl, pyridyl, pyridazinyl, pyrimidinyl, pyrazinyl, indolyl, indazolyl, benzimidazolyl, quinolyl, isoquinolyl, cinnolinyl, quinazolynyl, quinoxalinyl, naphthylidinyl, and the like.

Examples of the alkyl moiety of alkoxycarbonyl and alkoxy include a straight chain or branched chain alkyl moiety of one to twenty carbons such as methyl, ethyl, propyl, isopropyl, butyl, pentyl, hexyl, heptyl, decyl, dodecyl, eicosyl, and the like.

Examples of the substituents of the substituted alkanoyl, substituted alkoxycarbonyl and substituted alkoxy, which may be the same or different and in number of 1 to 3, include hydroxy; carboxy; aliphatic cyclic alkyl of three to eight carbons including cyclopropyl, cyclobutyl, cyclopentyl, cyclohexyl, cycloheptyl, cyclooctyl and the like; substituted and unsubstituted phenyl; substituted and unsubstituted fluorenyl, and the like. Examples of the substituents of the substituted phenyl, which may be the same or different and in number of 1 to 3, include alkyl, alkoxy, hydroxy, nitro, sulfo, cyano, halogen, and the like, and examples of the halogen include each of fluorine, chlorine, bromine, and iodine atoms. The alkyl moiety of alkyl and alkoxy serving as substituents of the substituted phenyl has the same meaning as the alkyl moiety of the aforementioned alkoxycarbonyl and alkoxy.

There are one to three same or different substituents in the substituted aroyl, substituted aryloxycarbonyl, substituted heteroarylcarbonyl and substituted heteroaryloxycarbonyl; and they have the same meanings as the above-mentioned substituents of the substituted phenyl.

One to two substituents in the substituted amino which are the same or different include, for example, substituted or unsubstituted alkyl, substituted or unsubstituted aryl, or the like. Alkyl has the same meaning as the aforementioned alkyl moiety of alkoxy or the like, and substituents of the substituted alkyl have the same meaning as the aforementioned substituents of the substituted alkoxy or the like. Aryl has the same meaning as the aforementioned aryl moiety of aroyl or aryloxycarbonyl, and the substituents of the substituted aryl have the same meaning as the aforementioned substituents of aroyl or aryloxycarbonyl.

The functional groups of the side chains of amino acid residues constituting C may be chemically modified or protected. Examples of such amino acid residues whose side-chain functional groups are chemically modified or protected are aspartic acid and glutamic acid residues whose side-chain carboxyl group is protected by a benzyl ester, cysteine residue whose side-chain thiol group has been carboxymethylated, or the like.

Examples of pharmaceutically acceptable salts are acid addition salts, metal salts, organic base addition salts, and the like. Examples of acid addition salts include inorganic acid salts such as hydrochloride, sulfate, phosphate, and the like; and organic acid salts such as acetate, maleate, fumarate, tartarate, citrate, and the like. Examples of metal salts include alkali metal salts such as sodium salt, potassium salt, and the like; alkaline earth metal salts such as magnesium salt, calcium salt, and the like, aluminum salt, zinc salt, and the like. Examples of organic base addition salts include salts formed with primary amines such as methyl amine, ethyl amine, aniline, and the like; secondary amines such as dimethyl amine, diethyl amine, pyrrolidine, piperidine, morpholine, piperazine, and the like; and tertiary amines such as trimethylamine, triethylamine, *N,N*-dimethylaniline, pyridine, and the like; ammonium salt, and the like.

2. Methods for producing the peptides of the present invention

(1) Chemical synthetic production method

Peptides of the present invention can be obtained by synthesis, followed by purification, using general peptide synthesis methods described in, for example, Izumiya, N., Kato, T., et al., "Fundamentals and Experiments of Peptide Synthesis (Peptide Gosei no Kiso to Jikken)", Maruzen, (1985); Aimoto, S. et al., "Experimental Chemical Course (Jikken Kagaku Koza)", ed. 4, vol. 22, "Organic Synthesis (Yuki Gosei) IV, Acid, Amino acid and Peptide", Maruzen, (1999); Int. J. Pept. Protein Res. 35, 161-214 (1990); Fields, G. B., Solid-Phase Peptide Synthesis, Methods in Enzymology, vol. 289, Academic Press, (1997); Pennington, M. W. and Dunn, B. M., Peptide Synthesis Protocols, Methods in Molecular Biology, vol. 35, Humana Press, (1994), and the like. Specific methods of synthesis include an azide method, acid chloride method, acid anhydride method, mixed acid anhydride method, dichloromethane method, active ester method, carboimidazole method, oxidation-reduction method, and the like. Furthermore, both solid-phase synthesis methods and liquid-phase synthesis methods can be applied to such synthesis. More specifically, a peptide of interest can be synthesized by condensing amino acids constituting a peptide of the present invention with a residual moiety, and by removing protecting groups when the product has a protecting group.

Furthermore, when the side chain of the amino acid residue constituting the peptide, the peptide's amino terminus, and/or the peptide's carboxy terminus is chemically modified or protected, peptides of the present invention can be produced by methods conventionally known in the field of peptide synthetic chemistry, such as methods of chemical modification after peptide synthesis, methods of peptide synthesis using a chemically modified amino acid, methods of appropriately selecting reaction conditions for the final deprotection in peptide synthesis, or the like (Izumiya, N., et al., "Fundamentals and Experiments of Peptide Synthesis (Peptide Gosei no Kiso to Jikken)", Maruzen, 1985; Yajima, H. ed, "The sequel of Development of Pharmaceuticals (Zoku Iyakuhin no Kaihatsu)", vol. 14, Peptide Synthesis, Hirokawa Shoten, 1991; The Japanese Biochemical Society ed., "Biochemistry Experimental Course (Seikagaku Jikken Koza)", vol. 1, "Chemistry of Protein IV-Chemical Modification and Peptide Synthesis", Tokyo Kagaku Dojin; and Ohno, M., et al., "Experimental Methods in Biological Chemistry (Seibutsukagaku Jikkenho)" vols. 12 and 13, "Chemical Modification of Proteins (Tanpakushitsu no Kagaku Shushoku), I and II", Japan Scientific Societies Press, 1981).

In addition, the peptides of the present invention can be synthesized by an automated peptide synthesizer.

The synthesis of the peptides by use of a peptide synthesizer is carried out, using amino acids with appropriately protected side chains, such as N α-Fmoc (9-fluorenylmethyloxycarbonyl)-amino acids, N α-Boc (*t*-butyloxycarbonyl)-amino acids, and the like, on a commercially available peptide synthesizer, for example, a peptide synthesizer manufactured by Shimadzu Corporation, a peptide synthesizer manufactured by Advanced ChemTech Inc., or the like, according to the respective synthesis programs. Protected amino acids and carrier resins used as source materials are available from Applied Biosystems, Shimadzu Corporation, Kokusan Kagaku (Kokusan Chemical Co., Ltd), EMD Biosciences, Inc., Watanabe Kagaku (Watanabe Chemical Industries, Ltd), Advanced ChemTech, Ana Spec, Inc., Peptide Institute, Inc, and the like.

The peptides of the present invention can be purified by combining general purification methods such as solvent extraction, distillation, column chromatography, liquid chromatography, recrystallization, and the like.

(2) Methods of production using genetic engineering techniques

When a peptide of the present invention comprises the aforementioned 20 essential amino acids, and if its side chain, N terminus, or C terminus is not modified, it can be produced by methods described in Molecular Cloning: A Laboratory Manual, 3rd edition, Cold Spring Harbor Laboratory Press (2001), or the like. For example, the peptides can be produced by expressing a DNA encoding a peptide of the present invention in a host cell by the following method.

DNAs encoding the peptides of the present invention can be synthesized using a DNA synthesizer by designing nucleotide sequences coding the amino acid sequences of the peptides of the present invention. When the peptides of the present invention are partial peptides of human VGF comprising the sequence shown in any of SEQ ID NOS: 1 to 12, they can be isolated by PCR using cDNAs of human brain cells or pancreatic cells as template. When DNAs encoding the peptides of the present invention are used to produce the peptides, stop codons are placed at the terminal ends of the regions encoding the peptides.

A method for producing the peptides of the present invention when the N terminus is a methionine is described below.

A recombinant vector is prepared by inserting a DNA encoding a peptide of the present invention obtained as described above to the downstream of a promoter of a suitable expression vector, and the recombinant vector is introduced into a host cell that is appropriate for the expression vector.

For example, any bacteria, yeasts, animal cells, insect cells, plant cells, and the like can be used as host cells so long as they can express the gene of interest.

Expression vectors that are used are those that can replicate autonomously in the above-mentioned host cells or can be integrated into a chromosome, and which contain a promoter at a position where the DNA encoding the peptide of the present invention can be transcribed.

When prokaryotes, such as bacteria or the like, are used as host cells, it is preferred that the recombinant vectors comprising the DNAs encoding the peptides of the present invention can replicate autonomously in prokaryotes, and at the same time, that the vectors are composed of a promoter, a ribosome-binding sequence, a DNA of the present invention, and a transcription termination sequence. A gene regulating the promoter may also be included.

Examples of the expression vectors are pSE420 (Invitrogen), pGEMEX-1 (Promega), pQE-30 (QIAGEN), pKYP10 (Japanese Published Unexamined Patent Application No. 110600/83), pKYP200 (Agric. Biol. Chem., 48, 669 (1984)), pLSA1 (Agric. Biol. Chem., 53, 277 (1989)), pGEL1 (Proc. Natl. Acad. Sci., USA, 82, 4306 (1985)), pBluescript II SK(-) (Stratagene), pTrs30 (prepared from transformed E. *coli* cell line (FERM BP-5407)), pTrs32 (prepared from transformed *E. coli* cell line (FERM BP-5408)), pGHA2 (prepared from transformed *E. coli* cell line (FERM BP-400)), pGKA2 (prepared from transformed E. *coli* cell line (FERM BP-6798)), pTerm2 (Japanese Published Unexamined Patent Application No. 22979/91), pGEX-2T (GE Healthcare), pET (Novagen), pKK223-2 (GE Healthcare), pMAL-c2X (New England Biolabs), and the like.

Any promoter can be used, so long as it can function in the host cells. Examples include promoters derived from *E. coli*, phage, and the like, such as trp promoter ($P_{trp}$), lac promoter, $P_L$ promoter, $P_R$ promoter, T7 promoter, and the like. In addition, artificially designed and modified promoters, such as a promoter in which two $P_{trp}$'s are linked in tandem ($P_{trp}$x2), tac promoter, lacT7 promoter, letI promoter, and the like, can be used.

It is preferred to use a plasmid in which the distances between the Shine-Dalgarno sequence which is ribosome binding sequence, and initiation codon are appropriately adjusted (for example, 6 to 18 bases).

In the recombinant vector of the present invention, a transcription termination sequence is not always necessary for the expression of the DNA of the present invention; however, it is preferred to place the transcription termination sequence immediately downstream of the structural gene.

Examples of host cells include microorganisms belonging to the genera *Escherichia, Serratia, Bacillus, Brevibacterium, Corynebacterium, Microbacterium, Pseudomonas*, and the like, such as *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia*

*coli* TB1, *Serratia ficaria, Serratia fonticola, Serratia liquefaciens, Serratia marcescens, Bacillus subtilis, Bacillus amyloliquefaciens, Brevibacterium ammoniagenes, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium glutamicum* ATCC13869, *Corynebacterium acetoacidophilum* ATCC 13870, *Microbacterium ammoniaphilum* ATCC 15354, *Pseudomonas putida, Pseudomonas* sp. D-0110, and the like.

As the method for introducing the recombinant DNAs, any method for introducing a DNA into the above-mentioned host cells can be used, and examples include methods using calcium ion (Proc. Natl. Acad. Sci. USA, 69, 2110 (1972)), protoplast methods (Japanese Published Unexamined Patent Application No. 2483942/88), methods described in Gene, 17, 107 (1982) and Molecular & General Genetics, 168, 111 (1979), and the like.

When yeasts are used as host cells, the expression vector may be, for example, YEp13 (ATCC37115), YEp24 (ATCC37051), YCp50 (ATCC37419), pHS19, pHS15, or the like.

Any promoter can be used, so long as it can be expressed in a yeast cell line; and examples include promoters of genes in the glycolytic pathway such as hexose kinase and the like, PHO5 promoter, PGK promoter, GAP promoter, ADH promoter, gal 1 promoter, gal 10 promoter, a heat shock polypeptide promoter, MF $\alpha$1 promoter, CUP 1 promoter, and the like.

The host cells include microorganisms belonging to the genera *Saccharomyces, Schizosaccharomyces, Kluyveromyces, Trichosporon, Schwanniomyces, Pichia, Candida* and the like, and examples include *Saccharomyces cerevisiae, Schizosaccharomyces pombe, Kluyveromyces lactis, Trichosporon pullulans, Schwanniomyces alluvius, Candida utilis*, and the like.

Any method for introducing a recombinant vector into yeast host cells can be used, so long as it ensures the introduction of DNA into yeast. Such methods include, for example, electroporation (Methods. in Enzymol., 194, 182 (1990)), the spheroplast method (Proc. Natl. Acad. Sci. USA, 75, 1929 (1978)), the lithium acetate method (J. Bacteriology., 153, 163 (1983)), and the method described in Proc. Natl. Acad. Sci. USA, 75, 1929 (1978).

When an animal cell is used as the host cell, suitable expression vectors include, for example, pcDNA3.1(+) (Invitrogen), pAGE107 (Japanese Published Unexamined Patent Application No. 22979/91; Cytotechnology, 3, 133 (1990)), pAS3-3 (Japanese Published Unexamined Patent Application No. 227075/90), pCDM8 (Nature, 329, 840 (1987)), pREP4 (Invitrogen), pAGE103 (J. Biochem., 101, 1307 (1987)) and the like.

Any promoter can be used, so long as it functions in the animal cells. Such promoters include, for example, the promoter of the IE (immediate early) gene of cytomegalovirus (CMV), SV 40 early promoter, retroviral promoter, metallothionein promoter, heat-shock promoter, and SR$\alpha$ promoter. Further, the enhancer of the IE gene of human CMV may be used in combination with a promoter.

The host cells to be used in the present invention include Namalwa cell, a human cell line; COS cell, derived from monkey; CHO cell, derived from Chinese hamster; HBT5637 (Japanese Published Unexamined Patent Application No. 299/88) and the like.

Any of the methods for introducing a recombinant vector into animal host cells can be used as long as it ensures the introduction of a DNA into animal cells. Such methods include, for example, electroporation (Cytotechnology, 3, 133 (1990)), the calcium phosphate method (Japanese Published Unexamined Patent Application No. 227075/90), and the lipofection method (Proc. Natl. Acad. Sci. USA, 84, 7413 (1987); Virology, 52, 456 (1973)).

When insect cells are used as host cells, a peptide can be expressed, for example, by the method described in Current Protocols in Molecular Biology; Baculovirus Expression Vectors, A Laboratory Manual, W.H. Freeman and Company, New York (1992); Bio/Technology, 6, 47 (1988), or the like.

More specifically, a vector for introducing a recombinant gene and a genome deficient baculovirus are co-transfected into insect cells to obtain a recombinant virus in the supernatant of an insect cell culture, and then the insect cells are infected with the recombinant virus to express the peptides.

Gene introducing vectors used in this method include, for example, pVL1392, pVL1393 (Becton, Dickinson and Company), pBlueBac4.5 (Invitrogen), and the like.

Baculoviruses that can be used in the present invention include, for example, *Autographa californica*, a nuclear polyhedrosis virus that is infectious to insects belonging to the family of Cabbage armyworm.

Insect cell that can be used in the present invention include, for example, Sf9 and Sf21 both of which are ovarian cells of *Spodoptera frugiperda* (Baculovirus Expression Vectors, A Laboratory Manual, W. H. Freeman and Company, New York, (1992)); and High5 (Invitrogen) that is an ovarian cell of *Trichoplusia ni*; and the like.

Methods for co-introducing the above-mentioned recombinant gene introducing vector and the baculovirus into insect cells to prepare recombinant viruses include, for example, the calcium phosphate method (Japanese Published Unexamined Patent Application No. 227075/90) and the lipofection method (Proc. Natl. Acad. Sci. USA, 84, 7413 (1987)).

When plant cells are used as host cells, the expression vector includes, for example, Ti plasmid, the tobacco mosaic virus vector, and the like.

Any promoter can be used, so long as it can be expressed in a plant cell, and examples include the 35S promoter of cauliflower mosaic virus, rice actin 1 promoter, and the like.

The host cells include, for example, plant cells and the like of tobacco, potato, tomato, carrot, soybean, rapeseed, alfalfa, rice, wheat, barley, and the like.

Any method for introducing the recombinant vector can be used, so long as it is a method for introducing a DNA into plant cells, and examples include the *Agrobacterium* method (Japanese Published Unexamined Patent Application No. 140885/84, Japanese Published Unexamined Patent Application No. 70080/85, WO94/00977), electroporation method (Japanese Published Unexamined Patent Application No. 251887/85), particle gun method (Granted/ Registered Japanese Patents 2606856 and 2517813), and the like.

A peptide of the present invention can be produced by culturing a transformant of the present invention which is obtained as described above in a medium to produce and accumulate the peptide of the present invention in the culture, and recovering it from the same.

The method for culturing the transformant of the present invention in a medium can be carried out according to the general methods that are used in culturing the host.

When a transformant of the present invention is obtained by using a prokaryote such as *Escherichia coli* or an eukaryote such as yeast as a host, the medium used for culturing may be either a natural medium or a synthetic medium, so long as it contains a carbon source, a nitrogen source, inorganic salts, and the like, and these can be assimilated by the transformant so that the transformant can be cultured efficiently.

Any carbon source can be used, so long as it can be assimilated by the transformant, and examples include carbohydrates such as glucose, fructose, sucrose, molasses containing them, starch, starch hydrolysate, and the like; organic acids such as acetic acid, propionic acid, and the like; alcohols such as ethanol, propanol, and the like.

The nitrogen source includes ammonia, ammonium salts of inorganic acids or organic acids such as ammonium chloride, ammonium sulfate, ammonium acetate, ammonium phosphate, and the like, other nitrogen-containing compounds, peptone, meat extract, yeast extract, corn steep liquor, casein hydrolysate, soybean meal and soybean meal hydrolysate, various fermenting microbial cells and the digest thereof, and the like.

The inorganic salts that can be used are, for example, monopotassium phosphate, dipotassium phosphate, magnesium phosphate, magnesium sulfate, sodium chloride, ferrous sulfate, manganese sulfate, copper sulfate, calcium carbonate, and the like.

Culturing is preferably carried out under aerobic conditions by shaking culture, submerged spinner culture under aeration, or the like. The culturing temperature is preferably 15°C to 40°C, and the preferred culturing time is generally 16 hours to 7 days. The pH is preferably maintained at 3.0 to 9.0 during culturing. The pH can be adjusted by using an inorganic or organic acid, an alkali solution, urea, calcium carbonate, ammonia, or the like.

Also, antibiotics such as ampicillin, tetracycline, and the like can be added to the medium during culturing, if necessary.

When culturing a microorganism transformed with a recombinant vector that uses an inducible promoter as a promoter, an inducer can be added to the medium, if necessary. For example, isopropyl-β-D-thiogalactopyranoside or the like can be added to the medium when culturing a microorganism transformed with a recombinant vector having a *lac* promoter, or indoleacrylic acid or the like can be added to the medium when culturing a microorganism transformed with a recombinant vector having a *trp* promoter.

The medium for culturing a transformant obtained using animal cells as host includes RPMI 1640 medium (The Journal of the American Medical Association, 199, 519 (1967)), Eagle's minimum essential medium (MEM) (Science, 122, 501 (1952)), modified Dulbecco's Eagle medium (Virology, 8, 396 (1959)), 199 Medium (Proceeding of the Society for the Biological Medicine, 73, 1 (1950)), and the above media with added fetal calf serum or the like.

Generally, culturing is preferably carried out in the presence of 5% $CO_2$ at pH 6 to 8 and at a temperature of 30°C to 40°C for one to seven days.

Furthermore, antibiotics such as kanamycin, penicillin, and the like can be added to the medium while culturing, if necessary.

The level of production can be increased using a gene amplification system that uses a dihydrofolate reductase gene, or the like according to the method described in the Japanese Published Unexamined Patent Application No. 227075/90.

The medium used for culturing a transformant obtained using insect cells as host includes generally used medium, such as TNM-FH medium (Becton Dickinson), Sf-900 II SFM medium (Invitrogen), ExCell 400 and ExCell 405 (both manufactured by JRH Biosciences), Grace's Insect Medium (Nature, 195, 788 (1962)), or the like.

Generally, culturing is preferably carried out at pH 6 to 7 and at a temperature of 25°C to 30°C for one to five days.

Furthermore, antibiotics such as gentamicin and the like may be added to the medium while culturing, if necessary.

A transformant obtained using plant cells as host can be cultured as cells or cultured after having differentiated into plant cells or organs. The medium used for culturing the transformant includes generally used medium, such as Murashige and Skoog medium, White medium, and the above media with added plant hormones such as auxin, cytokinine, or the like.

Generally, culturing is preferably carried out at pH 5 to 9 and at a temperature of 20°C to 40°C for three to 60 days.

Furthermore, antibiotics such as kanamycin, hygromycin, and the like can be added to the medium while culturing, if necessary.

As described above, the peptides of the present invention can be produced by culturing a transformant derived from a microorganism, an animal cell, or a plant cell containing a recombinant vector into which a DNA encoding the peptide of the present invention has been incorporated, to form and accumulate the peptide according to general culturing methods, and by collecting the peptide from culture.

The peptides of the present invention can be produced by preparing a fusion protein between any polypeptide (hereinafter referred to as polypeptide X) and a peptide of the present invention, and then isolating the peptide of the present invention from the fusion protein so as to avoid being degraded in the host cell. An expression vector that expresses the fusion protein can be prepared by adding a DNA encoding methionine or a specific protease recognition sequence to the 5'-end of the above-mentioned DNA encoding the peptide of the present invention, and then ligating it in frame with a DNA encoding polypeptide X in a polypeptide X expression vector. However, a methionine-encoding DNA is added only when the peptide of the present invention does not contain methionine. Any polypeptide may be used as polypeptide X, and examples include glutathione S-transferase, maltose binding protein, DsbA, DsbC, protein A, and the like. Examples of a specific protease recognition sequence are factor Xa recognition sequence (Ile-Glu-Gly-Arg), enterokinase recognition sequence (Asp-Asp-Asp-Asp-Lys), and the like. The polypeptide X expression vector can be prepared similarly to the above-mentioned expression vector for the peptide of the present invention, by inserting a DNA encoding polypeptide X instead of a DNA encoding the peptide of the present invention. Commercially available vectors for expressing a fusion protein, for example pGEX-3 vector for expressing a fusion protein with glutathione S-transferase (GE Healthcare), pMAL-c2X and pMAL-p2E vectors for expressing a fusion protein with a maltose binding protein (New England BioLabs), pET-39b(+) vector for expressing a fusion protein with DsbA (EMD Biosciences), or the like can also be used. If the peptide of the present invention is fused to polypeptide X via a specific protease recognition sequence, the peptide of the present invention can be cleaved from the fusion protein by treatment with a corresponding protease for the recognition sequence. If the peptide of the present invention is fused to the C terminus of polypeptide X via methionine, the peptide of the present invention can be cleaved from the fusion protein by cyanogen bromide treatment according to the method described in Japanese Published Unexamined Patent Application No. 102096/89. Subsequent to the treatment with protease or cyanogen bromide, the peptide of the present invention can be isolated and purified by combining gel filtration, reverse-phase HPLC, affinity chromatography, and the like.

The peptide of the present invention can be produced by adding a DNA encoding the signal peptide of a secretory protein to the 5' end of a DNA encoding the peptide of the present invention, using this DNA to prepare a recombinant vector in the same manner as described above, and transfecting a host cell with the vector and allowing secretion of the polypeptide into the medium as described in the following literature (J. Biol. Chem., 264, 17619 (1989); Proc. Natl. Acad. Sci., USA, 86, 8227 (1989); Genes Develop., 4, 1288 (1990); Japanese Published Unexamined Patent Application No. 336963/93; WO94/23021).

If the N terminus of the peptide of the present invention is not a methionine, the peptide can be produced by a method of producing the above-mentioned fusion protein and isolating or secreting the peptide into medium. For example, the peptide of the present invention comprising the amino acid sequence shown in SEQ ID NO: 9 is prepared as follows. First, a DNA comprising the nucleotide sequence shown in SEQ ID NO: 22 and a DNA comprising a nucleotide sequence that is complementary to the sequence of SEQ ID NO: 22 are chemically synthesized in a DNA synthesizer, and then the two are annealed to prepare a double-stranded DNA. The double-stranded DNA and an XmnI-cleaved pMAL-c2X are ligated to produce a plasmid in which the double-stranded DNA is inserted into the XmnI site of pMAL-c2X. The obtained plasmid encodes a fusion protein, in which a peptide comprising a factor Xa recognition sequence (Ile-Glu-Gly-Arg) and the amino acid sequence shown in SEQ ID NO: 1 is fused at the C terminus of the maltose binding protein. *Escherichia coli* is transformed using the obtained plasmid. The obtained transformant is cultured in a medium, and the fusion protein is expressed in the transformed cells. The cultured bacterial cells are isolated by centrifugation and disrupted, and a solution containing the fusion protein is obtained. The fusion protein is isolated from the obtained solution by affinity chromatography using a maltose-immobilized column, and then the fusion protein is treated with factor Xa to excise the peptide comprising the amino acid sequence shown in SEQ ID NO: 1 from the fusion protein. The peptide comprising the amino acid sequence shown in SEQ ID NO: 1 can be isolated and purified by gel filtration, reverse phase HPLC, or the like.

General methods for isolating and purifying proteins can be used to isolate and purify peptides produced from transformants of the present invention.

For example, when the peptide of the present invention is expressed in a soluble form in cells, the cells are collected by centrifugation upon completion of culturing, suspended in an aqueous buffer, and disrupted using an ultrasonicator, a French press, a Manton Gaulin homogenizer, a Dynomill, or the like to obtain a cell-free extract. A purified product can be obtained from the supernatant obtained by centrifuging the cell-free extract by general methods used for isolating and purifying a protein. More specifically, such methods can be used alone or in combination, and include solvent extraction, salting-out using ammonium sulfate or the like, desalting, precipitation using an organic solvent, anion exchange chromatography using resin, such as diethylaminoethyl (DEAE)-Sepharose, DIAION HPA-75 (Mitsubishi Chemical), or the like, cation exchange chromatography using resin, such as S-Sepharose FF (Pharmacia) or the like, hydrophobic chromatography using resin, such as butyl sepharose, phenyl sepharose, or the like, gel filtration using a molecular sieve, affinity chromatography, chromatofocusing, and electrophoresis such as isoelectronic focusing or the like.

If the peptide is expressed in an insoluble form in cells, the cells are collected in the same manner, and then disrupted and centrifuged to recover the insoluble form of the peptide as a precipitated fraction. The collected insoluble form of the peptide is solubilized with a protein denaturing agent. The solubilized solution is diluted or dialyzed to reconstitute the normal tertiary structure of the peptide by lowering the concentration of the protein-denaturing agent in the solubilized solution. Subsequent to this procedure, a purified product of the peptide can be obtained by the same purification and isolation method described above.

If the peptide of the present invention is secreted extracellularly, the peptide can be collected in the culture supernatant. Specifically, the culture supernatant is obtained by treating the culture in the same method described above, such as centrifugation or the like, and a purified product can be obtained from the culture supernatant using the same purification and isolation method described above.

3. Antibodies that specifically bind to the peptides of the present invention

Antibodies of the present invention bind to an epitope present in the amino acid sequence shown in SEQ ID NO: 1, and can bind specifically to peptides shown in SEQ ID NOS: 1 and 2. The antibodies of the present invention may be polyclonal antibodies or monoclonal antibodies. Antibodies of the present invention include antibody fragments such as Fab, Fab', F(ab')$_2$ prepared from polyclonal antibodies or monoclonal antibodies. The monoclonal antibodies include humanized chimeric antibodies comprising a constant region of a human antibody and a variable region of a monoclonal antibody produced in a non-human animal, and humanized CDR-grafted antibodies comprising a human antibody constant region and a variable region with complementarity-determining regions (CDRs) of a monoclonal antibody produced in a non-human animal inserted into a human framework region.

(1) Production of polyclonal antibodies

Polyclonal antibodies that bind to an epitope present in the amino acid sequence shown in SEQ ID NO: 1 can be prepared as follows. A peptide antigen comprising a portion of the amino acid sequence shown in SEQ ID NO: 1 is intradermally, intravenously, intraperitoneally or intramuscularly administered to a non-human animal. These polyclonal antibodies can bind specifically to the peptides of the present invention. In this case, it is desirable to covalently bind the antigenic peptide to a carrier protein such as keyhole limpet hemocyanin, bovine thyroglobulin, ovalbumin, or the like, and administer it with an adjuvant. The antigenic peptide can be covalently bound to a carrier protein by performing reactions using cross-linking reagents such as maleimide, carbodiimide, glutaraldehyde, and the like. In the case of a maleimide reaction, a peptide in which a cysteine residue has been added to the N terminus or C terminus of the amino acid sequence of the antigenic peptide is prepared by the method described in 2, and covalently bound via cysteine. Examples of an adjuvant include Freund's complete adjuvant, aluminum hydroxide gel, pertussis vaccine, and the like. A rabbit, goat, rat, mouse, hamster, or the like can be used as a non-human animal to be administered with the antigen, and the dose per administration for each animal is preferably an amount that contains 50 to 200 μg of the antigenic peptide.

The antigen is preferably administered, for example, every one to three weeks for three to ten times after the first administration until the antibody titer of the serum has sufficiently increased. Serum antibody titer can be measured by preparing serum samples from blood collected three to seven days after each administration, and using an enzyme immunoassay method, radioimmunoassay method, or the like. With reference to Enzyme-linked Immunosorbent Assay, Igaku Shoin (1976) and Antibodies - A Laboratory Manual, Cold Spring Harbor Laboratory (1988), the enzyme immunoassay method can be performed based on the procedure of: (i) covalently binding an antigenic peptide to a carrier protein that is different from the one used for the antigen, and immobilizing it onto an appropriate plate, (ii) blocking and washing the plate, (iii) reacting the plate with the serum prepared from the immunized animal and then washing it, (iv) reacting with an enzyme-labeled antibody against IgG of the immunized animal and then washing it, and then (v) reacting the plate with a substrate that develops color or luminesces from the label enzyme and measuring the level of coloring or luminescence as an indicator of antibody titer.

Serum is prepared by collecting blood from a non-human animal that shows a sufficient antibody titer against the antigenic peptide in its serum. This serum, or specifically antiserum, can be used as a polyclonal antibody; alternatively, a polyclonal antibody can be purified from this antiserum.

The method for purifying a polyclonal antibody from antiserum includes, for example, centrifugation; salting out with 40-50% saturated ammonium sulfate; caprylic acid precipitation (Antibodies, A Laboratory manual, Cold Spring Harbor Laboratory (1988)); and chromatography using a DEAE-sepharose column, an anion exchange column, a protein A- or G-column, a gel filtration column, and the like, which may be carried out alone or in combination.

(2) Production of monoclonal antibodies

Monoclonal antibodies that bind to an epitope present in the amino acid sequence shown in SEQ ID NO: 1 can be prepared by the following methods. These monoclonal antibodies can bind specifically to peptides of the present invention.

(a) Preparation of antibody-producing cells

Mice and rats are used as animals for antigen administration. The same antigen used for the production of polyclonal antibodies of (1) is administered, and a mouse or rat that shows a sufficient antibody titer against the antigenic peptide in its serum can be used as a supply source of antibody-producing cells. Splenocytes can be used as antibody-producing cells. The antibody-producing cells can be prepared from a mouse or rat that shows a sufficient antibody titer, for example, as described below.

The spleen of the mouse or rat which showed the antibody titer is excised three to seven days after the final administration of the antigen. The spleen is cut into pieces in MEM, the cells are loosened using a pair of forceps and centrifuged, the supernatant is discarded, and the precipitated splenocytes are collected. The obtained splenocytes are treated with Tris-ammonium chloride buffer (pH 7.65) for one to two minutes to remove erythrocytes and then washed three times with MEM, and the resulting splenocytes are used as antibody-producing cells.

(b) Preparation of myeloma cells

Cells of a cell line established from mouse or rat myeloma cells can be used as myeloma cells. Examples of myeloma cell lines include 8-azaguanine-resistant mouse (BALB/c-derived) myeloma cell lines P3-X63Ag8-U1 (Curr. Topics. Microbiol. Immunol., 81, 1 (1978); Europ. J. Immunol., 6, 511 (1976)), SP2/0-Ag14 (Nature, 276, 269 (1978)), P3-X63-Ag8653 (J. Immunol., 123, 1548 (1979)), P3-X63-Ag8 (Nature, 256, 495 (1975)), and the like. These cell lines are preferably subcultured in 8-azaguanine medium (a medium produced by supplementing RPMI-1640 medium with glutamine (1.5 mmol/L), 2-mercaptoethanol ($5 \times 10^{-5}$ mol/L), gentamicin (10 $\mu$g/ml) and fetal calf serum (10%) (hereinafter referred to as "normal medium"), and further supplemented with 8-azaguanine (15 $\mu$g/ml)); and it is preferable to culture in the normal medium for three to four days before cell fusion. Preferably, $2 \times 10^7$ or more cells are used in fusion.

(c) Production of hybridomas

Hybridomas can be produced by fusing the antibody-producing cells obtained in (a) with the myeloma cells obtained in (b), for example, by using polyethylene glycol as follows. The antibody-producing cells obtained in (a) and the myeloma cells obtained in (b) are washed well with MEM or PBS (1.83 g of disodium phosphate, 0.21 g of monopotassium phosphate, 7.65 g of sodium chloride and one liter of distilled water, pH 7.2), mixed in a ratio of 5:1 to 10: 1 (antibody-producing cell:myeloma cell), and centrifuged at 1,200 rpm for five minutes, and then the supernatant is discarded. Cells of the obtained precipitation fraction are thoroughly loosened. 2 g of polyethylene glycol-1000, 2 mL of MEM, and 0.7 mL of dimethyl sulfoxide are mixed, and 0.2 to 1 mL of the prepared solution is added for every $10^8$ antibody-producing cells while stirring at 37°C, and then 1 to 2 mL of MEM is added several times every one to two minutes. After the addition, the total volume is adjusted to 50 mL by adding MEM. The prepared solution is centrifuged at 900 rpm for five minutes, and then the supernatant is discarded.

For example, the fused cells can be cultured as described below, and hybridomas with high levels of antibody production can be selected. Cells obtained in the precipitation fraction are loosened gently and then suspended in 100 mL of HAT medium (a medium produced by supplementing the normal medium with hypoxanthine ($10^{-4}$ mol/L), thymidine ($1.5 \times 10^{-5}$ mol/L), and aminopterin ($4 \times 10^{-7}$ mol/L)), by repeated gentle sucking and squirting with a measuring pipette. The suspension is preferably dispensed into a 96-well incubation plate at 100 $\mu$L per well and cultured in a 5% $CO_2$ incubator at 37°C for 7 to 14 days. After culturing, a portion of the culture supernatant is collected, and is used instead of serum for measuring the antibody titer as described above in (1). Hybridomas with culture supernatants that have high antibody titer can be selected as hybridomas with high-level antibody production.

The hybridoma can be cloned, from which clones with high antibody productivity can be selected to obtain hybridoma cells that steadily show high levels of antibody production. Cloning can be performed, for example, by limiting dilution or the like, and is preferably repeated twice by using HT medium (a medium in which aminopterin is removed from HAT medium) for the first cloning and the normal medium for the second cloning. The above-mentioned antibody titer measurement is performed using the culture supernatant of each of the clones obtained by cloning, and a hybridoma clone whose culture supernatant has high antibody titer can be selected as a hybridoma cell that steadily shows high antibody production.

(d) Preparation of monoclonal antibodies

Monoclonal antibodies of the present invention can be prepared, for example, as described below from ascites where hybridomas selected in (c) are allowed to proliferate as ascites carcinoma in nude mice. Preferably, the hybridoma cells obtained in (c), which produce monoclonal antibodies of the present invention, are administered by intraperitoneal injection at a dose of 5 to 20 x $10^6$ cells/animal to 8- to 10-weeks-old mice or nude mice that have been administered with 0.5 mL of 2,6,10,14-tetramethylpentadecane (pristane) intraperitoneally and reared for 2 weeks. Ten to 21 days later, ascitic fluid is collected from the mouse in which the hybridoma has caused ascites tumor, and this is centrifuged at 3,000 rpm for 5 minutes to remove solid matter. The monoclonal antibody can be purified and obtained from the obtained ascites supernatant using the same method for polyclonal antibody.

The subclass of the antibody can be determined using a mouse monoclonal antibody typing kit or a rat monoclonal antibody typing kit. The amount of peptide can be determined by the Lowry method or by absorbance at 280 nm.

(3) Methods for measuring the peptides of the present invention using antibodies of the present invention

The peptides of the present invention can be immunologically detected or quantified using the antibodies of the present invention. Examples of immunological detection or quantification methods are competition method, sandwich method, immunohistochemistry, Western blotting, aggregation method ("Tan-Clone-Kotai-Manual (Experimental Manual for Monoclonal Antibody" Kodansha-Scientific, 1987; and "Zoku-Seikagaku Jikken Kouza 5, Meneki-seikagaku Kenkyuho (Sequel to the Lectures on Biochemical Experiments 5, Immunobiochemical research methods)", Tokyo Kagaku Dojin, 1986), and the like.

The competition method includes the following steps: reacting an antibody of the present invention with a test solution and a fixed amount of competing substance (produced by labeling a peptide of the present invention to be measured with an enzyme, biotin, radioisotope, fluorescent substance, or the like); allowing the peptide of the present invention in the test solution and the competing substance to competitively bind the antibody; measuring the amount of competing substance bound to the antibody using the label, and quantifying the peptide of the present invention from the level of binding. Examples include a method of fixing the antibody onto a solid phase such as plates, beads, or the like, allowing the peptide of the present invention and the competing substance to competitively bind the antibody, washing the solid phase, and measuring the amount of competing substance bound to the antibody on the solid phase; a method of allowing the peptide of the present invention and the competing substance to competitively bind the antibody, using γ-globulin and polyethylene glycol to precipitate immune complexes for separating unbound competing substance, and then measuring the amount of competing substance bound to the antibody; and the like. The peptide of the present invention in the sample solution can be quantified, for example, by preparing five to ten predetermined concentrations of the solution of the peptide of the present invention, measuring the level of binding between the competing substance and the antibody when these solutions are used as a sample solution, producing a standard curve by plotting the peptide concentration versus the binding level of the competing substance. The standard curve can be applied to the binding level of the competing substance to quantify the peptide of the present invention in the test solution.

The sandwich method uses two types of antibodies that bind specifically to a peptide of the present invention. Examples include a method of fixing one of the antibodies onto a solid phase such as plates, beads, or the like, reacting a sample solution with this solid phase, and after binding the peptide of the present invention in the sample to the antibody on the solid phase, reacting it with the other antibody which is labeled with an enzyme, biotin, radioisotope, fluorescent substance, or the like, to bind the labeled antibody to the peptide of the present invention bound to the antibody on the solid phase, the binding level of the labeled antibody is determined using the labeling substance, and this binding level is used to quantify the peptide of the present invention. The peptide of the present invention in the sample solution can be quantified, for example, by preparing five to ten predetermined concentrations of the solution of the peptide of the present invention, measuring the level of binding the labeled antibody when these solutions are used as a sample solution, producing a standard curve by plotting the peptide concentration versus the binding level of the label. The standard curve can be applied to the binding level of the labeled antibody to quantify the peptide of the present invention in the test solution.

Enzyme immunoassay is a quantification method used to label the competing substance or the antibody in the above-mentioned competition method or sandwich method with an enzyme such as alkaline phosphatase, peroxidase, or the like, react it with a reagent that develops color or luminescence from the label enzyme, and determine the binding level of the competing substance or labeled antibody from the level of color development or luminescence. Furthermore, radioimmunoassay is a quantification method used to label the competing substance or the antibody in the above-mentioned competition method or sandwich method with a radioisotope, and determining the binding level of the competing substance or the labeled antibody from radioactivity.

Immunohistochemistry is used to detect a peptide of the present invention in tissues or cells by reacting a frozen or paraffin-embedded section of tissues or cells with an antibody of the present invention labeled with an enzyme, biotin, radioisotope, fluorescent substance, gold colloid, or the like, and then detecting the antibody of the present invention using the labeling substance.

Western blotting is a method used to separate proteins and peptides included in a sample on an SDS-polyacrylamide gel, blot proteins and peptides from the gel onto a polyvinylidene difluoride (PVDF) membrane, nitrocellulose membrane, or the like, and after reacting this with an antibody of the present invention labeled with an enzyme, biotin, radioisotope, or the like, detect the antibody of the present invention using the labeling substance, and detect the peptide of the present invention on the membrane.

The aggregation method uses absorbance measurement to detect or quantify aggregates of particles formed from reaction of a test solution with latex particles or the like immobilized with an antibody of the present invention, and binding of the antibody on the particles to the peptide of the present invention in the sample.

4. VGF-related peptide-containing pharmaceutical formulations

In the present invention, any of the peptides of (a) to (f) described below is referred to as a "VGF-related peptide". The peptides of the present invention described in 1. are included in the VGF-related peptides:

(a) a peptide comprising the amino acid sequence shown in any of SEQ ID NOS: 1 to 12, and 23 to 29;

(b) a peptide comprising an amino acid sequence with a substitution, deletion, or addition of one to five amino acids in the amino acid sequence shown in any of SEQ ID NOS: 1 to 12, and 23 to 29, wherein the peptide has an activity of increasing the intracellular calcium ion concentration in cells of the hypothalamus, pituitary gland, kidney, heart, blood vessel, or brain tissue;

(c) a peptide comprising an amino acid sequence having 90% or higher homology to the amino acid sequence shown in any of SEQ ID NOS: 1 to 12, and 23 to 29, wherein the peptide has an activity of increasing the intracellular calcium ion concentration in cells of the hypothalamus, pituitary gland, kidney, heart, blood vessel, or brain tissue;

(d) a peptide comprising an amino acid sequence with a substitution, deletion, or addition of one to five amino acids in the amino acid sequence shown in any of SEQ ID NOS: 1, 25, 28, and 29, wherein the peptide has an activity of promoting vasopressin secretion from the posterior pituitary gland;

(e) a peptide comprising an amino acid sequence having 90% or higher homology to the amino acid sequence shown in any of SEQ ID NOS: 1, 25, 28, and 29, wherein the peptide has an activity of promoting vasopressin secretion from the posterior pituitary gland; and

(f) a peptide represented by the following formula (V)

$$R^9\text{-}E\text{-}R^{10} \qquad (V)$$

(wherein, $R^9$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^{10}$ represents hydroxy, substituted or unsubstituted alkoxy, or substituted or unsubstituted amino; and E represents a peptide residue of any one of the above-mentioned (a) to (e)).

Amino acid substitution, deletion, and addition, and homology of the amino acid sequence in the "VGF-related peptide" mentioned above have the same definitions as amino acid substitution, deletion, and addition, and homology of the amino acid sequence in the peptide of the present invention in 1. Each group in formula (V) has the same definition as in formulas (I) to (IV) of 1.

Since the VGF-related peptides and the pharmaceutically acceptable salts thereof have an activity of increasing intracellular calcium concentration of renal, vascular, or cardiac cells, they have circulation-modulating activity which modulates blood pressure and the amount of blood flow. Therefore, the VGF-related peptides and the pharmaceutically acceptable salts thereof can be used as active ingredients of circulation-modulating agents, vasopressors, and therapeutic agents for diseases of the circulatory system such as myocardial infarction, ischemic heart disease, cerebral infarction, or the like.

Since the VGF-related peptides and pharmaceutically acceptable salts thereof have an activity of increasing intracellular calcium concentration in cells of the hypothalamus, pituitary gland, or brain tissues, they have an energy-modulating activity which modulates food or water consumption, or energy metabolism. Therefore, the VGF-related peptides and the pharmaceutically acceptable salts thereof can be used as active ingredients of food consumption-modulating agents, water consumption-modulating agents, metabolism-modulating agents, and therapeutic agents for diseases associated with energy modulation, such as obesity, cibophobia, and insomnia.

Since the VGF-related peptides and pharmaceutically acceptable salts thereof have an activity of promoting vasopressin secretion from the posterior pituitary gland, they have circulation-modulating activity which modulates blood pressure, and the amount of blood flow and body fluid. Furthermore, since vasopressin is also involved in the reabsorption of water and electrolytes in the kidney, glycogenolysis, and the like, the VGF-related peptides and pharmaceutically acceptable salts thereof have an energy-modulating activity which modulates food or water consumption, or energy metabolism. Thus, the VGF-related peptides and pharmaceutically acceptable salts thereof can be used as active ingredients of circulation-modulating agents, vasopressors, and therapeutic agents for diseases of the circulatory system such as myocardial infarction, ischemic heart disease, cerebral infarction, or the like, as well as active ingredients of food consumption-modulating agents, water consumption-modulating agents, electrolyte metabolism-modulating agents, energy metabolism-modulating agents, and therapeutic agents for diseases associated with energy modulation, such as obesity, cibophobia, and insomnia.

Pharmaceutically acceptable salts of VGF-related peptides include pharmaceutically acceptable salts of the peptides of the present invention described in 1.

In pharmaceutical formulations containing a VGF-related peptide or a pharmaceutically acceptable salt thereof, the peptide or the pharmaceutically acceptable salt thereof may be included as an active ingredient as such, or in a mixture with any other therapeutic active ingredient. Such pharmaceutical formulations are produced by any method well known in the technical field of pharmaceutical formulation by mixing the active ingredient with one or more pharmaceutically acceptable carriers.

It is desirable to use the most effective route of administration for carrying out the treatment, and examples include oral administration and parenteral administration such as intraventricular administration, intravenous administration, and the like.

Dosage forms include tablets, powders, granules, syrups, injections, and the like.

For example, liquid preparations that are suitable for oral administration, such as syrups, can be produced using water; saccharides such as sucrose, sorbitol, fructose, and the like; glycols such as polyethylene glycol, propylene glycol, and the like; oils such as sesame oil, olive oil, soybean oil, and the like; antiseptics such as *p*-hydroxybenzoate esters, and the like; flavors such as strawberry flavor, peppermint, and the like. Tablets, powders, granules, and the like can be produced using excipients such as lactose, glucose, sucrose, mannitol, and the like; disintegrating agents such as starch, sodium alginate, and the like; lubricants such as magnesium stearate, talc, and the like; binders such as polyvinyl alcohol, hydroxypropylcellulose, gelatin, and the like; surfactants such as fatty acid ester, and the like; plasticizers such as glycerol, and the like.

Formulations suitable for parenteral administration preferably contain sterile aqueous agents that are isotonic to the recipient's blood and contain active compounds. For example, in the case of injections, injection solutions are prepared using a carrier containing a salt solution or glucose solution, or a mixture of salt solution and glucose solution, or the like.

For these parenteral agents, one or more of the examples shown for oral agents, such as diluents, antiseptics, flavors, excipients, disintegrators, lubricants, binders, surfactants, plasticizers, and the like, can also be added as supplementary components.

The dosage and the number of doses a peptide of the present invention or a pharmaceutically acceptable salt thereof vary depending on the form of administration, age and body weight of the patient, and characteristics or severity of the symptoms to be treated; in normal oral administration, 0.01 mg to 1 g, or preferably 0.05 mg to 50 mg is administered once or several times per day for an adult. In parenteral administration, such as intravenous administration or the like, 0.001 mg to 100 mg, or preferably 0.01 mg to 10 mg is administered once or several times per day for an adult. However, the dosage and the number of doses may vary depending on various conditions as mentioned above.

5. Measurement of energy-modulating activity or circulation-modulating activity of VGF-related peptides and pharmaceutically acceptable salts thereof

The energy-modulating activity or circulation-modulating activity of a VGF-related peptide or a pharmaceutically acceptable salt thereof can be confirmed when the following assays show that it has activity to increase intracellular calcium ion concentration.

(a) Measurement for the activity of increasing intracellular calcium ion concentration

(i) Use of apoaequorin-expressing transgenic mice

A hypothalamus, pituitary gland, kidney, heart, blood vessel, or a portion of brain tissue is collected from transgenic mice systemically expressing apoaequorin (WO02/010371) produced by introducing an apoaequorin gene expression vector into fertilized eggs. The obtained hypothalamus, pituitary gland, kidney, heart, blood vessel, or brain tissue is cut finely into pieces, suspended in a medium containing coelenterazine, and then incubated to incorporate coelenterazine into the cells to form aequorin (a complex of apoaequorin and coelenterazine). Since aequorin luminesces upon binding to intracellular calcium ions, the relative luminescence level in cells before and after addition of a medium containing the peptide or a pharmaceutically acceptable salt thereof is measured in a luminometer every second over time and is used as an indicator of intracellular calcium ion concentration. Increase in the relative luminescence level due to addition of the peptide or pharmaceutically acceptable salt thereof confirms that the peptide or pharmaceutically acceptable salt thereof has the activity to increase intracellular calcium ion concentration.

(ii) Use of calcium-binding fluorescence reagents

A finely cut hypothalamus, pituitary gland, kidney, heart, blood vessel, or brain tissue collected from animal, or a cell line derived from cells of the hypothalamus, pituitary gland, kidney, heart, blood vessel, or brain tissue is suspended in a buffer containing a calcium ion-binding fluorescence reagent, such as Fura-2, Indo-1, Fluo-3, or the like, whose excitation wavelength, fluorescence wavelength, or fluorescence intensity changes depending on the presence or absence of calcium ions, and the suspension is cultured to incorporate the reagent into the cells. The fluorescence excitation wavelength peak shifts from 380 nm to 340 nm as a result of Fura-2 binding to calcium ions. Therefore, the fluorescence intensity ratio between 380 nm excitation and 340 nm excitation is measured with a fluorometer before and after addition of a buffer containing the peptide or a pharmaceutically acceptable salt thereof, and is used as an indicator of intracellular calcium ion concentration. Increase in the fluorescence intensity ratio due to addition of a peptide or a pharmaceutically acceptable salt thereof confirms that this peptide or a pharmaceutically acceptable salt thereof has the activity to increase intracellular calcium ion concentration. The fluorescence wavelength shifts from 480 nm to 400 nm as a result of Indo-1 binding to calcium ions. The fluorescence intensity ratio between 400 nm and 480 nm before and after addition of a buffer containing the peptide or a pharmaceutically acceptable salt thereof is measured with a fluorometer, and used as an indicator of intracellular calcium ion concentration. Increase in the fluorescence intensity ratio due to addition of the peptide or a pharmaceutically acceptable salt thereof confirms that this peptide or pharmaceutically acceptable salt thereof has the activity to increase intracellular calcium ion concentration. The fluorescence intensity at a wavelength of 520 nm is markedly increased as a result of Fluo-3 binding to calcium ions. The fluorescence intensity ratio at a wavelength of 520 nm before and after addition of a buffer containing the peptide or a pharmaceutically acceptable salt thereof is measured with a fluorometer, and used as an indicator of intracellular calcium ion concentration. Increase in the fluorescence intensity ratio due to addition of the peptide or a pharmaceutically acceptable salt thereof confirms that this peptide or pharmaceutically acceptable salt thereof has the activity to increase intracellular calcium ion concentration.

(b) Energy-modulation regulating activity

A catheter is inserted into the lateral ventricle of an anesthetized animal such as rat or the like, and the peptide or a pharmaceutically acceptable salt thereof dissolved in physiological saline is administered into the lateral ventricle via the catheter transiently or several times during an appropriate period. The body weight, water consumption, food consumption, amount of locomotor activity, length of arousal, amount of body fat, body temperature, and the like are measured after administration and under free action. Alternatively, a catheter is inserted into the external jugular vein or the like of an anesthetized animal such as rat or the like, and the peptide or a pharmaceutically acceptable salt thereof dissolved in physiological saline is administered via the catheter through the external jugular vein or the like transiently or several times during an appropriate period. The body weight, water consumption, food consumption, amount of locomotor activity, length of arousal, amount of body fat, body temperature, and the like are measured after administration and under free action. Difference in these measured items compared to those of the non-administered group confirms that this peptide or a pharmaceutically acceptable salt thereof has the activity to regulate energy-modulation.

(c) Blood pressure increasing activity

Catheters are inserted into the external jugular vein and internal carotid artery of an anesthetized animal such as rat or the like, and arterial pressure is measured continuously by connecting the catheter in the internal carotid artery to a blood pressure monitor. The peptide or pharmaceutically acceptable salt dissolved in physiological saline is administered through the external jugular vein. The peptide or pharmaceutically acceptable salt is confirmed to have the activity to increase blood pressure, when comparison of the arterial pressures before and after administration of the peptide or pharmaceutically acceptable salt shows that the arterial pressure increases due to administration of the peptide or pharmaceutically acceptable salt.

(d) Vasopressin secretion-promoting activity

In transgenic (Tg) rats produced using a fusion gene, which is a vasopressin (arginine vasopressin: AVP) gene inserted with the enhanced green fluorescent protein (eGFP) gene, eGFP is expressed specifically in AVP neurons of the hypothalamus-pituitary system and their axons (Ueta et al., Endocrinology, 146, 406-413, 2005). Vasopressin secretion-promoting activity can be measured using pituitary gland excised from such AVP-eGFP Tg rats.

The pituitary gland is excised from an AVP-eGFP Tg rat and placed in a chamber of a perfusion apparatus filled with perfusate (140 mM NaCl, 5 mM KCl, 10 mM HEPES, 10 mM glucose, 1.2 mM $KH_2PO_4$, 1.2 mM $MgCl_2$, 2 mM $CaCl_2$; the pH and osmotic pressure are adjusted to 7.37 and 295 to 300 mOsml, respectively). Laser beam (488 nm) from an excitation light irradiation device ([161]C; manufactured by Spectra Physics), is irradiated onto the posterior pituitary gland through an optical fiber (GIF625-100; manufactured by Thorlabs). The eGFP fluorescent light as a result of excitation at nerve endings in the posterior pituitary gland is collected by a phototube (R6249HA; Hamamatsu Photonics) through another optical fiber. After conversion into an electric signal, it can be amplified with an amplifier (C7246; manufactured by Hamamatsu Photonics) to observe the change in the eGFP fluorescence. The amount of change serves as an indicator for the amount of change of AVP-eGFP present in the pituitary gland, and the vasopressin secretion-promoting activity of the VGF-related peptide or a pharmaceutically acceptable salt thereof can be measured.

Specifically, the recording time is set to 10 minutes. As a control (basal), only the perfusate is administered in the first five minutes. Then, a mixture of the perfusate and the VGF-related peptide or a pharmaceutically acceptable salt thereof prepared to have a final concentration of $10^{-6}$ M is administered in the last five minutes. The amount of vasopressin secreted from the pituitary gland can be measured by observing changes in the eGFP fluorescence.

6. Method of screening for substances that inhibit or promote the VGF-related peptide-induced increase of intracellular calcium ion concentration in cells of the hypothalamus, pituitary gland, kidney, heart, blood vessel, or brain tissue

Screening for substances that inhibit the increase of intracellular calcium ion concentration in cells of the hypothalamus, pituitary gland, kidney, heart, blood vessel, or brain tissue induced by a VGF-related peptide can be carried out by (i) measuring the cellular response elicited when the VGF-related peptide or a pharmaceutically acceptable salt thereof and a test substance are contacted with the cells of the hypothalamus, pituitary gland, kidney, heart, blood vessel, or brain tissue, (ii) comparing this with the cellular response in which the VGF-related peptide or a pharmaceutically acceptable salt thereof is contacted with the same cells in the absence of the test substance, and (iii) identifying the test substance as a substance that inhibits the increase of intracellular calcium ion concentration in cells of the hypothalamus, pituitary gland, kidney, heart, blood vessel, or brain tissue induced by the VGF-related peptide, when the cellular response is suppressed in the presence of the test substance.

Similarly, screening for substances that promote the increase of intracellular calcium ion concentration in cells of the hypothalamus, pituitary gland, kidney, heart, blood vessel, or brain tissue induced by the VGF-related peptide can be carried out by (i) measuring the cellular response elicited when the VGF-related peptide or a pharmaceutically acceptable salt thereof and a test substance are contacted with the cells of the hypothalamus, pituitary gland, kidney, heart, blood vessel, or brain tissue, (ii) comparing this with the cellular response when the VGF-related peptide or a pharmaceutically acceptable salt thereof is contacted with the same cells in the absence of the test substance, and (iii) identifying the test substance as a substance that promotes the increase of intracellular calcium ion concentration in cells of the hypothalamus, pituitary gland, kidney, heart, blood vessel, or brain tissue induced by the VGF-related peptide, when the cellular response is promoted in the presence of the test substance.

The cellular response may be any cellular response, for example, an increase in intracellular calcium ion concentration so long as it is a measurable cellular response elicited by the VGF-related peptide when it is contacted with cells of the hypothalamus, pituitary gland, kidney, heart, blood vessel, or brain tissue.

The cells of the hypothalamus, pituitary gland, kidney, heart, blood vessel, or brain tissue used in the above-mentioned screening method may be a cell line derived from a hypothalamus, pituitary gland, kidney, heart, blood vessel, or brain tissue, or a finely cut hypothalamus, pituitary gland, kidney, heart, blood vessel, or brain tissue collected from an animal, so long as they show cellular responses when contacted with a VGF-related peptide. As intracellular calcium ion concentration can be conveniently measured using a luminometer by measuring the luminescence level in the presence of coelenterazine, it is preferable to use cells obtained by finely cutting a hypothalamus, pituitary gland, kidney, heart, blood vessel, or brain tissue collected from a transgenic mouse that is produced by introducing the apoaequorin gene and systemically expresses apoaequorin (WO02/010371).

Substances that promote the increase of intracellular calcium ion concentration in cells of the hypothalamus, pituitary gland, kidney, heart, blood vessel, or brain tissue induced by the VGF-related peptides obtained by the above-mentioned screening method have energy-modulating activity or circulation-modulating activity similar to the VGF-related peptides. Therefore, they can be used as food consumption-modulating agents, water consumption-modulating agents, metabolism-modulating agents, circulation-modulating agents, vasopressors, or therapeutic agents for diseases associated with energy modulation, such as food or water consumption disorders, metabolic disorders, and sleep disorders, and diseases of the circulatory system such as myocardial infarction, ischemic heart disease, cerebral infarction, and the like. Substances that inhibit the increase of intracellular calcium ion concentration in cells of the hypothalamus, pituitary gland, kidney, heart, blood vessel, or brain tissue induced by the VGF-related peptides inhibit activities possessed by VGF-related peptides, such as, blood pressure increasing activity, and thus they may be used as antihypertensives.

7. Methods of screening for agonists or antagonists against VGF-related peptide receptors

Screening for agonists or antagonists against VGF-related peptide receptors can be carried out by (i) measuring the level of the VGF-related peptide or a pharmaceutically acceptable salt thereof binding to cells of the hypothalamus, pituitary gland, kidney, heart, blood vessel, or brain tissue, or to a membrane fraction of the cells, when the peptide or a pharmaceutically acceptable salt thereof and a test substance are contacted with these cells or their membrane fraction, (ii) comparing this with the level of the VGF-related peptide or a pharmaceutically acceptable salt thereof binding to the same cells or membrane fraction of these cells in the absence of the test substance, and (iii) identifying the test substance as an agonist or antagonist against the VGF-related peptide receptor when the binding level of the peptide or a pharmaceutically acceptable salt thereof decreases in the presence of the test substance.

The cells described in 5. above can be used as the cells of the hypothalamus, pituitary gland, kidney, heart, blood vessel, or brain tissue. These cells or their cell membrane fraction are suspended in a suitable buffer. The buffer may be any buffer so long as the binding between a VGF-related peptide and the cells or cell membrane fraction is not inhibited, and for example, a phosphate buffer, Tris-HCl buffer, or the like at pH 4 to 10 (or desirably pH 6 to 8) is used. Furthermore, surfactants such as CHAPS, Tween-80, digitonin, deoxycholic acid, or the like, or various proteins such as bovine serum albumin, gelatin, or the like can be added to the buffer to decrease non-specific binding. Furthermore, to suppress degradation of the polypeptides or ligands of the present invention by proteases, a protease inhibitor such as PMSF, leupeptin, E-64, pepstatin, or the like can be added.

Binding experiments are performed by placing a VGF-related peptide labeled with a radioisotope such as $^{125}I$, $^{3}H$, or the like and having a certain level of radioactivity, together with 10 $\mu$L to 10 mL of a suspension solution of these cells or a cell membrane fraction of these cells. The reaction is carried out at 0 to 50°C, preferably at 4 to 37°C, and for 20 minutes to 24 hours, preferably 30 minutes to 3 hours. The reaction is followed by filtration through a glass fiber filter or the like, and washing with a suitable amount of the same buffer. The radioactivity remaining on the glass fiber filter is measured using a $\gamma$-counter or liquid scintillation counter. This binding level is defined as the total binding level (A). A similar reaction is carried out under conditions in which a large excess of the same but unlabeled compound is added, and this binding level is defined as the non-specific binding level (B). A similar reaction is carried out under conditions in which a test substance is added, and this binding level is defined as C. The rate of binding inhibition of the test substance can be determined by the following equation.

$$\text{Inhibition rate (\%)} = [1 - \{(C - B) / (A - B)\}] \times 100$$

Similarly to VGF-related peptides, receptor agonists of VGF-related peptides that can be obtained by the above-described screening method have energy-modulating activity or circulation-modulating activity. Therefore, they may be used as food consumption-modulating agents, water consumption-modulating agents, metabolism-modulating agents, circulation-modulating agents, vasopressors, and therapeutic agents for diseases associated with energy modulation, such as food or water consumption disorders, metabolic disorders, and sleep disorders, and diseases of the circulatory system such as myocardial infarction, ischemic heart disease, cerebral infarction, and the like. Furthermore, receptor antagonists of VGF-related peptides inhibit activities possessed by VGF-related peptides such as blood pressure increasing activity, and thus they may be used as antihypertensives.

All prior-art documents cited herein have been incorporated herein by reference.

Examples

Hereinbelow, the present invention is specifically described with reference to Examples; however, it should not be construed as being limited thereto.

[Example 1]

Isolation of VGF-derived peptides and determination of their structures

A human pancreas-derived cell line ($10^8$ cells) was grown until confluent and cultured for six hours in a phenol red-free and serum-free RPMI medium, and the medium was collected. One-fiftieth volume of 1 mol/L hydrochloric acid was added to the supernatant obtained by collecting and centrifuging the medium, and then the sample was extracted using a Sep-Pak C18 cartridge (manufactured by Waters). The cartridge was washed with 0.1 % trifluoroacetic acid (hereinafter abbreviated as TFA), and the sample was eluted with 60% acetonitrile-0.1% TFA. After freeze-drying the eluate, it was dissolved in 60% acetonitrile-0.1% TFA, and the peptide fraction was collected by HPLC (HPLC pump L-2100; manufactured by Hitachi). Separation was carried out at a flow rate of 1.5 mL/min using a gel filtration HPLC column (TSKgel G2000SW$_{XL}$, 21.5 mm x 30 cm; manufactured by TOSO) that has been equilibrated with the same solution. This freeze-dried sample was dissolved in 1 mol/L acetic acid, then neutralized with 1 mol/L Tris (pH11), and then warmed at 37°C for one hour in a reduction reaction solution (1 mmol/L EDTA, 25 mmol/L dithiothreitol, 0.5 mol/L Tris, pH8.5). Subsequently, iodoacetamide was added at a final concentration of 50 mmol/L, and this was allowed to react in the dark for 15 minutes at room temperature. The reaction was stopped with glacial acetic acid, and desalting was performed using a Sep-Pak C18 cartridge. The desalted and freeze-dried sample was dissolved in 0.1 % TFA, and separation was performed by HPLC (HPLC pump L-6000 (manufactured by Hitachi)) at a flow rate of 50 μL/min using a reverse phase HPLC column (Vydac Protein & Peptide C18, 1 mm x 15 mm; manufactured by Grace Vydac), and the fractions were collected every 30 seconds. Each fraction was dried under reduced pressure, dissolved in 50% methanol/2% acetic acid, and analyzed using two types of mass spectrometric methods: the matrix assisted laser desorption ionization (MALDI) method and electrospray ionization (ESI) method. In the MALDI method, the above-mentioned sample was applied onto the target plate and then 0.5 μL of a solution of 2.5 mg/mL of α-cyano-4-hydroxycinnamic acid dissolved in 50% acetonitrile-0.1% TFA was added. The plate was subjected to mass spectrometry using a tandem time-of-flight (TOF) mass spectrometer (4700 Proteomics Analyzer; manufactured by Applied Biosystems) and the detected peptides were identified successively. Mass spectrometry using the ESI method was performed using a quadrupole time-of-flight mass spectrometer (Q-Tof2; manufactured by Micromass). The obtained tandem mass spectra were identified using an analysis software (Mascot MS/MS Ion Search; produced by Matrix Science) based on amino-acid sequence databases, NCBI and Swiss-Prot. As a result, the eight types of peptides shown in SEQ ID NOS: 1 to 4, 7, and 9 to 11, each of which comprises a portion of the amino acid sequence of human VGF were discovered. The N terminus of the peptide shown in SEQ ID NO: 1 was pyroglutamylated. The four peptides shown in SEQ ID NOS: 5, 6, 8, and 12 are rat counterparts of the human-derived peptides shown in SEQ ID NOS: 9, 10, 11, and 4, respectively.

[Example 2] Preparation of VGF-derived peptides

Each of the peptides discovered in Example 1 was chemically synthesized by request (Sigma-Genosys).
Structures of the synthesized peptides were confirmed by mass spectrometry using the MALDI method on a MALDI-TOF mass spectrometer, Autoflex (manufactured by Brucker). A saturated α-cyano-4-hydroxycinnamic acid (produced by Sigma-Aldrich) solution was prepared with 50% acetonitrile-0.1% TFA and used as the matrix. The peptide structures were confirmed by mass spectrometry using the MALDI method on a MALDI-TOF mass spectrometer Voyager DE Pro (Applied Biosystems). A two-fold diluted solution of the saturated α-cyano-4-hydroxycinnamic acid (produced by Sigma-Aldrich) solution prepared with 50% acetonitrile-0.1% TFA was used as the matrix. Furthermore, the peptide structures were also confirmed by amino acid analyses (Anal. Biochem., 222, 19 (1994)). Hydrolysis was performed in hydrochloric acid vapor at 110°C for 22 hours using a Pico-Tag Workstation (Waters), and the amino acid composition of the hydrolysis product was determined using an amino acid analyzer L-8500 (manufactured by Hitachi).

[Example 3]

Measurement of the activity of VGF-derived peptides to increase intracellular calcium ion concentration

Whether Peptides 1 to 8 synthesized in Example 2 have the activity of increasing intracellular calcium ion concentration was investigated using the organs of transgenic mice introduced with the apoaequorin gene and systemically expressing apoaequorin (hereinafter referred to as apoaequorin-expressing mice). In apoaequorin-expressing mouse cells, light is emitted when apoaequorin binds to a calcium ion in the presence of the luminescent substrate coelenterazine and thus the intracellular calcium ion concentration can be monitored. Patent Document (WO02/010371) discloses the method for producing apoaequorin-expressing mice, method for evaluating biologically active substances that use biological samples derived from the mice, and experimental results of evaluating biologically active peptides using the mouse organs as shown below. More specifically, it is reported that when angiotensin II was added at a final

concentration of 1 μmol/L to each of the organs obtained from the apoaequorin-expressing mice, strong luminescence was observed in the blood vessels, uterus, and adrenal glands; and when bradykinin was added at a final concentration of 10 μmol/L, strong luminescence was observed in the blood vessels, uterus, and adrenal glands. Therefore, apoaequorin-expressing mice can be used in the evaluation of the physiological activities of novel peptides.

Apoaequorin-expressing mice were produced according to the method disclosed in Reference Example 4 of Patent Document (WO02/010371). The apoaequorin-expressing mice were sacrificed, individual organs including thymus, hypothalamus, spleen, bone, aorta, heart, kidney, adrenal gland, pancreas, pituitary gland, uterus, medulla oblongata, and spinal cord were removed; and each of the organs was cut into small cubes of approximately 1 to 2 mm³. Next, in 5-mL tubes (Rohren-Tubes; manufactured by Sarstedt, No. 55.476), three portions of each of the prepared organs were added to 50 μL of a 10 μmol/L solution of coelenterazine (manufactured by Molecular Probes) dissolved in RPMI 1640 medium, and these were cultured at 37°C for three hours. After culturing, RPMI 1640 medium was added, and then Peptides 1 to 5 dissolved in RPMI 1640 medium were added 25 seconds after the addition of RPMI 1640 until each peptide had final concentrations of 1 μmol/L and of 5 μmol/L, and the relative luminescence levels were measured every second immediately after the addition of RPMI 1640 medium by using a luminometer (AutoLumat LB953, manufactured by Berthold).

As a result, luminescence was observed in the thymus, hypothalamus (Fig. 1), pituitary gland (Fig. 2), and medulla oblongata for the peptide of SEQ ID NO: 1; in the hypothalamus (Fig. 3), pituitary gland (Fig. 4), heart (Fig. 5), and medulla oblongata for the peptide of SEQ ID NO: 2; in the thymus, hypothalamus (Fig. 6), pituitary gland (Fig. 7), kidney (Fig. 8), and medulla oblongata for the peptide of SEQ ID NO: 3; in the hypothalamus (Fig. 9), pituitary gland (Fig. 10), and pancreas for the peptide of SEQ ID NO: 4; in the hypothalamus (Fig. 11), pituitary gland (Fig. 12), heart (Fig. 13), kidney (Fig. 14), spleen, uterus, medulla oblongata, and spinal cord for the peptide of SEQ ID NO: 5; in the thymus, hypothalamus (Fig. 15), pituitary gland (Fig. 16), aorta (Fig. 17), kidney (Fig. 18), spleen, heart, uterus, and medulla oblongata for the peptide of SEQ ID NO: 6; in the hypothalamus (Fig. 19), pituitary gland (Fig. 20), aorta (Fig. 21), heart (Fig. 22), and spleen for the peptide of SEQ ID NO: 7; in the thymus, pituitary gland (Fig. 23), heart (Fig. 24), spleen, medulla oblongata, and spinal cord for the peptide of SEQ ID NO: 8; in the hypothalamus (Fig. 25) and pituitary gland (Fig. 26) for the peptide of SEQ ID NO: 23; in the hypothalamus (Fig. 27), pituitary gland (Fig. 28), and pancreas (Fig. 29) for the peptide of SEQ ID NO: 24; in the hypothalamus (Fig. 30) and pituitary gland (Fig. 31) for the peptide of SEQ ID NO: 25; and in the pituitary gland (Fig. 32) for the peptide of SEQ ID NO: 28. From the above, it was found that these peptides have an activity of increasing intracellular calcium ion concentration in cells of the hypothalamus, pituitary gland, kidney, heart, blood vessel, and the like, which are organs involved in energy modulation or of the circulation system.


[Example 4] Antibody production


(1) Animal immunization and antiserum preparation


To conjugate a carrier protein to a peptide comprising ESPGPERVW, which is a portion of human VGF and corresponds to the C-terminal portion of SEQ ID NO: 1, a peptide in which a cysteine residue is added to the N terminus of the ESPGPERVW peptide was chemically synthesized as in Example 2. 6.0 mg of the peptide was covalently bonded with 10 mg of maleimide-activated keyhole limpet hemocyanin (Imj ect Activated mcKLH; manufactured by Pierce) via the cysteine residue. The covalent bonding reaction was performed according to the manual provided by Pierce. The obtained conjugate between the peptide and KLH was dialyzed against physiological saline, and this was used as an antigen. The antigen was dispensed and stored at -35°C until use. 0.5 mL of the obtained antigen solution in physiological saline was mixed with an equivalent amount of the Freund's complete adjuvant to prepare a stable emulsion, and intradermally administered seven times to a male rabbit (New Zealand white rabbit) for immunization in two-weeks intervals. After repeated administration, antibody titer was measured, serum was prepared from rabbits showing an increase in antibody titer, and this was used as the antiserum.


(2) Antibody titer measurement


Antibody titer was measured by radioimmunoassay (RIA) as indicated below. Namely, 100 μL of RIA buffer containing a peptide labeled with a specified amount of [¹²⁵I] (approximately 20000 cpm, 500 to 550 Bq, approximately 9 fmol of peptide) was added to 100 μL of antiserum sequentially diluted with RIA buffer (25 mmol/L EDTA, 80 mmol/L sodium chloride, 0.05% sodium azide, 0.5% *N*-ethylmaleimide-treated BSA, 50 mmol/L sodium phosphate buffer containing 0.5% TritonX-100 (pH7.4)) in a polystyrene tube, and this was incubated at 4°C for 40 hours to link the antibody in the antiserum to the labeled peptide. To measure non-specific binding, reactions using an antiserum-free RIA buffer solution instead of the antiserum were carried out as control. After incubation, 100 μL of a 1% bovine γ-globulin (manufactured by Sigma-Aldrich) solution (50 mmol/L sodium phosphate buffer containing 80 mmol/L sodium chloride and 0.05% sodium azide (pH7.4)) was added and mixed; then 500 μL of a 23% polyethylene glycol #6000 (manufactured by Nakalai Tesque) solution (50 mmol/L sodium phosphate buffer containing 80 mmol/L sodium chloride and 0.05% sodium azide (pH7.4)) was further added and mixed. This was left on ice for ten minutes or more, and then centrifuged for 15 minutes at 3000 rpm to precipitate the immune complex. Supernatant containing the unbound [125I]-labeled peptide was removed by aspiration, and radioactivity A (cpm) of the precipitate was measured using a γ-counter. Radioactivity N was similarly measured in the tubes for non-specific binding reaction (non-specific binding level; cpm), and value N for non-specific binding was subtracted from the precipitate's radioactivity value A, and the value obtained was defined as the specific binding level of the antiserum. Radioactivity T (cpm) of the 100 μL RIA buffer containing the [125I]-labeled Peptide 1 used in the reaction was measured using a γ-counter, and the percentage ratio (X) of specific binding level to radioactivity of added antigenic peptide was determined by the following equation. Dilution ratio of antiserum was plotted against X, and the inverse of the dilution ratio at which X becomes 30% was used as the indictor for antibody titer.

$$X\ (\%) = \{(A - N)/T\} \times 100$$

[125I] labeling of the antigenic peptide was carried out using the lactoperoxidase method, and a peptide synthesized with a tyrosine residue added to the N-terminus of the antigenic peptide was labeled. Specifically, 10 μg of the peptide was dissolved in 25 μL of 0.4 mol/L sodium acetate buffer (pH 5.6), then 10 μl of 0.1 mol/L sodium acetate buffer (pH 5.6) solution containing 200 ng of lactoperoxidase, 5 μl of 3.7 MBq/μL Na125I (18.5 MBq), and 5 μL of 0.002% hydrogen peroxide were added, and this was reacted with stirring at 30°C for ten minutes. In addition, 5 μL of 0.002% hydrogen peroxide was added, and reacted with stirring at 30°C for ten minutes. 500 μL of water was added and fractions of the labeled peptide were collected by C18 reverse phase HPLC using a solvent system of 10% to 60% acetonitrile gradient/0.1% TFA. The peptide solutions were diluted with 60% acetonitrile-0.1% TFA. After adding *N*-ethyl maleimide-treated BSA at a final concentration of 0.5%, the solution was dispensed into aliquots and stored at -85°C.

(3) Binding specificity of antisera

Of the obtained antisera, the above-mentioned antiserum showing an antibody titer of $3 \times 10^5$ (specifically, an antiserum in which 30% of the antigenic peptide added in the above-mentioned RIA showed binding activity even at $9 \times 10^5$-fold dilution) was used to examine the binding specificity against the six types of VGF-derived peptides using RIA. Namely, to 100 μL of antiserum diluted $9 \times 10^5$ folds with RIA buffer, 100 μL of RIA buffer containing a fixed amount of [125I]-labeled Peptide 1 (approximately 20000 cpm, 500-550 Bq, approximately 9 fmol in terms of peptide amount) and 100 μL of RIA buffer solutions each containing a sequentially diluted VGF-derived peptide were added, this was incubated at 4°C for 40 hours, and the labeled Peptide 1 and VGF-derived peptide were competitively bound to the antibody in the antiserum. As controls, reactions that use RIA buffer instead of the antiserum to measure non-specific binding, and reactions that use RIA buffer instead of VGF-derived peptide solutions to measure the maximum binding level were carried out. Reactions for non-specific binding were performed in quadruplicates, and the other reactions were performed in duplicates, and for each of the reactants, the immune complex was precipitated as in the above-mentioned antibody titer measurements, and its radioactivity (cpm) was measured. The average radioactivity of non-specific binding reaction is defined as non-specific binding level N, the radioactivity value for each VGF-derived peptide addition reaction is defined as Y, and the radioactivity value of maximum binding reaction is defined as Z. The percentage ratio ($B/B_0$) of antiserum specific binding level (B) with peptide addition to the maximum binding level ($B_0$), was determined using the following formula.

$$B/B_0\ (\%) = \{(Y - N)/(Z - N)\} \times 100$$

When the antiserum also binds to the added peptide, the added peptide competitively inhibits the binding of antiserum to antigenic peptide in an amount-dependent manner; therefore, the specific binding level of antiserum to antigenic peptide is decreased when the peptide is added. Accordingly, the amount of peptide that achieves 50% $B/B_0$, more specifically, the amount of peptide that yields 50% inhibition of the maximum binding level, was used as an indicator of the peptide binding activity to the antiserum. A smaller amount of peptide required to yield 50% inhibition indicates a greater binding activity.

As a result, the peptide comprising the amino acid sequence of ESPGPERVW, a portion of human VGF and corresponding to the C-terminal portion of SEQ ID NO: 1 added with a tyrosine residue at its N terminus, inhibited the binding of the antiserum in an amount-dependent manner and the amount of the peptide that yields 50% inhibition was 9.0 fmol. Binding was examined for non-VGF peptides, such as angiotensin II, calcitonin gene-related peptide, Leu-enkephalin, neuromedin U-8, vasopressin, Met-enkephalin-Arg-Gly-Leu, adrenomedullin, atrial natriuretic peptide, calcitonin, peptide HI, corticotropin-releasing factor, PAMP-20, calcitonin receptor-stimulating peptide, neurotensin, secretin, neuropeptide Y, β melanocyte-stimulating hormone, melanin-concentrating hormone, somatostatin, and glucagon in the same way as described above using 1, 10, and 100 pmol of the peptides, but none of these peptides showed inhibition at concentrations of 1, 10, and 100 pmol, and thereby confirmed that the antibody specifically binds to peptides derived from human VGF.

[Example 5] Continuous measurement of AVP-eGFP fluorescence in rat posterior pituitary gland

In transgenic (Tg) rats produced using a fusion gene, which is a vasopressin (arginine vasopressin: AVP) gene inserted with the enhanced green fluorescent protein (eGFP) gene, eGFP is expressed specifically in AVP neurons of the hypothalamus-pituitary gland system and their axons (Ueta et al., Endocrinology, 146, 406-413, 2005). The pituitary gland rapidly excised from AVP-eGFP Tg rats were used in this experiment.

Tg rats, both male and female, with body weights 250 g to 350 g were used. Until use in the experiments, the rats were allowed free access to food and water with 12-hour dark/light cycles (light period 7:00 to 19:00) in the Animal Center of the University (temperature, $24 \pm 1°C$; humidity, $54 \pm 5\%$).

Immediately after cervical dislocation, the pituitary gland was excised from AVP-eGFP Tg rats, and placed in a chamber of a perfusion apparatus filled with perfusate (140 mM NaCl, 5 mM KCl, 10 mM HEPES, 10 mM glucose, 1.2 mM $KH_2PO_4$, 1.2 mM $MgCl_2$, 2 mM $CaCl_2$; the pH and osmotic pressure were adjusted to 7.37 and 295 to 300 mOsml, respectively). Laser beam (488 nm) from an excitation light irradiation device (161C; manufactured by Spectra Physics) was irradiated onto the posterior pituitary gland through an optical fiber (GIF625-100; manufactured by Thorlabs). The eGFP fluorescent light as a result of excitation at nerve endings in the posterior pituitary gland was collected by a phototube (R6249HA; Hamamatsu Photonics) through another optical fiber. After conversion into an electric signal, it was amplified with an amplifier (C7246; manufactured by Hamamatsu Photonics). An excitation light shielding filter was placed in front of the phototube to prevent the detection of excitation light itself. The amplified signal was stored in a computer with recording software (chart v3.4; manufactured by Castle Hill) through an analog/digital converter (MacLab/4; manufactured by Castle Hill).

The recording time was 10 minutes. As a control (basal), only the perfusate was administered in the first five minutes. Then, the perfusate mixed with Peptide 12 (SEQ ID NO: 28), Peptide 1 (SEQ ID NO: 1), or Peptide 11 (SEQ ID NO: 25) to make a final concentration of $10^{-6}$ M was administered in the last five minutes and changes in eGFP fluorescence were observed. The peptide reagents were adjusted to make a final concentration of 10 nmol by dissolving each in the above-described perfusate added with 0.05% BSA. The temperature of the perfusate was kept at $37 \pm 0.5°C$ during the experiment. The obtained data was presented as the rate of signal decrease after peptide administration by taking the control (basal) as 1. The results are shown in Figs. 33 to 35. As shown in Figs. 33 to 35, peptide administration reduced eGFP fluorescence, and AVP-eGFP stored in the nerve endings of the posterior pituitary gland was released by exocytosis.

Industrial Applicability

The present invention provides novel peptides having energy-modulating activity or circulation-modulating activity, antibodies that specifically bind to the peptides, and methods which use the peptides for screening for substances that promote or suppress the activity of the peptides, or for agonists or antagonists of the receptors for the peptides. Since the peptides, and substances that promote or suppress the activity of the peptides, and agonists or antagonists of the receptors for the peptides, which are obtained by the screening methods of the present invention, have energy-modulating activity or circulation-modulating activity, they are useful as food consumption-modulating agents, water consumption-modulating agents, metabolism-improving agents, circulation-modulating agents, and vasopressors, and can be used for treating diseases associated with energy modulation such as food or water consumption disorders, metabolic disorders, and sleep disorders, or diseases of the circulatory system such as myocardial infarction, ischemic heart disease, cerebral infarction, and the like.

[Sequence Listing Free Text]

SEQ ID NO: 1 - Inventors: Yamasaki, Motoo; Takahashi, Noriyuki; Minamino, Naoto; Inventors: Sasaki, Kazuki; Takao, Toshifumi; Satomi, Yoshinori; Inventors: Ueta, Yoichi

**Claims**

1. A peptide of any one of (a) to (d) below or a pharmaceutically acceptable salt thereof:

    (a) a peptide comprising the amino acid sequence of any one of SEQ ID NOS: 1 to 9, 11, 12, and 26 (but excluding a peptide consisting of the amino acid sequence of any one of SEQ ID NOS: 13 to 21);
    (b) a peptide comprising an amino acid sequence with substitution, deletion, or addition of one to five amino acids in the amino acid sequence of any one of SEQ ID NOS: 1 to 9, 11, 12, and 26, wherein the peptide has an activity of increasing the intracellular calcium ion concentration in a cell of hypothalamus, pituitary gland, kidney, heart, blood vessel, or brain tissue;
    (c) a peptide comprising an amino acid sequence having 90% or higher homology to the amino acid sequence of any one of SEQ ID NOS: 1 to 9, 11, 12, and 26, wherein the peptide has an activity of increasing the intracellular calcium ion concentration in a cell of hypothalamus, pituitary gland, kidney, heart, blood vessel, or brain tissue; and
    (d) a peptide represented by the following formula (I)

    $$R^1\text{-}A\text{-}R^2 \qquad (I)$$

    (wherein, $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue of the peptide of any one of the above-mentioned (a) to (c)).

2. A DNA encoding any one of the peptides of (a) to (c) of claim 1.

3. A recombinant vector obtainable by incorporating the DNA of claim 2 into a vector.

4. A transformant obtainable by introducing the recombinant vector of claim 3 into a host cell.

5. A method for producing a peptide, which comprises culturing the transformant of claim 4 in a medium so as to produce and accumulate said peptide in the culture, and recovering said peptide from the culture.

6. An antibody that binds to an epitope present in the amino acid sequence of SEQ ID NO: 1 or 26.

7. A method of detecting or quantifying the peptide of claim 1, which comprises using the antibody of claim 6.

8. An energy-modulating agent comprising as an active ingredient at least one peptide selected from (a) to (f) below or a pharmaceutically acceptable salt thereof:

    (a) a peptide comprising the amino acid sequence of any one of SEQ ID NOS: 1 to 12, and 23 to 29;
    (b) a peptide comprising an amino acid sequence with substitution, deletion, or addition of one to five amino acids in the amino acid sequence of any one of SEQ ID NOS: 1 to 12, and 23 to 29, wherein the peptide has an activity of increasing the intracellular calcium ion concentration in a cell of hypothalamus, pituitary gland, or brain tissue;
    (c) a peptide comprising an amino acid sequence having 90% or higher homology to the amino acid sequence of any one of SEQ ID NOS: 1 to 12, and 23 to 29, wherein the peptide has an activity of increasing the intracellular calcium ion concentration in a cell of hypothalamus, pituitary gland, or brain tissue;
    (d) a peptide comprising an amino acid sequence with substitution, deletion, or addition of one to five amino acids in the amino acid sequence of any one of SEQ ID NOS: 1, 25, 28, and 29, wherein the peptide has an activity of promoting vasopressin secretion from the posterior pituitary gland;
    (e) a peptide comprising an amino acid sequence having 90% or higher homology to the amino acid sequence of any one of SEQ ID NOS: 1, 25, 28, and 29, wherein the peptide has an activity of promoting vasopressin secretion from the posterior pituitary gland; and
    (f) a peptide represented by the following formula (II)

    $$R^3\text{-}B\text{-}R^4 \qquad (II)$$

(wherein, R³ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; R⁴ represents hydroxy, substituted or unsubstituted alkoxy, or substituted or unsubstituted amino; and B represents a peptide residue of the peptide of any one of the above-mentioned (a) to (e)).

9. A circulation-modulating agent comprising as an active ingredient at least one peptide selected from (a) to (f) below or a pharmaceutically acceptable salt thereof:

(a) a peptide comprising the amino acid sequence of any one of SEQ ID NOS: 1 to 12;
(b) a peptide comprising an amino acid sequence with substitution, deletion, or addition of one to five amino acids in the amino acid sequence of any one of SEQ ID NOS: 1 to 12, wherein the peptide has an activity of increasing the intracellular calcium ion concentration in a cell of kidney, heart, or blood vessel;
(c) a peptide comprising an amino acid sequence having 90% or higher homology to the amino acid sequence of any one of SEQ ID NOS: 1 to 12, wherein the peptide has an activity of increasing the intracellular calcium ion concentration in a cell of kidney, heart, or blood vessel;
(d) a peptide comprising an amino acid sequence with substitution, deletion, or addition of one to five amino acids in the amino acid sequence of any one of SEQ ID NOS: 1, 25, 28, and 29, wherein the peptide has an activity of promoting vasopressin secretion from the posterior pituitary gland;
(e) a peptide comprising an amino acid sequence having 90% or higher homology to the amino acid sequence of any one of SEQ ID NOS: 1, 25, 28, and 29, wherein the peptide has an activity of promoting vasopressin secretion from the posterior pituitary gland; and
(f) a peptide represented by the following formula (III)

$$R^5\text{-}C\text{-}R^6 \qquad (III)$$

(wherein, R⁵ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; R⁶ represents hydroxy, substituted or unsubstituted alkoxy, or substituted or unsubstituted amino; and C represents a peptide residue of the peptide of any one of the above-mentioned (a) to (e)).

10. A method of screening for a substance that inhibits peptide-induced increase of intracellular calcium ion concentration in a cell of hypothalamus, pituitary gland, kidney, heart, blood vessel, or brain tissue, which comprises:

measuring the cellular response elicited when a test substance and the peptide of any one of (a) to (f) of claim 8 or 9 or a pharmaceutically acceptable salt thereof are contacted with a cell of hypothalamus, pituitary gland, kidney, heart, blood vessel, or brain tissue; and
identifying the test substance as a substance that inhibits the peptide-induced increase of intracellular calcium ion concentration in the cell of hypothalamus, pituitary gland, kidney, heart, blood vessel, or brain tissue, if the test substance suppresses the cellular response compared to the cellular response when said peptide or a pharmaceutically acceptable salt thereof is contacted with said cell in the absence of the test substance.

11. A method of screening for a substance that promotes peptide-induced increase of intracellular calcium ion concentration in a cell of hypothalamus, pituitary gland, kidney, heart, blood vessel, or brain tissue, which comprises:

measuring the cellular response elicited when a test substance and the peptide of any one of (a) to (f) of claim 8 or 9 or a pharmaceutically acceptable salt thereof are contacted with a cell of hypothalamus, pituitary gland, kidney, heart, blood vessel, or brain tissue; and
identifying the test substance as a substance that promotes the peptide-induced increase of intracellular calcium ion concentration in the cell of hypothalamus, pituitary gland, kidney, heart, blood vessel, or brain tissue, if the test substance promotes the cellular response as compared to the cellular response when said peptide or a pharmaceutically acceptable salt thereof is contacted with said cell in the absence of the test substance.

12. A method of screening for a peptide receptor agonist or antagonist, the method comprising:

measuring the binding level of the peptide of any one of (a) to (f) of claim 8 or 9 or a pharmaceutically acceptable salt thereof to a cell of hypothalamus, pituitary gland, kidney, heart, blood vessel, or brain tissue, or a membrane fraction of said cell, when the test substance and the peptide or a pharmaceutically acceptable salt thereof are contacted with said cell or cell membrane fraction; and

identifying the test substance as an agonist or antagonist for the receptor of said peptide if the test substance causes a decrease in the binding level of said peptide or a pharmaceutically acceptable salt thereof as compared to the binding level when said peptide or a pharmaceutically acceptable salt thereof is contacted with said cell or a membrane fraction of said cell in the absence of the test substance.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

A-8: HYPOTHALAMUS 5 $\mu$M

FIG. 25

A-8: PITUITARY GLAND 5 μM

FIG. 26

FIG. 27

A-11: PITUITARY GLAND 1 μM

FIG. 28

FIG. 29

FIG. 30

A-12: PITUITARY GLAND 5 μM

FIG. 31

A-8+DLEAQA: PITUITARY GLAND 5 $\mu$M

FIG. 32

FIG. 33

FIG. 34

FIG. 35

## INTERNATIONAL SEARCH REPORT

| International application No. |
|---|
| PCT/JP2007/073026 |

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C12N15/09, A61K38/00, A61P1/14, A61P3/00, A61P3/04, A61P3/14, A61P9/10, A61P25/20, A61P43/00, C07K14/475, C07K16/22, C12N1/15, C12N1/19, C12N1/21, C12N5/10, C12P21/02, C12Q1/02, G01N33/15, G01N33/50, G01N33/53

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS/MEDLINE/WPIDS (STN), GenBank/EMBL/DDBJ/GeneSeq, UniProt/Geneseq

C. DOCUMENTS CONSIDERED TO BE RELEVANT

| Category* | Citation of document, with indication, where appropriate, of the relevant passages | Relevant to claim No. |
|---|---|---|
| Y | Nadia Canu, et al., 'Cloning, Structural Organization Analysis, and Chromosomal Assignment of the Human Gene for the Neurosecretory Protein VGF.' Genomics, Vol. 45, No.2, (1997), p. 443-446 | 1-12 |
| Y | J.W. Liu, et al., 'Peptide V : a VGF-derived neuron peptide purified from bovine posterior pituitary.' Endocrinology, Vol. 135, No.6, (1994), p.2742-2748 | 1-12 |
| Y | WO 2002/082075 A2 (BIOVISION A.G.), 17 October, 2002 (17.10.02), & JP 2004-531250 A & EP 1373905 A2 & US 2004/0142388 A1 | 1-12 |

|☒| Further documents are listed in the continuation of Box C. | |☐| See patent family annex. |

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 05 March, 2008 (05.03.08) | 18 March, 2008 (18.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
|---|
| PCT/JP2007/073026 |

C (Continuation).   DOCUMENTS CONSIDERED TO BE RELEVANT

| Category* | Citation of document, with indication, where appropriate, of the relevant passages | Relevant to claim No. |
|---|---|---|
| Y | Seung Hahm, et al.,' Targeted Deletion of the Vgf Gene Indicates that the Encoded Secretory Peptide Precursor Plays a Novel Role in the Regulation of Energy Balance.' Neuron, Vol.23, No.3, (1999), p.537-548 | 8-12 |
| Y | Levi A., et al., 'Processing, Distribution, and Function of VGF, a Neuronal and Endocrine Peptide Precursor: Honoring John William Daly, Part II.' Cellular and Molecular Neurobiology, Vol.24, No.4, (2004), p. 517-533 | 8-12 |

Form PCT/ISA/210 (continuation of second sheet) (April 2007)

**INTERNATIONAL SEARCH REPORT**

International application No.

PCT/JP2007/073026

Continuation of A. CLASSIFICATION OF SUBJECT MATTER
  (International Patent Classification (IPC))

C12N15/09(2006.01)i, A61K38/00(2006.01)i, A61P1/14(2006.01)i,
A61P3/00(2006.01)i, A61P3/04(2006.01)i, A61P3/14(2006.01)i,
A61P9/10(2006.01)i, A61P25/20(2006.01)i, A61P43/00(2006.01)i,
C07K14/475(2006.01)i, C07K16/22(2006.01)i, C12N1/15(2006.01)i,
C12N1/19(2006.01)i, C12N1/21(2006.01)i, C12N5/10(2006.01)i,
C12P21/02(2006.01)i, C12Q1/02(2006.01)i, G01N33/15(2006.01)i,
G01N33/50(2006.01)i, G01N33/53(2006.01)i

        (According to International Patent Classification (IPC) or to both national
        classification and IPC)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0107477 A
- WO 0282075 A
- JP 58110600 A
- JP 3022979 A
- JP 248394288 B
- JP 2227075 A
- JP 63000299 A
- JP 59140885 A
- JP 60070080 A

- WO 9400977 A
- JP 60251887 A
- JP 2606856 B
- JP 2517813 B
- JP 1102096 A
- JP 5336963 A
- WO 9423021 A
- WO 02010371 A

### Non-patent literature cited in the description

- *Science, (USA),* 1985, vol. 229 (4711), 393-395
- *Genomics, (USA),* 1997, vol. 45 (2), 443-446
- *Neuron, (USA),* 1999, vol. 23 (3), 537-548
- *Cellular and Molecular Neurobiology, (USA),* 2004, vol. 24 (4), 517-533
- *Journal of Neurochemistry, (UK),* 2002, vol. 81 (3), 565-574
- *Proceeding of the National Academy of Sciences of the United States of America, (USA),* 2006, vol. 103 (39), 14584-14589
- *European Journal of Neuroscience, (France),* 2004, vol. 20 (11), 3035-3040
- *Journal of Chromatography B: Biomedical Sciences and Applications, (Holland),* 2001, vol. 754 (2), 357-367
- *Endocrinology, (USA),* 1994, vol. 135 (6), 2742-2748
- *Endocrinology, (USA),* 1999, vol. 140 (8), 3727-3735
- *The EMBO Journal, (UK),* 1989, vol. 8 (8), 2217-2223
- *FEMS Microbiol Lett.,* 1999, vol. 174, 247
- **Izumiya, N. ; Kato, T. et al.** Fundamentals and Experiments of Peptide Synthesis. Maruzen, 1985
- Experimental Chemical Course. **Aimoto, S. et al.** Organic Synthesis (Yuki Gosei) IV, Acid, Amino acid and Peptide. Maruzen, 1999, vol. 22
- *Int. J. Pept. Protein Res.,* 1990, vol. 35, 161-214
- Solid-Phase Peptide Synthesis. **Fields, G. B.** Methods in Enzymology. Academic Press, 1997, vol. 289

- **Pennington, M. W. ; Dunn, B. M.** Peptide Synthesis Protocols, Methods in Molecular Biology. Humana Press, 1994, vol. 35
- **Izumiya, N. et al.** Fundamentals and Experiments of Peptide Synthesis. Maruzen, 1985
- The sequel of Development of Pharmaceuticals. Peptide Synthesis. Hirokawa Shoten, 1991, vol. 14
- Biochemistry Experimental Course. Chemistry of Protein IV-Chemical Modification and Peptide Synthesis. Tokyo Kagaku Dojin, vol. 1
- Experimental Methods in Biological Chemistry. **Ohno, M. et al.** Chemical Modification of Proteins. Japan Scientific Societies Press, 1981, vol. 12, 13
- Molecular Cloning: A Laboratory Manual. Cold Spring Harbor Laboratory Press, 2001
- *Agric. Biol. Chem.,* 1984, vol. 48, 669
- *Agric. Biol. Chem.,* 1989, vol. 53, 277
- *Proc. Natl. Acad. Sci., USA,* 1985, vol. 82, 4306
- *Proc. Natl. Acad. Sci. USA,* 1972, vol. 69, 2110
- *Gene,* 1982, vol. 17, 107
- *Molecular & General Genetics,* 1979, vol. 168, 111
- *Methods. in Enzymol.,* 1990, vol. 194, 182
- *Proc. Natl. Acad. Sci. USA,* 1978, vol. 75, 1929
- *J. Bacteriology.,* 1983, vol. 153, 163
- *Cytotechnology,* 1990, vol. 3, 133
- *Nature,* 1987, vol. 329, 840
- *J. Biochem.,* 1987, vol. 101, 1307
- *Proc. Natl. Acad. Sci. USA,* 1987, vol. 84, 7413
- *Virology,* 1973, vol. 52, 456

- Current Protocols in Molecular Biology; Baculovirus Expression Vectors. A Laboratory Manual. W.H. Freeman and Company, 1992
- *Bio/Technology,* 1988, vol. 6, 47
- Baculovirus Expression Vectors. A Laboratory Manual. W. H. Freeman and Company, 1992
- *The Journal of the American Medical Association,* 1967, vol. 199, 519
- *Science,* 1952, vol. 122, 501
- *Virology,* 1959, vol. 8, 396
- *Proceeding of the Society for the Biological Medicine,* 1950, vol. 73, 1
- *Nature,* 1962, vol. 195, 788
- *J. Biol. Chem.,* 1989, vol. 264, 17619
- *Proc. Natl. Acad. Sci., USA,* 1989, vol. 86, 8227
- *Genes Develop.,* 1990, vol. 4, 1288
- Enzyme-linked Immunosorbent Assay. Igaku Shoin, 1976
- Antibodies - A Laboratory Manual. Cold Spring Harbor Laboratory, 1988
- Antibodies, A Laboratory manual. Cold Spring Harbor Laboratory, 1988
- *Curr. Topics. Microbiol. Immunol.,* 1978, vol. 81, 1
- *Europ. J. Immunol.,* 1976, vol. 6, 511
- *Nature,* 1978, vol. 276, 269
- *J. Immunol.,* 1979, vol. 123, 1548
- *Nature,* 1975, vol. 256, 495
- Tan-Clone-Kotai-Manual. Kodansha-Scientific, 1987
- Zoku-Seikagaku Jikken Kouza 5, Meneki-seikagaku Kenkyuho. Tokyo Kagaku Dojin, 1986
- **Ueta et al.** *Endocrinology,* 2005, vol. 146, 406-413

SEQUENCE LISTING

<110> Kyowa Hakko Kogyo Co., Ltd.
Japan as Represented by the President of National Cardiovascular Center
Osaka University

<120> Novel peptide

<130> K5-A0704P

<150> JP2007-14455
<151> 2007-01-25

<160> 29

<170> PatentIn version 3.2

<210> 1
<211> 30
<212> PRT
<213> Homo sapiens

<220>
<223> Inventor: Yamasaki, Motoo; Takahashi, Noriyuki; Minamino, Naoto;
Inventor: Sasaki, Kazuki; Takao, Toshifumi; Satomi, Yoshinori;
Inventor: Ueta, Yoichi

<220>
<221> MOD_RES
<222> (1)..(1)
<223> PYRROLIDONE CARBOXYLIC ACID

<400> 1

Gln Gln Glu Thr Ala Ala Ala Glu Thr Glu Thr Arg Thr His Thr Leu
1               5                   10                  15

Thr Arg Val Asn Leu Glu Ser Pro Gly Pro Glu Arg Val Trp
            20                  25                  30

<210> 2
<211> 12
<212> PRT
<213> Homo sapiens

<400> 12
Val Asn Leu Glu Ser Pro Gly Pro Glu Arg Val Trp
1               5                   10

<210> 3
<211> 11
<212> PRT
<213> Homo sapiens

<400> 3

Asn Ala Pro Pro Glu Pro Val Pro Pro Pro Arg
1               5                   10

<210> 4
<211> 11
<212> PRT
<213> Homo sapiens

<400> 4

Glu Glu Asp Glu Val Tyr Pro Pro Gly Pro Tyr
1           5           10

<210>   5
<211>   26
<212>   PRT
<213>   Rattus norvegicus

<400>   5

Ala Ser Trp Gly Glu Phe Gln Ala Arg Val Pro Glu Arg Ala Pro Leu
1           5           10              15

Pro Pro Ser Val Pro Ser Gln Phe Gln Ala
            20          25

<210>   6
<211>   20
<212>   PRT
<213>   Rattus norvegicus

<400>   6

Gly Leu Gln Glu Thr Gln Gln Glu Arg Glu Asn Glu Arg Glu Glu Glu
1           5           10              15

Ala Glu Gln Glu
            20

<210>   7
<211>   18
<212>   PRT
<213>   Homo sapiens

<400>   7

Gln Asn Ala Leu Leu Phe Ala Glu Glu Glu Asp Gly Glu Ala Gly Ala
1           5           10              15

Glu Asp

<210>   8
<211>   8
<212>   PRT
<213>   Rattus norvegicus

<400>   8

Ser Gln Glu Glu Ala Pro Gly His
1           5

<210>   9
<211>   26
<212>   PRT
<213>   Homo sapiens

<400>   9

Ala Ser Trp Gly Glu Phe Gln Ala Arg Val Pro Glu Arg Ala Pro Leu
1           5           10              15

Pro Pro Pro Ala Pro Ser Gln Phe Gln Ala
            20          25

28

<210> 10
<211> 21
<212> PRT
<213> Homo sapiens

<400> 10

Gly Leu Gln Glu Ala Ala Glu Glu Arg Glu Ser Ala Arg Glu Glu Glu
1               5                   10                  15

Glu Ala Glu Gln Glu
            20


<210> 11
<211> 8
<212> PRT
<213> Homo sapiens

<400> 11

Ser Gln Glu Glu Thr Pro Gly His
1               5


<210> 12
<211> 11
<212> PRT
<213> Rattus norvegicus

<400> 12

Glu Glu Asp Glu Val Phe Pro Pro Gly Pro Tyr
1               5                   10


<210> 13
<211> 615
<212> PRT
<213> Homo sapiens

<400> 13

Met Lys Ala Leu Arg Leu Ser Ala Ser Ala Leu Phe Cys Leu Leu Leu
1               5                   10                  15

Ile Asn Gly Leu Gly Ala Ala Pro Pro Gly Arg Pro Glu Ala Gln Pro
                20                  25                  30

Pro Pro Leu Ser Ser Glu His Lys Glu Pro Val Ala Gly Asp Ala Val
            35                  40                  45

Pro Gly Pro Lys Asp Gly Ser Ala Pro Glu Val Arg Gly Ala Arg Asn
        50                  55                  60

Ser Glu Pro Gln Asp Glu Gly Glu Leu Phe Gln Gly Val Asp Pro Arg
65                  70                  75                  80

Ala Leu Ala Ala Val Leu Leu Gln Ala Leu Asp Arg Pro Ala Ser Pro
                85                  90                  95

Pro Ala Pro Ser Gly Ser Gln Gln Gly Pro Glu Glu Glu Ala Ala Glu
100 105 110

Ala Leu Leu Thr Glu Thr Val Arg Ser Gln Thr His Ser Leu Pro Ala
115 120 125

Pro Glu Ser Pro Glu Pro Ala Ala Pro Pro Arg Pro Gln Thr Pro Glu
130 135 140

Asn Gly Pro Glu Ala Ser Asp Pro Ser Glu Glu Leu Glu Ala Leu Ala
145 150 155 160

Ser Leu Leu Gln Glu Leu Arg Asp Phe Ser Pro Ser Ser Ala Lys Arg
165 170 175

Gln Gln Glu Thr Ala Ala Ala Glu Thr Glu Thr Arg Thr His Thr Leu
180 185 190

Thr Arg Val Asn Leu Glu Ser Pro Gly Pro Glu Arg Val Trp Arg Ala
195 200 205

Ser Trp Gly Glu Phe Gln Ala Arg Val Pro Glu Arg Ala Pro Leu Pro
210 215 220

Pro Pro Ala Pro Ser Gln Phe Gln Ala Arg Met Pro Asp Ser Gly Pro
225 230 235 240

Leu Pro Glu Thr His Lys Phe Gly Glu Gly Val Ser Ser Pro Lys Thr
245 250 255

His Leu Gly Glu Ala Leu Ala Pro Leu Ser Lys Ala Tyr Gln Gly Val
260 265 270

Ala Ala Pro Phe Pro Lys Ala Arg Arg Pro Glu Ser Ala Leu Leu Gly
275 280 285

Gly Ser Glu Ala Gly Glu Arg Leu Leu Gln Gln Gly Leu Ala Gln Val
290 295 300

Glu Ala Gly Arg Arg Gln Ala Glu Ala Thr Arg Gln Ala Ala Ala Gln
305 310 315 320

Glu Glu Arg Leu Ala Asp Leu Ala Ser Asp Leu Leu Leu Gln Tyr Leu
325 330 335

Leu Gln Gly Gly Ala Arg Gln Arg Gly Leu Gly Gly Arg Gly Leu Gln
340 345 350

Glu Ala Ala Glu Glu Arg Glu Ser Ala Arg Glu Glu Glu Glu Ala Glu
355 360 365

Gln Glu Arg Arg Gly Gly Glu Glu Arg Val Gly Glu Glu Asp Glu Glu

370            375            380

Ala Ala Glu Ala Glu Ala Glu Ala Glu Glu Ala Glu Arg Ala Arg Gln
385                 390                 395                 400

Asn Ala Leu Leu Phe Ala Glu Glu Glu Asp Gly Glu Ala Gly Ala Glu
                405                 410                 415

Asp Lys Arg Ser Gln Glu Glu Thr Pro Gly His Arg Arg Lys Glu Ala
                420                 425                 430

Glu Gly Thr Glu Glu Gly Gly Glu Glu Glu Asp Asp Glu Glu Met Asp
            435                 440                 445

Pro Gln Thr Ile Asp Ser Leu Ile Glu Leu Ser Thr Lys Leu His Leu
        450                 455                 460

Pro Ala Asp Asp Val Val Ser Ile Ile Glu Glu Val Glu Glu Lys Arg
465                 470                 475                 480

Lys Arg Lys Lys Asn Ala Pro Pro Glu Pro Val Pro Pro Pro Arg Ala
                485                 490                 495

Ala Pro Ala Pro Thr His Val Arg Ser Pro Gln Pro Pro Pro Pro Ala
        500                 505                 510

Pro Ala Pro Ala Arg Asp Glu Leu Pro Asp Trp Asn Glu Val Leu Pro
        515                 520                 525

Pro Trp Asp Arg Glu Glu Asp Glu Val Tyr Pro Pro Gly Pro Tyr His
        530                 535                 540

Pro Phe Pro Asn Tyr Ile Arg Pro Arg Thr Leu Gln Pro Pro Ser Ala
545                 550                 555                 560

Leu Arg Arg Arg His Tyr His His Ala Leu Pro Pro Ser Arg His Tyr
                565                 570                 575

Pro Gly Arg Glu Ala Gln Ala Arg Arg Ala Gln Glu Glu Ala Glu Ala
            580                 585                 590

Glu Glu Arg Arg Leu Gln Glu Gln Glu Glu Leu Glu Asn Tyr Ile Glu
            595                 600                 605

His Val Leu Leu Arg Arg Pro
    610                 615

<210>   14
<211>   617
<212>   PRT
<213>   Rattus norvegicus

<400>   14

31

```
Met Lys Thr Phe Thr Leu Pro Ala Ser Val Leu Phe Cys Phe Leu Leu
1               5                   10                  15

Leu Ile Arg Gly Leu Gly Ala Ala Pro Pro Gly Arg Ser Asp Val Tyr
            20                  25                  30

Pro Pro Pro Leu Gly Ser Glu His Asn Gly Gln Val Ala Glu Asp Ala
        35                  40                  45

Val Ser Arg Pro Lys Asp Asp Ser Val Pro Glu Val Arg Ala Ala Arg
        50                  55                  60

Asn Ser Glu Pro Gln Asp Gln Gly Glu Leu Phe Gln Gly Val Asp Pro
65                  70                  75                  80

Arg Ala Leu Ala Ala Val Leu Leu Gln Ala Leu Asp Arg Pro Ala Ser
                85                  90                  95

Pro Pro Ala Val Pro Ala Gly Ser Gln Gln Gly Thr Pro Glu Glu Ala
            100                 105                 110

Ala Glu Ala Leu Leu Thr Glu Ser Val Arg Ser Gln Thr His Ser Leu
        115                 120                 125

Pro Ala Ser Glu Ile Gln Ala Ser Ala Val Ala Pro Pro Arg Pro Gln
    130                 135                 140

Thr Gln Asp Asn Asp Pro Glu Ala Asp Asp Arg Ser Glu Glu Leu Glu
145                 150                 155                 160

Ala Leu Ala Ser Leu Leu Gln Glu Leu Arg Asp Phe Ser Pro Ser Asn
                165                 170                 175

Ala Lys Arg Gln Gln Glu Thr Ala Ala Ala Glu Thr Glu Thr Arg Thr
            180                 185                 190

His Thr Leu Thr Arg Val Asn Leu Glu Ser Pro Gly Pro Glu Arg Val
            195                 200                 205

Trp Arg Ala Ser Trp Gly Glu Phe Gln Ala Arg Val Pro Glu Arg Ala
    210                 215                 220

Pro Leu Pro Pro Ser Val Pro Ser Gln Phe Gln Ala Arg Met Ser Glu
225                 230                 235                 240

Asn Val Pro Leu Pro Glu Thr His Gln Phe Gly Glu Gly Val Ser Ser
                245                 250                 255

Pro Lys Thr His Leu Gly Glu Thr Leu Thr Pro Leu Ser Lys Ala Tyr
            260                 265                 270

Gln Ser Leu Ser Ala Pro Phe Pro Lys Val Arg Arg Leu Glu Gly Ser
        275                 280                 285

Phe Leu Gly Gly Ser Glu Ala Gly Glu Arg Leu Leu Gln Gln Gly Leu
    290                 295                 300

Ala Gln Val Glu Ala Gly Arg Arg Gln Ala Glu Ala Thr Arg Gln Ala
305                 310                 315                 320

Ala Ala Gln Glu Glu Arg Leu Ala Asp Leu Ala Ser Asp Leu Leu Leu
            325                 330                 335

Gln Tyr Leu Leu Gln Gly Gly Ala Arg Gln Arg Asp Leu Gly Gly Arg
            340                 345                 350

Gly Leu Gln Glu Thr Gln Gln Glu Arg Glu Asn Glu Arg Glu Glu Glu
    355                 360                 365
```

32

```
Ala Glu Gln Glu Arg Arg Gly Gly Gly Glu Asp Glu Val Gly Glu Glu
    370                 375             380
Asp Glu Glu Ala Ala Glu Ala Glu Ala Glu Ala Glu Glu Ala Glu Arg
    385             390             395                 400
Ala Arg Gln Asn Ala Leu Leu Phe Ala Glu Glu Glu Asp Gly Glu Ala
                405             410                 415
Gly Ala Glu Asp Lys Arg Ser Gln Glu Glu Ala Pro Gly His Arg Arg
            420             425             430
Lys Asp Ala Glu Gly Thr Glu Glu Gly Gly Glu Glu Asp Asp Asp
        435             440             445
Glu Glu Met Asp Pro Gln Thr Ile Asp Ser Leu Ile Glu Leu Ser Thr
    450             455             460
Lys Leu His Leu Pro Ala Asp Asp Val Val Ser Ile Ile Glu Glu Val
465             470             475                 480
Glu Glu Lys Arg Lys Arg Lys Lys Asn Ala Pro Pro Glu Pro Val Pro
            485             490             495
Pro Pro Arg Ala Ala Pro Ala Pro Thr His Val Arg Ser Pro Gln Pro
        500             505             510
Pro Pro Pro Ala Pro Ala Arg Asp Glu Leu Pro Asp Trp Asn Glu Val
        515             520             525
Leu Pro Pro Trp Asp Arg Glu Glu Asp Glu Val Phe Pro Pro Gly Pro
    530             535             540
Tyr His Pro Phe Pro Asn Tyr Ile Arg Pro Arg Thr Leu Gln Pro Pro
545             550             555                 560
Ala Ser Ser Arg Arg Arg His Phe His His Ala Leu Pro Pro Ala Arg
            565             570             575
His His Pro Asp Leu Glu Ala Gln Ala Arg Arg Ala Gln Glu Glu Ala
        580             585             590
Asp Ala Glu Glu Arg Arg Leu Gln Glu Gln Glu Glu Leu Glu Asn Tyr
        595             600             605
Ile Glu His Val Leu Leu His Arg Pro
    610             615
```

```
<210>  15
<211>  37
<212>  PRT
<213>  Homo sapiens

<400>  15

Asn Ala Pro Pro Glu Pro Val Pro Pro Pro Arg Ala Ala Pro Ala Pro
1               5               10              15
Thr His Val Arg Ser Pro Gln Pro Pro Pro Ala Pro Ala Pro Ala
            20              25              30
Arg Asp Glu Leu Pro
            35
```

```
<210>   16
<211>   38
<212>   PRT
<213>   Homo sapiens

<400>   16

Asn Ala Pro Pro Glu Pro Val Pro Pro Pro Arg Ala Ala Pro Ala Pro
1               5                   10                  15

Thr His Val Arg Ser Pro Gln Pro Pro Pro Pro Ala Pro Ala Pro Ala
            20                  25                  30

Arg Asp Glu Leu Pro Asp
            35


<210>   17
<211>   75
<212>   PRT
<213>   Rattus norvegicus

<400>   17

Asn Ala Pro Pro Glu Pro Val Pro Pro Pro Arg Ala Ala Pro Ala Pro
1               5                   10                  15

Thr His Val Arg Ser Pro Gln Pro Pro Pro Pro Ala Pro Ala Arg Asp
            20                  25                  30

Glu Leu Pro Asp Trp Asn Glu Val Leu Pro Pro Trp Asp Arg Glu Glu
            35                  40                  45

Asp Glu Val Phe Pro Pro Gly Pro Tyr His Pro Phe Pro Asn Tyr Ile
        50              55                  60

Arg Pro Arg Thr Leu Gln Pro Pro Ala Ser Ser
65                  70                  75


<210>   18
<211>   129
<212>   PRT
<213>   Rattus norvegicus

<400>   18

Asn Ala Pro Pro Glu Pro Val Pro Pro Pro Arg Ala Ala Pro Ala Pro
1               5                   10                  15

Thr His Val Arg Ser Pro Gln Pro Pro Pro Pro Ala Pro Ala Arg Asp
            20                  25                  30

Glu Leu Pro Asp Trp Asn Glu Val Leu Pro Pro Trp Asp Arg Glu Glu
            35                  40                  45

Asp Glu Val Phe Pro Pro Gly Pro Tyr His Pro Phe Pro Asn Tyr Ile
        50              55                  60
```

Arg Pro Arg Thr Leu Gln Pro Pro Ala Ser Ser Arg Arg Arg His Phe
65                70                75                80

His His Ala Leu Pro Pro Ala Arg His His Pro Asp Leu Glu Ala Gln
                85                90                95

Ala Arg Arg Ala Gln Glu Glu Ala Asp Ala Glu Glu Arg Arg Leu Gln
            100                105                110

Glu Gln Glu Glu Leu Glu Asn Tyr Ile Glu His Val Leu Leu His Arg
        115                120                125

Pro


<210>   19
<211>   45
<212>   PRT
<213>   Homo sapiens

<400>   19

Gly Gly Glu Glu Arg Val Gly Glu Glu Asp Glu Glu Ala Ala Glu Ala
1                5                10                15

Glu Ala Glu Ala Glu Glu Ala Glu Arg Ala Arg Gln Asn Ala Leu Leu
            20                25                30

Phe Ala Glu Glu Glu Asp Gly Glu Ala Gly Ala Glu Asp
        35                40

<210>   20
<211>   52
<212>   PRT
<213>   Homo sapiens

<400>   20

Ser Gln Glu Glu Thr Pro Gly His Arg Arg Lys Glu Ala Glu Gly Thr
1                5                10                15

Glu Glu Gly Gly Glu Glu Glu Asp Asp Glu Glu Met Asp Pro Gln Thr
            20                25                30

Ile Asp Ser Leu Ile Glu Leu Ser Thr Lys Leu His Leu Pro Ala Asp
        35                40                45

Asp Val Val Ser
        50

<210>   21
<211>   59
<212>   PRT
<213>   Homo sapiens

<400>   21

Ser Gln Glu Glu Thr Pro Gly His Arg Arg Lys Glu Ala Glu Gly Thr
1                5                10                15

Glu Glu Gly Gly Glu Glu Glu Asp Asp Glu Glu Met Asp Pro Gln Thr
            20                25                30

Ile Asp Ser Leu Ile Glu Leu Ser Thr Lys Leu His Leu Pro Ala Asp
          35                    40                    45

Asp Val Val Ser Ile Ile Glu Glu Val Glu Glu
          50                    55


<210> 22
<211> 75
<212> DNA
<213> Artificial

<220>
<223> DNA encoding amino acid sequence of SEQ ID NO: 9

<220>
<221> CDS
<222> (1)..(78)

<400> 22

gct tct tgg ggt gaa ttt caa gct cgt gtt cct gaa cgt gct cct ctt      48
Ala Ser Trp Gly Glu Phe Gln Ala Arg Val Pro Glu Arg Ala Pro Leu
1                   5                   10                  15

ccc cct cca gct ccg tct caa ttt caa gct tga                          81
Pro Pro Pro Ala Pro Ser Gln Phe Gln Ala
                20                  25


<210> 23
<211> 24
<212> PRT
<213> Homo sapiens

<221> MOD_RES
<222> 24
<223> AMIDATION

<400> 23

Thr Leu Gln Pro Pro Ser Ala Leu Arg Arg Arg His Tyr His His Ala
1                   5                   10                  15

Leu Pro Pro Ser Arg His Tyr Pro
                20


<210> 24
<211> 19
<212> PRT
<213> Homo sapiens

<400> 24

Asn Ala Pro Pro Glu Pro Val Pro Pro Pro Arg Ala Ala Pro Ala Pro
1                   5                   10                  15

Thr His Val


<210> 25
<211> 20
<212> PRT
<213> Homo sapiens

<400> 25

```
Glu Glu Asp Glu Val Tyr Pro Pro Gly Pro Tyr His Pro Phe Pro Asn
                      5                  10                  15
Tyr Ile Arg Pro


<210> 26
<211> 30
<212> PRT
<213> Homo sapiens

<400> 26

Gln Gln Glu Thr Ala Ala Ala Glu Thr Glu Thr Arg Thr His Thr Leu
1                5                  10                  15

Thr Arg Val Asn Leu Glu Ser Pro Gly Pro Glu Arg Val Trp
                 20                  25                  30


<210>   27
<211>   24
<212>   PRT
<213>   Homo sapiens

<400>   27

Thr Leu Gln Pro Pro Ser Ala Leu Arg Arg Arg His Tyr His His Ala
  1                5                  10                  15

Leu Pro Pro Ser Arg His Tyr Pro
                  20


<210>   28
<211>   30
<212>   PRT
<213>   Rattus norvegicus

<400>   28

Thr Leu Gln Pro Pro Ala Ser Ser Arg Arg Arg His Phe His His Ala
  1                5                  10                  15

Leu Pro Pro Ala Arg His His Pro Asp Leu Glu Ala Gln Ala
                 20                  25


<210>   29
<211>   30
<212>   PRT
<213>   Homo sapiens

<400>   29

Thr Leu Gln Pro Pro Ser Ala Leu Arg Arg Arg His Tyr His His Ala
  1                5                  10                  15

Leu Pro Pro Ser Arg His Tyr Pro Gly Arg Glu Ala Gln Ala
                 20                  25
```